(19) Europäisches Patentamt / European Patent Office / Office européen des brevets

(11) **EP 3 845 218 A1**

(12) **EUROPEAN PATENT APPLICATION**
published in accordance with Art. 153(4) EPC


(43) Date of publication:
**07.07.2021 Bulletin 2021/27**

(21) Application number: **19845244.3**

(22) Date of filing: **31.07.2019**

(51) Int Cl.:
***A61K 9/14*** *(2006.01)* ***A61K 9/51*** *(2006.01)*
***A61K 38/40*** *(2006.01)* ***A61K 38/18*** *(2006.01)*
***A61P 17/02*** *(2006.01)*

(86) International application number:
**PCT/KR2019/009531**

(87) International publication number:
**WO 2020/027571 (06.02.2020 Gazette 2020/06)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(30) Priority: **31.07.2018 US 201862712321 P**



(71) Applicant: **Lemonex Inc.**
**Seoul 08826 (KR)**

(72) Inventor: **WON, Cheol Hee**
**Seoul 08741 (KR)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**


(54) **PHARMACEUTICAL COMPOSITION FOR WOUND HEALING**


(57) The present invention provides a composition which can deliver a polypeptide having epithelial or endothelial cell growth promoting ability or a substance for increasing activity or expression thereof in a sustained manner with high efficiency, thereby treating the wound with excellent efficiency.


[FIG. 14]

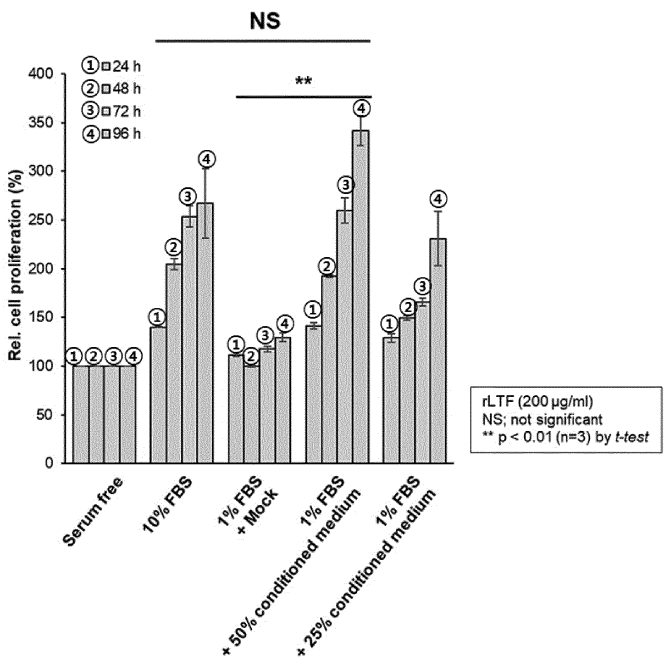


EP 3 845 218 A1

## Description

[Technical Field]

The present invention relates to a pharmaceutical composition having excellent wound healing effects.

[Background Art]

A drug delivery system refers to a medical technique that can efficiently deliver a required amount of drugs, such as proteins, nucleic acids or other small molecules by minimizing side effects while maximizing efficacy and effects of existing drugs. This technique capable of saving costs and time required for development of new drugs has recently become one of advanced techniques that create a new added value in the pharmaceutical industry, in combination with nanotechnique. Since the late 1980s, in particular, companies in the technically advanced countries such as United States, Japan, etc. have made every effort to develop drug delivery systems along with the development of new drugs.

To date, viral genes, recombinant proteins, liposomes, cationic polymers, and various types of nanoparticles and nanomaterials have been used for drug delivery into animal cells. However, many cationic liposomes and cationic polymers have been found to be unsuitable due to their high toxicity to cells for clinical applications. Further, a method of chemically modifying a main chain of the nucleic acid has been attempted for stable cell membrane penetration of the nucleic acid. However, such a method is not suitable for clinical applications because it is expensive, time consuming, and requires labor intensive processes. As a significant attempt, drug delivery systems (DDSs) utilizing various types of nanoparticles, including quantum dots, magnetic particles or gold nanoparticles, have been developed. However, these particles have a disadvantage in that they are toxic to cells, have a structure through which biopolymers such as nucleic acids are not easily introduced, and also have low efficiency of introduction into the cells.

Efficient delivery systems are needed for studying functions of bioactive substances *in vivo* or for intracellular delivery. However, the development of a universal delivery system capable of delivering a wide range of bioactive substances, a system capable of accommodating and delivering a large amount of drugs, and a system for releasing drugs in a sustained manner are still in shortage.

[Summary of Invention]

[Problems to be Solved by Invention]

An object of the present invention is to provide a pharmaceutical composition having excellent wound healing effects.

[Means for Solving Problems]

To achieve the above objects, the following technical solutions are adopted in the present invention.

1. A pharmaceutical composition for treatment of wounds, including:

porous silica particles on which a polypeptide having epithelial or endothelial cell growth promoting ability or a substance for increasing activity or expression thereof is supported,
wherein the porous silica particles are characterized in that t, at which an absorbance ratio in the following Equation 1 becomes 1/2, is 24 or more:

[Equation 1]

$$A_t/A_0$$

(wherein $A_0$ is absorbance of the porous silica particles measured by putting 5 ml of suspension containing 1 mg/ml of porous silica particles into a cylindrical permeable membrane having pores with a pore diameter of 50 kDa,
15 ml of the same solvent as the suspension comes into contact with an outside of the permeable membrane, and the inside/outside of the permeable membrane are horizontally stirred at 60 rpm and at 37 °C,
pH of the suspension is 7.4, and
$A_t$ indicates absorbance of the porous silica particle measured after lapse of "t" hours since $A_o$ was measured).

2. The composition according to the above 1, wherein the porous silica particles are prepared by: reacting silica particles having pores with a pore diameter of less than 5 nm with a swelling agent at 120 to 180 °C for 24 to 96 hours to expand the pores with a pore diameter of less than 5 nm; and calcining the silica particles having expanded pores at a temperature of 400 °C or higher for at least 3 hours.

3. The composition according to the above 1, wherein an average diameter of the porous silica particles ranges from 150 to 1000 nm, a BET surface area ranges from 200 to 700 $m^2/g$, and a volume per gram ranges from 0.7 to 2.2 ml.

4. The composition according to the above 1, wherein the polypeptide is lactoferrin, an epidermal growth factor, a hepatocyte growth factor or a vascular endothelial growth factor.

5. The composition according to the above 4, wherein the lactoferrin protein is composed of a sequence of SEQ ID NO: 1.

6. The composition according to the above 4, wherein the epidermal growth factor is composed of a sequence of SEQ ID NO: 2, the hepatocyte growth factor is composed of a sequence of SEQ ID NO: 3, and the vascular endothelial growth factor is composed of a sequence of SEQ ID NO: 4.

7. The composition according to the above 1, wherein the substance is a plasmid on which a gene encoding the sequence of SEQ ID NO: 1 is supported.

8. The composition according to the above 1, wherein the porous silica particles carry a polypeptide having epithelial or endothelial cell growth promoting ability, and are negatively charged at neutral pH on an outer surface of the particles or an inside of the pores.

9. The composition according to the above 1, wherein the porous silica particles carry a plasmid, on which a gene encoding the polypeptide having epithelial or endothelial cell growth promoting ability is supported, and are positively charged at neutral pH on an outer surface of the particles or an inside of the pores.

[Advantageous Effects]

The composition of the present invention can deliver a polypeptide having epithelial or endothelial cell growth promoting ability or a substance for increasing activity or expression thereof in a sustained manner with high efficiency, thereby treating the wound with excellent efficiency.

The composition of the present invention may release a polypeptide having epithelial or endothelial cell growth promoting ability or a substance for increasing activity or expression thereof in a sustained manner, so as to maintain the polypeptide having epithelial or endothelial cell growth promoting ability or the substance for increasing activity or expression thereof *in vivo* for a long time, thereby exhibiting excellent medicinal effects.

[Brief Description of Drawings]

FIG. 1 is micrographs of porous silica particles according to one embodiment of the present invention.

FIG. 2 is micrographs of porous silica particles according to one embodiment of the present invention.

FIG. 3 is micrographs of small pore particles obtained in a manufacturing process of the porous silica particles according to one embodiment of the present invention.

FIG. 4 is micrographs of the small pore particles according to one embodiment of the present invention.

FIG. 5 is micrographs of the porous silica particles for each pore diameter according to one embodiment of the present invention.

DDV (Degradable Delivery Vehicle) is the particles according to an embodiment, wherein the number in parenthesis means the diameter of the particle and the number of subscripts means the pore diameter. For example, $DDV(200)_{10}$ refers to a particle having a particle diameter (that is, particle size) of 200 nm and a pore diameter of 10 nm according to an embodiment.

FIG. 6 is micrographs to identify biodegradability of the porous silica particles according to one embodiment of the present invention.

FIG. 7 a view illustrating a tube having a cylindrical permeable membrane according to one illustrative example.

FIG. 8 is a graph illustrating results of decreasing absorbance of the porous silica particles over time according to one embodiment of the present invention.

FIG. 9 is diagrams illustrating results of decreasing absorbance of the porous silica particles for each particle size over time according to one embodiment of the present invention.

FIG. 10 is diagrams illustrating results of decreasing absorbance of the porous silica particles for each pore diameter over time according to one embodiment of the present invention.

FIG. 11 is a graph illustrating results of decreasing absorbance of the porous silica particles for each pH of the environment over time according to one embodiment of the present invention.

FIG. 12 is a graph illustrating results of decreasing absorbance of the porous silica particles over time according to one embodiment of the present invention.

FIG. 13 is diagrams illustrating results of increased VEGF expression by porous silica particles that carry lactoferrin protein or a gene encoding the same according to one embodiment of the present invention.

FIG. 14 a graph illustrating results of inducing cell proliferation in the porous silica particles that carry lactoferrin protein or a gene encoding the same according to one embodiment of the present invention.

FIG. 15 is diagrams illustrating results of inducing cell proliferation and angiogenesis in the porous silica particles that carry lactoferrin protein or a gene encoding the same according to one embodiment of the present invention.

FIGS. 16 to 19 are diagrams illustrating wound healing results by the porous silica particles that carry lactoferrin protein or a gene encoding the same according to one embodiment of the present invention.

[Mode for Carrying out Invention]

In the detailed description of the present invention, specific meanings of terms are defined, however, substantially accepted as common meanings understood by those skilled in the art and not intended to be limited to the specific meanings defined below.

The pharmaceutical composition for treating wounds according to the present invention may include porous silica particles on which a polypeptide having epithelial or endothelial cell growth promoting ability or a substance for increasing activity or expression thereof is supported.

The polypeptide having epithelial or endothelial cell growth promoting ability may be used without limitation as long as it has epithelial cell or endothelial cell growth promoting ability, for example, may be lactoferrin, cell growth factors, and the like, wherein the cell growth factors may specifically be an epithelial cell growth factor, a hepatocyte growth factor or a vascular endothelial growth factor, but it is not limited thereto.

Polypeptides having epithelial or endothelial cell growth-promoting ability may be derived from the same species as a subject to be administered, but it is not limited thereto. Further, even polypeptides derived from other species may be used as long as they have the same/similar functions depending on similarity between sequences. For example, if the subject to be administered is a human, the lactoferrin may include an amino acid sequence of SEQ ID NO: 1 derived from the human, and specifically, may be composed of the sequence of SEQ ID NO: 1. Further, the epithelial cell growth factor may include an amino acid sequence of SEQ ID NO: 2, the hepatocyte growth factor may include an amino acid sequence of SEQ ID NO: 3, and the vascular endothelial growth factor may include an amino acid sequence of SEQ ID NO: 4, specifically, these plasmids may be composed of the above corresponding sequences, respectively.

A substance for increasing activity or expression of the polypeptide having epidermal or endothelial cell growth promoting ability is not limited as long as it can increase the activity or expression descried above. For example, the substance may be a plasmid carrying a gene that encodes the polypeptide having epidermal or endothelial cell growth promoting ability.

Plasmids known in the art may be used without limitation. For example, the plasmid carrying a lactoferrin gene may include a nucleotide sequence of SEQ ID NO: 5, the plasmid carrying an epidermal growth factor may include a nucleotide sequence of SEQ ID NO: 6, the plasmid carrying a hepatocyte growth factor may include a nucleotide sequence of SEQ ID NO: 7, and the plasmid carrying a vascular endothelial growth factor may include a nucleotide sequence of SEQ ID NO: 8, specifically, these plasmids may be composed of the above corresponding sequences, respectively.

The subject to be administrated may include entire mammals including a human, and in particular, may be human, but it is not limited thereto.

In the present invention, the term "wound" means that the tissue is cut, torn, broken, burned, traumatized, etc., or refers to injury to a human body derived from a disorder or disease causing such injury. The wound may be an open wound with an open surface, or a closed wound with no open surface. One example of such wounds may be an open wound in the skin. The open wound may include lesions, sores, necrosis and ulcers. The necrosis may be relevant to dead tissues resulting from infection, injury or infarction. The ulcer may be a local defect or dent in the surface of an organ or tissue, occurred by stripping of necrotic tissue.

One example of such wounds may be wounds with damaged epidermis; dermis; epidermis and dermis; or epidermis, dermis and subcutaneous fat layer of the skin.

Further, the above wounds may include, for example, injuries, hypertrophic scars, keloids, cuts, incisions (e.g., surgical incisions), abrasions, lacerations, fractures, contusions, burns or amputations.

Injury healing or treatment means inhibiting development of skin damage, reduction of skin damage, or removal of skin damage. The injury specifically includes burns, ulcers, traumas, post-surgical, childbirth, chronic wounds, or damage due to dermatitis.

The porous silica particles of the present invention are particles based on silica ($SiO_2$) material and have a nano-scale particle size.

The porous silica nanoparticles of the present invention are porous particles, each of which has nano-scale pores and can carry a polypeptide having epithelial or endothelial cell growth promoting ability or a substance for increasing activity or expression thereof on a surface thereof and/or an inside of the pores.

The porous silica particles of the present invention are biodegradable particles, which carry a polypeptide having epithelial or endothelial cell growth promoting ability or a substance for increasing activity or expression thereof and, can release the same, that is, the polypeptide having epithelial or endothelial cell growth promoting ability or the substance for increasing activity or expression thereof while being biodegraded in the body when administered to the body. Specifically, the porous silica particles of the present invention may be slowly degraded in the body, so as to release the polypeptide having epithelial or endothelial cell growth promoting ability or the substance for increasing activity or expression thereof in a sustained manner. For example, "t", at which a ratio of absorbance of the following Equation 1 becomes 1/2, is 24 or more:

[Equation 1]

$$A_t/A_0$$

(wherein $A_0$ is absorbance of the porous silica particles measured by putting 5 ml of suspension containing 1 mg/ml of porous silica particles into a cylindrical permeable membrane having pores with a pore diameter of 50 kDa,
15 ml of the same solvent as the suspension comes into contact with an outside of the permeable membrane, and the inside/outside of the permeable membrane are horizontally stirred at 60 rpm and at 37 °C,
pH of the suspension is 7.4, and
$A_t$ indicates absorbance of the porous silica particle measured after lapse of "t" hours since $A_o$ was measured).

The above Equation 1 means what a rate the porous silica particles are degraded in an environment similar to the body.

As shown in FIG. 7, for example, absorbances $A_0$ and $A_t$ in the above Equation 1 may be measured after placing porous silica particles and a suspension in a cylindrical permeable membrane and also placing the same suspension outside the permeable membrane.

The porous silica particles of the present invention are biodegradable, and may be slowly degraded in the suspension. The diameter of 50 kDa corresponds to about 5 nm, which allows biodegradable porous silica particles to pass through a permeable membrane having a diameter of 50 kDa, and a cylindrical permeable membrane is under horizontal agitation at 60 rpm to evenly blend the suspension, such that the degraded porous silica particles can come out of the permeable membrane.

The absorbance in the above Equation 1 may be measured, for example, under an environment in which the suspension outside the permeable membrane is replaced with a new suspension. The suspension may be continuously replaced, or replaced every period wherein the period is periodic or irregular. For example, the suspension may be replaced at 1 hour interval, 2 hours interval, 3 hours interval, 6 hours interval, 12 hours interval, 24 hours interval, 2 days interval, 3 days interval, 4 days interval, 7 days interval, etc., within a range of 1 hour to 1 week, but it is not limited thereto.

The absorbance ratio of 1/2 means that the absorbance is half of the initial absorbance after t hours, that is, that approximately half of the porous silica particles are degraded.

The suspension may be a buffer solution, for example, at least one selected from the group consisting of phosphate buffered saline (PBS) and simulated body fluid (SBF), and more specifically, PBS.

"t" in the above Equation 1 of the present invention, at which the absorbance ratio becomes 1/2, may be 24 or more, for example, t may range from 24 to 120. That is, within the above range, t may range from 24 to 96, 24 to 72, 30 to 70, 40 to 70, 50 to 65, etc., but it is not limited thereto.

With regard to the porous silica particles of the present invention, t at which the absorbance ratio in the above Equation 1 becomes 1/5 may range from 70 to 140. For example, t may range from 80 to 140, 80 to 120, 80 to 110, 70 to 140, 70 to 120, 70 to 110, etc. within the above range, but it is not limited thereto.

With regard to the porous silica particles of the present invention, t at which the absorbance ratio in the above Equation 1 becomes 1/20 may range from 130 to 220. For example, t may range from 130 to 200, 140 to 200, 140 to 180, 150 to 180, etc. within the above range, but it is not limited thereto.

With regard to the porous silica particles of the present invention, t at which the absorbance ratio in the above Equation 1 becomes 0.01 or less may be 250 or more. For example, t may be 300 or more, 350 or more, 400 or more, 500 or more, 1000 or more, etc. and the upper limit may be 2000, but it is not limited thereto.

With regard to the porous silica particles of the present invention, the absorbance ratio and t in the above Equation 1 have high positive correlation. For example, Pearson correlation coefficient may be 0.8 or more, and for example, 0.9 or more and 0.95 or more.

"t" in the above Equation 1 means how fast the porous silica particles are degraded under the environment similar to the body. That is, t may be regulated by adjusting, for example, a surface area, a particle size, a pore diameter, substituents on the surface of the porous silica particles and/or the inside of the pores, compactness of the surface and the like.

For example, the surface area of the particles may be increased to reduce t, or the surface area may be decreased to increase t. The surface area may be regulated by adjusting the particle size and the pore diameter of the particles. Further, if direct exposure of the porous silica particles to the environment (such as solvents) is reduced by placing substituents on the surface of the particles and/or the inside of the pores, t may be increased. Further, when the porous silica particles support or carry the polypeptide having epithelial or endothelial cell growth promoting ability or the substance for increasing activity or expression thereof, and when increasing affinity between the polypeptide having epithelial or endothelial cell growth promoting ability or the substance for increasing activity or expression thereof and the porous silica particles, direct exposure of the porous silica particles to the environment may be reduced, thereby increasing t. In addition, t may be increased by preparing the particles with more compact surface. As described above, various examples of adjusting t in the above Equation 1 have been described, but it is not limited thereto.

The porous silica particles of the present invention may have a spherical shape, but it is not limited thereto.

The porous silica particles of the present invention may have an average diameter of, for example, 150 to 1000 nm. For example, the average diameter may range from 150 to 800 nm, 150 to 500 nm, 150 to 400 nm, 150 to 300 nm, and 150 to 200 nm, etc. within the above range, but it is not limited thereto.

The porous silica particles of the present invention may have an average pore diameter of, for example, 1 to 100 nm. For example, the average diameter may range from 5 to 100 nm, 7 to 100 nm, 7 to 50 nm, 10 to 50 nm, 10 to 30 nm, 7 to 30 nm, etc., within the above range, but it is not limited thereto. The porous silica particles having a large diameter as described above may carry a large amount of polypeptide having epithelial or endothelial cell growth promoting ability (hereinafter, often abbrev. to "cell growth promoting polypeptide") or a substance for increasing activity or expression thereof, or may further carry a large-sized polypeptide having epithelial or endothelial cell growth promoting ability or a substance for increasing activity or expression thereof.

The porous silica particles of the present invention may have a BET surface area of, for example, 200 to 700 $m^2/g$. For example, the BET surface area may range from 200 to 700 $m^2/g$, 200 to 650 $m^2/g$, 250 to 650 $m^2/g$, 300 to 700 $m^2/g$, 300 to 650 $m^2/g$, 300 to 600 $m^2/g$, 300 to 550 $m^2/g$, 300 to 500 $m^2/g$, 300 to 450 $m^2/g$, etc. within the above range, but it is not limited thereto.

Porous silica nanoparticles of the present invention may have a volume per gram, for example, 0.7 to 2.2 ml. For example, the volume may range from 0.7 to 2.0 ml, 0.8 to 2.2 ml, 0.8 to 2.0 ml, 0.9 to 2.0 ml, 1.0 to 2.0 ml, etc. within the above range, but it is not limited thereto. If the volume per gram is too small, a degradation rate may be too high. Further, it is difficult to manufacture excessively large particles or particles having an intact shape.

The porous silica particles of the present invention may have hydrophilic substituents and/or hydrophobic substituents on an outer surface thereof and/or an inside of the pores. For example, only hydrophilic substituents or only hydrophobic substituents may exist on both the surface of the particles and inside of the pores, hydrophilic substituents or hydrophobic substituents may be present on either the surface of the particles or the inside of the pores, or hydrophilic substituents may be present on the surface of the particles while hydrophobic substituents may exist inside of the pores, or vice versa.

Release of the cell growth promoting polypeptide or the substance for increasing activity or expression thereof supported on the porous silica particles according to the present invention is mainly performed by degradation of nanoparticles. Specifically, interaction of the porous silica particles with the release environment of the cell growth promoting polypeptide or the substance for increasing activity or expression thereof is adjusted to regulate a degradation rate of the nanoparticles, so that a release rate of the cell growth promoting polypeptide or the substance for increasing activity or expression thereof can be regulated. Alternatively, the cell growth promoting polypeptide or the substance for increasing activity or expression thereof may be diffused and released from the nanoparticles, wherein adjusting substituents may regulate a binding force of the cell growth promoting polypeptide or the substance for increasing activity or expression thereof to the nanoparticles, thereby controlling release of the cell growth promoting polypeptide or the substance for increasing activity or expression thereof.

Further, in order to increase a binding force of the porous silica particle to a poorly soluble (hydrophobic) polypeptide having epithelial or endothelial cell growth promoting ability or a substance for increasing activity or expression thereof, hydrophobic substituents may be present inside the pores of the particle. Further, in aspects of easy use and formulation, the surface of the particle may also be treated to have hydrophilic substituents.

The hydrophilic substituents may include, for example, hydroxyl group, carboxy group, amino group, carbonyl group, sulfhydryl group, phosphate group, thiol group, ammonium group, ester group, imide group, thioimide group, keto group, ether group, indene group, sulfonyl group, polyethyleneglycol group and the like. Further, the hydrophobic substituent may include, for example, substituted or unsubstituted C1 to C30 alkyl group, substituted or unsubstituted C3 to C30 cycloalkyl group, substituted or unsubstituted C6 to C30 aryl group, substituted or unsubstituted C2 to C30 heteroaryl group, halogen group, C1 to C30 ester group, halogen-containing group and the like.

Further, the porous silica particles of the present invention may be positively charged, negatively charged and/or uncharged at an outer surface thereof and/or an inside of the pores. For example, both the surface of the particle and the inside of the pores may be positively charged or negatively charged, and only the surface of the particles or the inside of the pores may be positively charged or negatively charged. Alternatively, the surface of the particle may be positively charged while the inside of the pores may be negatively charged or vice versa. Specifically, when the porous silica particles carry a polypeptide, the outer surface of the particles and/or the inside of the pores may be negatively charged. Alternatively, when a plasmid loaded with a gene encoding a polypeptide is supported on the porous silica particles, the outer surface of the particles and/or the inside of the pores may be positively charged.

The charging may be performed, for example, by the presence of a cationic substituent or an anionic substituent.

The cationic substance may include, for example, an amino group or any other nitrogen-containing group. Further, the anionic substituent may include, for example, carboxy group (-COOH), sulfonic acid group ($-SO_3H$), thiol group (-SH), etc. as an acidic group, but it is not limited thereto.

Likewise, when interaction of the porous silica particles with release environment of the cell growth promoting polypeptide or the substance for increasing activity or expression thereof is regulated by adjusting the substituents through charging, a degradation rate of nanoparticles may be regulated to control a release rate of the cell growth promoting polypeptide or the substance for increasing activity or expression thereof. Further, the cell growth promoting polypeptide or the substance for increasing activity or expression thereof may also be diffused and released from the nanoparticles. In this regard, adjusting the substituents may regulate a binding force of the cell growth promoting polypeptide or the substance for increasing activity or expression thereof to the nanoparticles, thereby controlling release of the cell growth promoting polypeptide or the substance for increasing activity or expression thereof.

In addition, the porous silica particles of the present invention may include substituents for the purposes of: supporting the cell growth promoting polypeptide or the substance for increasing activity or expression thereof on the surface of the particles and/or inside the pores; delivery of the cell growth promoting polypeptide or the substance for increasing activity or expression thereof; supporting other substances for other purposes; or binding of additional substituents. Further, the porous silica particles may also include antibodies, ligands, cell permeable peptides, or aptamers bound thereto.

The substituents on the surface of the particles and/or the inside of the pores, charge, binders, etc. described above may be added by, for example, surface modification.

Surface modification may be performed, for example, by reacting a compound having a substituent to be introduced with the particles, wherein the compound may be, for example, alkoxysilane having C1 to C10 alkoxy group, but it is not limited thereto. The alkoxysilane has one or more alkoxy groups, for example, 1 to 3 alkoxy groups. Further, there may be a substituent to be introduced into a site where the alkoxy group is not bound, or a substituent substituted with the same.

The porous silica particles of the present invention may be manufactured, for example, through small pore particle preparation and pore expansion processes and, if necessary, may be manufactured further through calcination, or surface modification process and the like. If both the calcination and the surface modification processes have been implemented, the particles may be surface-modified after calcination.

The small pore particles may be, for example, particles having an average pore diameter of 1 to 5 nm.

The small pore particles may be harvested by adding a surfactant and a silica precursor in a solvent, followed by agitation and homogenization.

The solvent may be water and/or an organic solvent, and the organic solvent may include, for example: ethers such as 1,4-dioxane (particularly cyclic ethers); halogenated hydrocarbons such as chloroform, methylene chloride, carbon tetrachloride, 1,2-dichloroethane, dichloroethylene, trichloroethylene, perchloroethylene, dichloropropane, amyl chloride, 1,2-dibromoethane, etc.; ketones such as acetone, methylisobutylketone, γ-butyrolactone, 1,3-dimethyl-imidazolidinone, methylethylketone, cyclohexanone, cyclopentanone, 4-hydroxy-4-methyl-2-pentanone, etc.; aromatic carbon-based materials such as benzene, toluene, xylene, tetramethylbenzene, etc.; alkyl amides such as N,N-dimethylformamide, N,N-dibutylformamide, N,N-dimethylacetamide, N-methylpyrrolidone, etc.; alcohols such as methanol, ethanol, propanol, butanol, etc.; glycol ethers (cellosolve) such as ethyleneglycol monoethyl ether, ethyleneglycol monomethyl ether, ethyleneglycol monobutyl ether, diethyleneglycol monoethyl ether, diethyleneglycol monomethyl ether, diethyleneglycol monobutyl ether, propyleneglycol monomethyl ether, propyleneglycol monoethyl ether, dipropyleneglycol diethyl ether, triethyleneglycol monoethyl ether, etc.; others such as dimethylacetamide (DMAc), N,N-diethylacetamide, dimethylformamide (DMF), diethylformamide (DEF), N,N-dimethylacetamide (DMAc), N-methylpyrrolidone (NMP), N-ethylpyrrolidone (NEP), 1,3-dimethyl-2-imidazolidinone, N,N-dimethylmethoxyacetamide, dimethyl sulfoxide, pyridine, dimethyl sulfone, hexamethylphosphoamide, tetramethylurea, N-methylcarrolactam, tetrahydrofuran, m-dioxane, P-dioxane, 1,2-dimethoxyethane and the like. Specifically, alcohol, more specifically methanol may be used, but it is not limited thereto.

When using a mixed solvent of water and the organic solvent, a relative ratio of water and organic solvent may be, for example, in a volume ratio of 1:0.7 to 1.5, for example, 1:0.8 to 1.3, but it is not limited thereto.

The surfactant may include, for example, cetyltrimethylammonium bromide (CTAB), hexadecyltrimethylammonium bromide (TMABr), hexadecyltrimethylpyridinium chloride (TMPrCl), tetramethylammonium chloride (TMACl), etc., and specifically, CTAB may be used.

The surfactant may be added, for example, in an amount of 1 to 10 g, for example, 1 to 8 g, 2 to 8 g or 3 to 8 g per liter of solvent, but it is not limited thereto.

The silica precursor may be added after stirring with addition of a surfactant to the solvent. The silica precursor may be, for example, tetramethyl orthosilicate (TMOS), but it is not limited thereto.

The stirring may be conducted, for example, for 10 to 30 minutes, but it is not limited thereto

The silica precursor may be added in an amount of 0.5 to 5 ml per liter of solvent, for example, 0.5 ml to 4 ml, 0.5 to 3 ml, 0.5 to 2 ml, 1 to 2 ml, etc. within the above range, but it is not limited thereto.

If necessary, sodium hydroxide may further be used as a catalyst, specifically, and may be added under stirring after addition of the surfactant and before addition of the silica precursor to the solvent.

The sodium hydroxide may be added in an amount of 0.5 to 8 ml per liter of solvent, for example, 0.5 to 5 ml, 0.5 to 4 ml, 1 to 4 ml, 1 to 3 ml, 2 to 3 ml, etc. within the above range with respect to 1 M aqueous sodium hydroxide solution, but it is not thereto.

After addition of the silica precursor, the solution may be reacted with stirring. The stirring may be conducted for 2 to 15 hours, for example, 3 to 15 hours, 4 to 15 hours, 4 to 13 hours, 5 to 12 hours, 6 to 12 hours, 6 to 10 hours, etc. within the above range, but it is not limited thereto. If the stirring time (reaction time) is too short, nucleation may be insufficient.

After agitation, the solution may be aged. Aging may be performed for 8 to 24 hours, for example, for 8 to 20 hours, 8 to 18 hours, 8 to 16 hours, 8 to 14 hours, 10 to 16 hours, 10 to 14 hours, etc. within the above range, but it is not limited thereto.

Thereafter, the reaction product may be washed and dried to harvest porous silica particles and, if necessary, separation of unreacted material may proceed before washing.

Separation of the unreacted material may be implemented by separating the supernatant, for example, through centrifugation. For example, centrifugation may be conducted at 6,000 to 10,000 rpm, and the centrifugation time may range from 3 to 60 minutes, for example, 3 to 30 minutes, 3 to 30 minutes, 5 to 30 minutes, etc. within the above range, but it is not limited thereto.

The washing may be conducted with water and/or an organic solvent. Specifically, since different substances are dissolved in different solvents, water and the organic solvent may be used alternately once or several times, or the washing may be conducted with water or the organic solvent alone once or several times. The several times described above may be 2 times or more and 10 times or less, for example, 3 times or more and 10 times or less, 4 times or more and 8 times or less, 4 times or more and 6 times or less.

The organic solvent may include, for example: ethers such as 1,4-dioxane (particularly cyclic ethers); halogenated hydrocarbons such as chloroform, methylene chloride, carbon tetrachloride, 1,2-dichloroethane, dichloroethylene, trichloroethylene, perchloroethylene, dichloropropane, amyl chloride, 1,2-dibromoethane, etc.; ketones such as acetone, methylisobutylketone, γ-butyrolactone, 1,3-dimethyl-imidazolidinone, methylethylketone, cyclohexanone, cyclopentanone, 4-hydroxy-4-methyl-2-pentanone, etc.; aromatic carbon-based materials such as benzene, toluene, xylene, tetramethylbenzene, etc.; alkyl amides such as N,N-dimethylformamide, N,N-dibutylformamide, N,N-dimethylacetamide, N-methylpyrrolidone, etc.; alcohols such as methanol, ethanol, propanol, butanol, etc.; glycol ethers (cellosolve) such as ethyleneglycol monoethyl ether, ethyleneglycol monomethyl ether, ethyleneglycol monobutyl ether, diethyleneglycol monoethyl ether, diethyleneglycol monomethyl ether, diethyleneglycol monobutyl ether, propyleneglycol monomethyl ether, propyleneglycol monoethyl ether, dipropyleneglycol diethyl ether, triethyleneglycol monoethyl ether, etc.; others such as dimethylacetamide (DMAc), N,N-diethylacetamide, dimethylformamide (DMF), diethylformamide (DEF), N,N-dimethylacetamide (DMAc), N-methylpyrrolidone (NMP), N-ethylpyrrolidone (NEP), 1,3-dimethyl-2-imidazolidinone, N,N-dimethylmethoxyacetamide, dimethyl sulfoxide, pyridine, dimethyl sulfone, hexamethylphosphoamide, tetramethylurea, N-methylcarrolactam, tetrahydrofuran, m-dioxane, P-dioxane, 1,2-dimethoxyethane and the like. Specifically, alcohol, more specifically methanol may be used, but it is not limited thereto.

The washing may be conducted under centrifugation, for example, at 6,000 to 10,000 rpm, and the centrifugation time may range from 3 to 60 minutes, for example, 3 to 30 minutes, 3 to 30 minutes, 5 to 30 minutes, etc. within the above range, but it is not limited thereto.

Alternatively, the washing may be conducted by filtering out particles through a filter without centrifugation. The filter may have pores in a size of less than or equal to the diameter of the porous silica particles. When filtering the reaction solution with such a filter as described above, only particles remain on the filter, which may be washed by pouring water and/or an organic solvent on the filter.

In the washing, water and the organic solvent may be used alternately once or several times, or the washing may be conducted with water or the organic solvent alone once or several times. The several times described above may be 2 times or more and 10 times or less, for example, 3 times or more and 10 times or less, 4 times or more and 8 times or less, 4 times or more and 6 times or less.

The drying may be conducted, for example, at 20 to 100 °C, but it is not limited thereto, and may also be conducted in a vacuum state.

Thereafter, the pore of the harvested porous silica particles may be expanded, and such pore expansion may be conducted using a pore swelling agent.

The pore swelling agent may include, for example, trimethylbenzene, triethylbenzene, tripropylbenzene, tributylbenzene, tripentylbenzene, trihexylbenzene, toluene, benzene, etc., and specifically, trimethylbenzene may be used, but it is not limited thereto.

Further, the pore swelling agent used herein may be, for example, N, N-dimethylhexadecylamine (DMHA), but it is not limited thereto.

The pore expansion may be performed, for example, by mixing the porous silica particles in the solvent with a pore swelling agent and heating the mixture to induce reaction.

The solvent may be water and/or an organic solvent, and the organic solvent may include, for example: ethers such as 1,4-dioxane (particularly cyclic ethers); halogenated hydrocarbons such as chloroform, methylene chloride, carbon tetrachloride, 1,2-dichloroethane, dichloroethylene, trichloroethylene, perchloroethylene, dichloropropane, amyl chloride, 1,2-dibromoethane, etc.; ketones such as acetone, methylisobutylketone, cyclohexanone, etc.; aromatic carbon-based materials such as benzene, toluene, xylene, etc.; alkyl amides such as N,N-dimethylformamide, N,N-dibutylformamide, N,N-dimethylacetamide, N-methylpyrrolidone, etc.; alcohols such as methanol, ethanol, propanol, butanol and the like. Specifically, alcohol, more specifically methanol may be used, but it is not limited thereto.

The porous silica particles may be added in a ratio of 10 to 200 g per liter of solvent, for example, 10 to 150 g, 10 to 100 g, 30 to 100 g, 40 to 100 g, 50 to 100 g, 50 to 80 g, 60 to 80 g, etc., within the above range, but it is not limited thereto.

The porous silica particles may be evenly dispersed in a solvent. For example, the porous silica particles may be added to the solvent and ultrasonically dispersed. In the case of using a mixed solvent, the porous silica particles may be dispersed in a first solvent, followed by adding a second solvent thereto.

The pore swelling agent may be added in a ratio of 10 to 200 parts by volume ("vol. parts") to 100 vol. parts of solvent, for example, 10 to 150 vol. parts, 10 to 100 vol. parts, 10 to 80 vol. parts, 30 to 80 vol. parts, 30 to 70 vol. parts, etc. within the above range, but it is not limited thereto.

The reaction may be carried out at 120 to 190 °C, for example, 120 to 190 °C, 120 to 180 °C, 120 to 170 °C, 130 to 170 °C, 130 to 160 °C, 130 to 150 °C, 130 to 140 °C, etc. within the above range, but it is not limited thereto.

The reaction may be carried out for 6 to 96 hours, for example, 30 to 96 hours, 30 to 96 hours, 30 to 80 hours, 30 to 72 hours, 24 to 80 hours, 24 to 72 hours, 36 to 96 hours, 36 to 80 hours, 36 to 72 hours, 36 to 66 hours, 36 to 60 hours, 48 to 96 hours, 48 to 88 hours, 48 to 80 hours, 48 to 72 hours, 6 to 96 hours, 7 to 96 hours, 8 to 80 hours, 9 to 72 hours, 9 to 80 hours, 6 to 72 hours, 9 to 96 hours, 10 to 80 hours, 10 to 72 hours, 12 to 66 hours, 13 to 60 hours, 14 to 96 hours, 15 to 88 hours, 16 to 80 hours, 17 to 72 hours, etc. within the above range, but it is not limited thereto.

The time and temperature may be desirably adjusted within the ranges exemplified above so that the reaction may be carried out sufficiently but not excessively. For example, when the reaction temperature is reduced, the reaction time may be increased, and when the reaction temperature is increased, the reaction time may be shortened. If the reaction is not sufficiently performed, pore expansion may be insufficient. On the other hand, if the reaction proceeds excessively, the particles may collapse due to overexpansion of the pores.

The reaction may be carried out, for example, by gradually raising the temperature. Specifically, the reaction may be carried out by gradually raising the temperature at a rate of 0.5 to 15 °C/min from the room temperature to the above-defined temperature. For example, the temperature may be raised at a rate of 1 to 15 °C/min, 3 to 15 °C/min, 3 to 12 °C/min, 3 to 10 °C/min, etc., but it is not limited thereto.

The reaction may be carried out under stirring. For example, the stirring may be implemented at a speed of 100 rpm or more, and specifically, at a speed of 100 to 1000 rpm, but it is not limited thereto.

After the reaction, the reaction solution may be cooled slowly, for example, by gradually decreasing the temperature. Specifically, the reaction may be carried out by gradually decreasing the temperature at a rate of 0.5 to 20 °C/min from the above-defined temperature to room temperature. For example, the temperature may be decreased at a rate of 1 to 20 °C/min, 3 to 20 °C/min, 3 to 12 °C/min, 3 to 10 °C/min, etc. within the above range, but it is not limited thereto.

After cooling, the reaction product may be washed and dried to harvest porous silica particles having expanded pores. If necessary, unreacted material may be first separated before washing.

Separation of the unreacted material may be implemented by separating the supernatant, for example, through centrifugation. Herein, centrifugation may be conducted, for example, at 6,000 to 10,000 rpm, and the centrifugation time may range from 3 minutes to 60 minutes. For example, the centrifugation may be conducted for 3 to 30 minutes, 5 to 30 minutes, etc. within the above range, but it is not limited thereto.

The washing may be conducted with water and/or an organic solvent. Specifically, since different substances are dissolved in different solvents, water and the organic solvent may be used alternately once or several times, or the washing may be conducted with water or the organic solvent alone once or several times. The several times described above may be 2 times or more and 10 times or less, for example, 3 times, 4 times, 5 times, 6 times, 7 times, 8 times, etc.

The organic solvent may include, for example: ethers such as 1,4-dioxane (particularly cyclic ethers); halogenated hydrocarbons such as chloroform, methylene chloride, carbon tetrachloride, 1,2-dichloroethane, dichloroethylene, trichloroethylene, perchloroethylene, dichloropropane, amyl chloride, 1,2-dibromoethane, etc.; ketones such as acetone, methylisobutylketone, cyclohexanone, etc.; aromatic carbon-based materials such as benzene, toluene, xylene, etc.; alkyl amides such as N,N-dimethylformamide, N,N-dibutylformamide, N,N-dimethylacetamide, N-methylpyrrolidone, etc.; alcohols such as methanol, ethanol, propanol, butanol and the like. Specifically, alcohol, more specifically methanol may be used, but it is not limited thereto.

The washing may be conducted under centrifugation, for example, at 6,000 to 10,000 rpm, and the centrifugation time may range from 3 to 60 minutes, for example, 3 to 30 minutes, 3 to 30 minutes, 5 to 30 minutes, etc. within the above range, but it is not limited thereto.

Alternatively, the washing may be conducted by filtering out particles through a filter without centrifugation. The filter may have pores in a size of less than or equal to the diameter of the porous silica particles. When filtering the reaction solution with such a filter as described above, only particles remain on the filter, which may be washed by pouring water and/or an organic solvent on the filter.

In the washing, water and the organic solvent may be used alternately once or several times, or the washing may be conducted with water or the organic solvent alone once or several times. The several times described above may be 2 times or more and 10 times or less, for example, 3 times or more and 10 times or less, 4 times or more and 8 times or less, 4 times or more and 6 times or less.

The drying may be conducted, for example, at 20 to 100 °C, but it is not limited thereto, and may also be conducted in a vacuum state.

Thereafter, the harvested particles may be subjected to calcination, which is a process of heating the particles to remove silanol groups present on the surface of the particles and inside of the pores so as to reduce reactivity of the particles, provide a more compact structure, and remove organic matter filling the pores. For example, the particles may be heated to a temperature of 400 °C or higher. The upper limit of the temperature is not particularly limited but may be 1000 °C, 900 °C, 800 °C, 700 °C, etc. The heating may be conducted, for example, for 3 hours or more, 4 hours or more. The upper limit of the heating time is not particularly limited but may be 24 hours, 12 hours, 10 hours, 8 hours, 6 hours, 5 hours, etc. More particularly, the heating may be conducted at 400 to 700 °C for 3 to 8 hours or at 500 to 600 °C for 4 to 5 hours, but it is not limited thereto.

Removing the organic matter filling the pores can prevent some problems of cytotoxicity or foaming caused by the remaining organic matter.

Then, the harvested porous silica particles may be subjected to surface modification, and the surface modification may be performed on the surface of the particles and/or the inside of the pores. Both the particle surface and the inside of the pores may be surface-modified in the same manner, or may be surface-modified differently.

The particles may be charged or have hydrophilic and/or hydrophobic properties through surface modification.

More specifically, in order to effectively support the polypeptide having epithelial or endothelial cell growth promoting ability (that is, the cell growth promoting polypeptide) and the substance for increasing activity or expression thereof, surface modification of the porous silica particles may be performed by having at least one substituent selected from the group consisting of amino, aminoalkyl, alkylamino, heterocyclic aromatic compound group containing a nitrogen atom, cyan and guanidine groups.

Surface modification may be performed, for example, by reacting a compound having a hydrophilic, hydrophobic, cationic or anionic substituent to be introduced with the particles, wherein the compound may be, for example, alkoxysilane having a C1 to C10 alkoxy group, but it is not limited thereto.

The alkoxysilane has one or more alkoxy groups, for example, 1 to 3 alkoxy groups. Further, there may be a substituent to be introduced into a site where the alkoxy group is not bound, or a substituent substituted with the same.

When alkoxysilane reacts with the porous silica particles, a covalent bond is formed between a silicon atom and an oxygen atom so that the alkoxysilane may be bound to the surface of the silicone particles and/or the inside of the pores. Since the alkoxysilane has a substituent to be introduced, the corresponding substituent may be introduced into the surface of the porous silica particles and/or the inside of the pores.

The reaction may be carried out by reacting the porous silica particles dispersed in a solvent with alkoxysilane.

The solvent may be water and/or an organic solvent, and the organic solvent may include, for example: ethers such as 1,4-dioxane (particularly cyclic ethers); halogenated hydrocarbons such as chloroform, methylene chloride, carbon tetrachloride, 1,2-dichloroethane, dichloroethylene, trichloroethylene, perchloroethylene, dichloropropane, amyl chloride, 1,2-dibromoethane, etc.; ketones such as acetone, methylisobutylketone, γ-butyrolactone, 1,3-dimethyl-imidazolidinone, methylethylketone, cyclohexanone, cyclopentanone, 4-hydroxy-4-methyl-2-pentanone, etc.; aromatic carbon-based materials such as benzene, toluene, xylene, tetramethylbenzene, etc.; alkyl amides such as N,N-dimethylformamide, N,N-dibutylformamide, N,N-dimethylacetamide, N-methylpyrrolidone, etc.; alcohols such as methanol, ethanol, propanol, butanol, etc.; glycol ethers (cellosolve) such as ethyleneglycol monoethyl ether, ethyleneglycol monomethyl ether, ethyleneglycol monobutyl ether, diethyleneglycol monoethyl ether, diethyleneglycol monomethyl ether, diethyleneglycol monobutyl ether, propyleneglycol monomethyl ether, propyleneglycol monoethyl ether, dipropyleneglycol diethyl ether, triethyleneglycol monoethyl ether, etc.; others such as dimethylacetamide (DMAc), N,N-diethylacetamide, dimethylformamide (DMF), diethylformamide (DEF), N,N-dimethylacetamide (DMAc), N-methylpyrrolidone (NMP), N-ethylpyrrolidone (NEP), 1,3-dimethyl-2-imidazolidinone, N,N-dimethylmethoxyacetamide, dimethyl sulfoxide, pyridine, dimethyl sulfone, hexamethylphosphoamide, tetramethylurea, N-methylcarrolactam, tetrahydrofuran, m-dioxane, P-dioxane, 1,2-dimethoxyethane and the like. Specifically, alcohol, more specifically methanol may be used, but it is not limited thereto.

The positively charging may be performed by reacting the porous silica particles with alkoxysilane having a basic group such as a nitrogen-containing group, for example, an amino group or an aminoalkyl group. Specifically, N-[3-(trimethoxysilyl)propyl]ethylenediamine, N1-(3-trimethoxysilylpropyl)diethylenetriamine, (3-aminopropyl)trimethoxysilane, N-[3-(trimethoxysilyl)propyl]aniline, trimethoxy[3-(methylamino)propyl]silane, 3-(2-aminoethylamino)propyldimethoxymethylsilane, etc. may be used, but it is not limited thereto.

The negatively charging may be performed by reacting the porous silica particles with alkoxysilane having an acidic group such as a carboxyl group, sulfonic acid group, thiol group, methyl phosphonate group, phosphonate group, etc. Specifically, (3-Mercaptopropyl) trimethoxysilane may be used, but it is not limited thereto.

The hydrophilic property may be obtained by reacting the porous silica particles with alkoxysilane having a hydrophilic group, for example, hydroxyl group, carboxy group, amino group, carbonyl group, sulfhydryl group, phosphate group, thiol group, ammonium group, ester group, imide group, thioimide group, keto group, ether group, indene group, sulfonyl group, polyethyleneglycol group and the like. Specifically, N-[3-(trimethoxysilyl)propyl]ethylenediamine, N1-(3-trimethoxysilylpropyl)diethylenetriamine, (3-aminopropyl)trimethoxysilane, (3-mercaptopropyl) trimethoxysilane, trimethoxy[3-(methylamino)propyl]silane, 3-(2-aminoethylamino)propyldimethoxymethylsilane may be used, but it is not limited thereto.

The hydrophobic property may be obtained by reacting the porous silica particles with alkoxysilane having a hydrophobic substituent, for example, substituted or unsubstituted C1 to C30 alkyl group, substituted or unsubstituted C3 to C30 cycloalkyl group, substituted or unsubstituted C6 to C30 aryl group, substituted or unsubstituted C2 to C30 heteroaryl group, halogen group, C1 to C30 ester group, halogen-containing group and the like. Specifically, trimethoxy(octadecyl)silane, trimethoxy-n-octylsilane, trimethoxy(propyl)silane, isobutyl(trimethoxy)silane, trimethoxy(7-octen-1-yl)silane, trimethoxy(3,3,3-trifluoropropyl)silane, trimethoxy(2-phenylethyl)silane, vinyltrimethoxysilane, cyanomethyl, 3-(trimethoxysilyl)propyl]trithiocarbonate, (3-bromopropyl)trimethoxysilane, etc. may be used, but it is not limited thereto.

Further, in order to increase a binding force of the porous silica particles to a poorly soluble (hydrophobic) polypeptide having epithelial or endothelial cell growth promoting ability or a substance for increasing activity or expression thereof through surface modification, hydrophobic substituents may be present inside of the pores of the particle. Further, in aspects of easy use and formulation, the surface of the particles may also be treated to have hydrophilic substituents. In addition, there may be a substituent on the surface of the particles in order to bind another polypeptide having epithelial or endothelial cell growth promoting ability or a substance for increasing activity or expression thereof.

Further, the surface modification may be performed in combination. For example, surface modification may be performed twice or more on the outer surface of the particles or the inside of the pores. As a specific example, a compound including a carboxyl group may be bound to silica particles having amino groups introduced therein through amide bond in order to change the positively-charged particles to have different surface properties, but it is not limited thereto.

The reaction of the porous silica particles with alkoxysilane may be carried out, for example, under heating. The heating may be conducted at 80 to 180 °C, for example, 80 to 160 °C, 80 to 150 °C, 100 to 160 °C, 100 to 150 °C, 110 to 150 °C, etc. within the above range, but it is not limited thereto.

The reaction of the porous silica particles with alkoxysilane may be carried out for 4 to 20 hours, for example, 4 to 18 hours, 4 to 16 hours, 6 to 18 hours, 6 to 16 hours, 8 to 18 hours, 8 to 16 hours, 8 to 14 hours, 10 to 14 hours, etc. within the above range, but it is not limited thereto.

The reaction temperature, time and an amount of the compound used for surface modification may be desirably selected according to an extent of surface modification. In other words, reaction conditions will vary depending on hydrophilic property, hydrophobic property and a level of charge with regard to the polypeptide having epithelial or endothelial cell growth promoting ability or the substance for increasing activity or expression thereof. Specifically, by controlling the hydrophilic property, hydrophobic property and the level of charge of the porous silica particles, a release rate of the cell growth promoting polypeptide or the substance for increasing activity or expression thereof may be controlled. For example, if the cell growth promoting polypeptide or the substance for increasing activity or expression thereof have strong negative charge at neutral pH, the reaction temperature may be raised, the reaction time may be extended or the amount of the treated compound may be increased so as to make the porous silica particles to have strong positive charge, but it is not limited thereto.

Further, the porous silica particles of the present invention may be manufactured through, for example, preparation of small pore particles, pore expansion, surface modification, and internal pore modification.

Preparation of small pore particles and pore expansion may be performed by the above-described processes and, after preparation of the small pore particles and after pore expansion, washing and drying processes may be implemented.

If necessary, unreacted materials may be separated before washing, and separation of the unreacted materials may be conducted by separating the supernatant through centrifugation.

Centrifugation may be conducted at 6,000 to 10,000 rpm, and the centrifugation time may range from 3 to 60 minutes, for example, 3 to 30 minutes, 3 to 30 minutes, 5 to 30 minutes, etc. within the above range, but it is not limited thereto.

The washing after preparation of small pore particles may be conducted by a method/condition within the above-described range, but it is not limited thereto.

The washing after pore expansion may be conducted under more moderate conditions than the above embodiments. For example, washing may be conducted three times or less, but it is not limited thereto.

The surface modification and internal pore modification may be performed by the above-described processes, respectively. Herein, surface modification and then internal pore modification may be performed in this order, and a washing process may be further conducted between the above two processes.

When the washing is conducted in more moderated conditions after preparation of small pore particles and pore expansion, a reaction solution such as a surfactant used for particle production and pore expansion is filled in the pores so that the inside of the pores is not modified during surface modification and, instead, only the surface of the particles may be modified. Thereafter, the reaction solution inside of the pores may be washed out and removed.

Particle washing between the surface modification and the internal pore modification processes may be carried out using water and/or an organic solvent. Specifically, since different substances are dissolved in different solvents, water and the organic solvent may be used alternately once or several times, or the washing may be conducted with water or the organic solvent alone once or several times. The several times described above may be 2 times or more and 10 times or less, for example, 3 times or more and 10 times or less, 4 times or more and 8 times or less, 4 times or more and 6 times or less.

The washing may be carried out under centrifugation, for example at 6,000 to 10,000 rpm, and the centrifugation time may range from 3 to 60 minutes, for example, 3 to 30 minutes, 3 to 30 minutes, 5 to 30 minutes, etc. within the above range, but it is not limited thereto.

Alternatively, the washing may be conducted by filtering out particles through a filter without centrifugation. The filter may have pores in a size of less than or equal to the diameter of the porous silica particles. When filtering the reaction solution with such a filter as described above, only particles remain on the filter, which may be washed by pouring water and/or an organic solvent on the filter.

In the washing, water and the organic solvent may be used alternately once or several times, or the washing may be conducted with water or the organic solvent alone once or several times. The several times described above may be 2 times or more and 10 times or less, for example, 3 times or more and 10 times or less, 4 times or more and 8 times or less, 4 times or more and 6 times or less.

The drying may be conducted, for example, at 20 to 100 °C, but it is not limited thereto, and may also be conducted in a vacuum state.

The polypeptide having epithelial or endothelial cell growth promoting ability, that is, the cell growth promoting polypeptide or the substance for increasing activity or expression thereof may be supported on the surface of the porous silica particles and/or inside the pores. Herein, the supporting may be performed, for example, by mixing porous silica particles in a solvent with the cell growth promoting polypeptide or the substance for increasing activity or expression thereof.

The solvent may be water and/or an organic solvent, and the solvent may include, for example: ethers such as 1,4-dioxane (particularly cyclic ethers); halogenated hydrocarbons such as chloroform, methylene chloride, carbon tetrachloride, 1,2-dichloroethane, dichloroethylene, trichloroethylene, perchloroethylene, dichloropropane, amyl chloride, 1,2-dibromoethane, etc.; ketones such as acetone, methylisobutylketone, cyclohexanone, etc.; aromatic carbon-based materials such as benzene, toluene, xylene, etc.; alkyl amides such as N,N-dimethylformamide, N,N-dibutylformamide, N,N-dimethylacetamide, N-methylpyrrolidone, etc.; alcohols such as methanol, ethanol, propanol, butanol and the like. Specifically, alcohol, more specifically methanol may be used, but it is not limited thereto.

Further, PBS (phosphate buffered saline solution), SBF (simulated body fluid), borate-buffered saline, tris-buffered saline may be used as the solvent.

The cell growth promoting polypeptide or the substance for increasing activity or expression thereof may be gradually released over an extended time. Such sustained release may be continuous or discontinuous, or linear or nonlinear. Further, the release may vary depending on characteristics of the porous silica particles and/or interaction between the porous silica particles and the cell growth promoting polypeptide or the substance for increasing activity or expression thereof.

The cell growth promoting polypeptide or the substance for increasing activity or expression thereof supported on the porous silica particles are released when the porous silica particles are biodegraded. Specifically, the porous silica particles according to the present invention are slowly degraded to allow release of the cell growth promoting polypeptide or the substance for increasing activity or expression thereof in a sustained manner. Such release may be controlled by, for example, adjusting surface area, particle size, pore diameter, substituents on the surface of the particles and/or the inside of the pores, surface compactness, etc. with regard to the porous silica particles, but it is not limited thereto.

The cell growth promoting polypeptide or the substance for increasing activity or expression thereof supported on the porous silica particles may be released while being separated and diffused from the porous silica particles. Since release is influenced by correlation between the porous silica particles, the cell growth promoting polypeptide or the substance for increasing activity or expression thereof, and release environment of the same. Therefore, regulating the correlations may control the release of the cell growth promoting polypeptide or the substance for increasing activity or expression thereof. For example, by enhancing or weakening a binding force of the porous silica particles to the cell growth promoting polypeptide or the substance for increasing activity or expression thereof through surface modification, the release of the cell growth promoting polypeptide or the substance for increasing activity or expression thereof may be controlled.

More specifically, in the case where the supported cell growth promoting polypeptide or substance for increasing activity or expression thereof are poorly soluble (hydrophobic), the surface of the particles and/or the inside of the pores have hydrophobic substituents so as to increase a binding force of the porous silica particles to the cell growth promoting polypeptide or the substance for increasing activity or expression thereof, whereby the cell growth promoting polypeptide or the substance for increasing activity or expression thereof may be released in a sustained manner. For example, the porous silica particles may be surface-modified with alkoxysilane having a hydrophobic substituent.

As used herein, the term "poorly soluble" means to be insoluble, practically insoluble or only slightly soluble (in water), which is a word defined in "Pharmaceutical Science" 18th Edition (issued by U.S.P., Remington, Mack Publishing Company).

The poorly soluble material may have, for example, water solubility of less than 10 g/L, specifically, less than 5 g/L, and more specifically, less than 1 g/L at 1 atmosphere and 25 °C, but it is not limited thereto.

When the supported cell growth promoting polypeptide or substance for increasing activity or expression thereof is water-soluble (hydrophilic), the surface of the particles and/or the inside of the pores have hydrophilic substituents so as to increase a binding force of the porous silica particles to the cell growth promoting polypeptide or the substance for increasing activity or expression thereof, whereby the cell growth promoting polypeptide or the substance for increasing activity or expression thereof may be released in a sustained manner. For example, the porous silica particles may be surface-modified with alkoxysilane having a hydrophilic substituent.

For example, the water-soluble substance may have a water solubility of 10 g/L or more at 1 atmosphere and 25 ° C, but it is not limited thereto.

In the case where the supported cell growth promoting polypeptide or substance for increasing activity or expression thereof is charged, the surface of the particles and/or the inside of the pores are charged with opposite charges, so as to increase a binding force of the porous silica particles to the cell growth promoting polypeptide or the substance for increasing activity or expression thereof, whereby the cell growth promoting polypeptide or the substance for increasing activity or expression thereof may be released in a sustained manner. For example, the porous silica particles may be surface-modified with alkoxysilane having an acidic group or a basic group.

Specifically, if the cell growth promoting polypeptide or the substance for increasing activity or expression thereof is positively charged at neutral pH, the surface of the particles and/or the inside of the pores may be negatively charged at neutral pH so as to increase a binding force of the porous silica particles to the cell growth promoting polypeptide or the substance for increasing activity or expression thereof, whereby the cell growth promoting polypeptide or the substance for increasing activity or expression thereof may be released in a sustained manner. For example, the porous silica particles may be surface-modified with alkoxysilane having an acidic group such as a carboxyl group (-COOH), a sulfonic acid group ($-SO_3H$), etc.

Further, if the cell growth promoting polypeptide or the substance for increasing activity or expression thereof is negatively charged at neutral pH, the surface of the particles and/or the inside of the pores may be positively charged so as to increase a binding force of the porous silica particles to the cell growth promoting polypeptide or the substance for increasing activity or expression thereof, whereby the cell growth promoting polypeptide or the substance for increasing activity or expression thereof may be released in a sustained manner. For example, the porous silica particles may be surface-modified with alkoxysilane having a basic group such as an amino group or any other nitrogen-containing group.

The polypeptide having epithelial or endothelial cell growth promoting ability or the substance for increasing activity or expression thereof may be released for a period of, for example, 7 days to 1 year or more, depending on types of treatment to be required, release environments and types of porous silica particles to be used.

Further, since the porous silica particles of the present invention are 100% biodegradable, the polypeptide having epithelial or endothelial cell growth promoting ability or the substance for increasing activity or expression thereof may be 100% released.

The composition of the present invention has wound healing effects that may stably deliver the polypeptide having epithelial or endothelial cell growth promoting ability or the substance for increasing activity or expression thereof, which is supported on porous silica particles in the body, and may release the same to a target in a sustained manner, thereby promoting expression of factors such as vascular endothelial growth factor (VEFG).

The pharmaceutical composition of the present invention may further include a pharmaceutically acceptable carrier, and may be formulated along with such a carrier. As used herein, the term "pharmaceutically acceptable carrier" refers to a carrier or diluent that does not stimulate the organism and does not inhibit biological activities and properties of the administered compound. Pharmaceutical carriers acceptable in the composition formulated as a liquid solution are sterile and biocompatible, and may include saline, sterile water, Ringer's solution, buffered saline, albumin injectable solutions, dextrose solution, maltodextrin solution, glycerol, ethanol, and a mixture of one or more of these components. Further, if necessary, other typical additives such as antioxidants, buffers and bacteriostatic agents may be added. Diluents, dispersants, surfactants, binders and lubricants may also be added to formulate the pharmaceutical composition into injectable formulations, pills, capsules, granules or tablets such as aqueous solutions, suspensions, emulsions and the like.

The composition of the present invention is applicable in any formulation and may be prepared in oral or parenteral formulations. The pharmaceutical formulations of the present invention may include forms suitable for oral, rectal, nasal, topical (including the cheek and sublingual), subcutaneous, vaginal or parenteral (intramuscular, subcutaneous) administration. Alternatively, forms suitable for administration by inhalation or insufflations may also be included.

The pharmaceutical composition of the present invention is administered in a pharmaceutically effective amount. Effective dose levels may be determined depending on types of disease of the patient, severity, activity of drug, sensitivity to drug, administration time, administration route and rate of release, duration of treatment, factors including concurrent medications, and other factors well known in the medical field. The pharmaceutical composition of the present invention may be administered as an individual therapeutic agent or in combination with other therapeutic agents, may be administered sequentially or simultaneously with conventional therapeutic agents, and may be administered in single or multiple doses. Taking all of the above factors into consideration, it is important to administer the pharmaceutical composition in an amount that can achieve maximum effects with a minimum amount without side effects, which may be easily determined by those skilled in the art.

The dosage of the pharmaceutical composition according to the present invention may vary widely depending on the weight, age, sex, health conditions or diet of a patient, administration time, administration method, excretion rate and severity of the disease, and the appropriate dosage depends on, for example, an amount of drug accumulated in the patient's body and/or specific efficacy of the nucleic acid molecules of the present invention used. Generally, the amount may be calculated on the basis of EC50, which is generally determined to be effective in in *vivo* animal models and *in vitro,* for example, from 0.01 μg to 1 g per kg of body weight. Further, the pharmaceutical composition of the present invention may be administered once or several times per unit time during unit periods of time such as daily, weekly, monthly or yearly, or may be continuously administered using an infusion pump for a long time. The number of repeated administration doses is determined in consideration of a residential time of drug in the body, a drug concentration in the body, etc. Even after treatment according to the course of disease treatment, the composition may be further administered for preventing recurrence, i.e., relapse of the disease.

The pharmaceutical composition of the present invention may further include a compound to maintain/increase one or more of active ingredients exhibiting the same or similar functions in relation to treatment of wounds or the solubility and/or absorption of at least one active ingredient. Further, the composition may also optionally include chemotherapeutic agents, antiinflammatory agents, antiviral agents and/or immunomodulators and the like.

Further, the pharmaceutical composition of the present invention may be formulated using any method known in the art to allow rapid, sustained or delayed release of the active ingredient after administration to a mammal. The formulation may be produced in a form of powders, granules, tablets, emulsions, syrups, aerosols, soft or hard gelatin capsules, sterile injectable solutions, sterile powders.

The present invention also relates to a wound treatment method.

The wound treatment method of the present invention including administering porous silica particles on which a polypeptide having epithelial or endothelial cell growth promoting ability or a substance for increasing activity or expression thereof is supported.

The above polypeptide having epithelial or endothelial cell growth promoting ability or the substance for increasing activity or expression thereof may be within the above-described range.

The porous silica particles may belong to the ranges exemplified above or may be prepared according to the methods/conditions within the ranges exemplified above.

The subject may be a mammal including a human, and specifically a human.

The polypeptide having epithelial or endothelial cell growth promoting ability or the substance for increasing activity or expression thereof may be formulated in a composition form according to the method within the above-described range.

Administration methods are not limited and may include, for example, oral, rectal, nasal, topical (including buccal and sublingual), subcutaneous, vaginal or parenteral (including intramuscular, subcutaneous and intravenous) administration, or administration by inhalation or insufflation.

Further, the present invention relates to use of porous silica particles on which a polypeptide having epithelial or endothelial cell growth promoting ability or a substance for increasing activity or expression thereof is supported in manufacturing a pharmaceutical composition for treatment of wounds.

The above polypeptide having epithelial or endothelial cell growth promoting ability or the substance for increasing activity or expression thereof may be within the above-described range.

The porous silica particles may belong to the ranges exemplified above or may be prepared according to the methods/conditions within the ranges exemplified above.

Hereinafter, the present invention will be described in detail with reference to the following examples. Hereinafter, the porous silica particles of the present invention may be abbreviated as 'DegradaBALL or DDV'.

## Experimental Procedures

Example 1. Porous silica particles (DDV or DegradaBALL)

### 1. Preparation of porous silica particles

#### (1) Preparation of porous silica particles

#### 1) Preparation of small pore particles

960 mL of distilled water (DW) and 810 mL of MeOH were put into a 2 L round bottom flask. 7.88 g of CTAB was added to the flask, followed by rapid addition of 4.52 mL of 1 M NaOH under stirring. After adding a homogeneous mixture while stirring for 10 minutes, 2.6 mL of TMOS was further added. After stirring for 6 hours to mix uniformly, the reaction solution was aged for 24 hours.

Then, the reaction solution was centrifuged at 8000 rpm and 25 °C for 10 minutes to remove the supernatant, centrifuged at 8000 rpm and 25 °C for 10 minutes, and washed five times with ethanol and distilled water alternately.

Thereafter, the resultant product was dried in an oven at 70 °C to harvest 1.5 g of powdery microporous silica particles (pore average diameter of 2 nm and particle size of 200 nm).

#### 2) Pore expansion

1.5 g of microporous silica particle powder was added to 10 ml of ethanol and subjected to ultrasonic dispersion, and 10 ml of water and 10 ml of TMB (trimethyl benzene) were further added, followed by ultrasonic dispersion.

Thereafter, the dispersion was placed in an autoclave and reacted at 160 °C for 48 hours.

The reaction was initiated at 25 °C and performed while raising the temperature at a rate of 10 °C/min, then slowly cooled in an autoclave at a rate of 1 to 10 °C/min.

The cooled reaction solution was centrifuged at 8000 rpm for 10 minutes at 25 °C to remove the supernatant, and centrifuged at 8000 rpm for 10 minutes at 25 °C and washed five times with ethanol and distilled water alternately.

Then, the product was dried in an oven at 70 °C to harvest powdery porous silica particles (pore diameter of 10 to 15 nm, and particle size of 200 nm).

#### 3) Calcination

The porous silica particles prepared in 2) were put in a glass vial, heated at 550 °C for 5 hours, and cooled slowly to room temperature after completing the reaction to prepare particles.

#### (2) Preparation of porous silica particles

Porous silica particles were prepared by the same method as Example 1-1-(1), except that the reaction conditions at the time of pore expansion were changed to 140 °C and 72 hours.

**(3) Preparation of porous silica particles (10 L scale)**

Porous silica particles were prepared by the same method as Example 1-1-(1), except that a 5 times larger container was used and each material was used in a 5 times capacity.

**(4) Preparation of porous silica particles (particle size of 300 nm)**

Porous silica particles were prepared by the same method as Example 1-1-(1), except that 920 ml of distilled water and 850 ml of methanol were used to prepare the small pore particles.

**(5) Preparation of porous silica particles (particle size of 500 nm)**

Porous silica particles were prepared by the same method as Example 1-1-(1), except that 800 ml of distilled water, 1010 ml of methanol, and 10.6 g of CTAB were used to prepare the small pore particles.

**(6) Preparation of porous silica particles (particle size of 1000 nm)**

Porous silica particles were prepared by the same method as Example 1-1-(1), except that 620 ml of distilled water, 1380 ml of methanol, and 7.88 g of CTAB were used to prepare the small pore particles.

**(7) Preparation of porous silica particles (pore diameter of 4 nm)**

Porous silica particles were prepared by the same method as Example 1-1-(1), except that 2.5 mL of TMB was used for pore expansion.

**(8) Preparation of porous silica particles (pore diameter of 7 nm)**

Porous silica particles were prepared by the same method as Example 1-1-(1), except that 4.5 mL of TMB was used for pore expansion.

**(9) Preparation of porous silica particles (pore diameter of 17 nm)**

Porous silica particles were prepared by the same method as Example 1-1-(1), except that 11 mL of TMB was used for pore expansion.

**(10) Preparation of porous silica particles (pore diameter of 23 nm)**

Porous silica particles were prepared by the same method as Example 1-1-(1), except that 12.5 mL of TMB was used for pore expansion.

**(11) Preparation of porous silica particles (dual modification)**

**1) Preparation of small pore particles**

Small pore particles were prepared by the same method as Example 1-1-(1)-1).

**2) Pore expansion**

Small pore particles were reacted with TMB, cooled and centrifuged by the same method as Example 1-1-(1)-2) to remove the supernatant. Thereafter, the remaining solution was centrifuged under the same conditions as Example 1-1-(1)-2), washed three times with ethanol and distilled water alternately, and then dried under the same conditions as Example 1-1-(1)-2), thereby harvesting powdery porous silica particles (pore diameter 10 to 15 nm, and particle size of 200 nm).

**3) Surface modification**

After dispersing 0.8 g to 1 g of porous silica particles having expanded pores in 50 mL of toluene, 5 mL of (3-aminopropyl)triethoxysilane was added thereto, followed by heating under reflux at 120 °C for 12 hours. The procedure is followed by the washing and drying procedures described above, followed by 1 mL of triethylene glycol (PEG3, 2-[2-(2-methoxyethoxy)ethoxy] acetic acid) and 100 mg of EDC (1-ethyl-3-(3-dimethylaminopropyl)carbodiimide) and 200 mg of N-hydroxysuccinimide (NHS) were dispersed in 30 mL of PBS and allowed to react at room temperature for 12 hours under stirring. The product was then washed and dried.

Since the reaction solution of the previous step remained inside of the pores, the inside of the pores was not modified.

**4) Washing inside pores**

800 mg of surface-modified particle powder was dissolved in 40 ml of 2M HCl/ethanol and refluxed under vigorous stirring for 12 hours.

Thereafter, the cooled reaction solution was centrifuged at 8000 rpm for 10 minutes to remove the supernatant, centrifuged at 8000 rpm and 25 °C for 10 minutes, and washed five times with ethanol and distilled water alternately.

Thereafter, the product was dried in an oven at 70 °C, thereby harvesting powdery porous silica particles.

**5) Modifying inside pores**

① A propyl group was introduced into the pore by the same method as Example 1-2-(2)-1) described below

② An octyl group was introduced into the pore by the same method as Example 1-2-(2)-2) described below

**2. Surface modification of porous silica particles**

**(1) Positively charging**

**1) Particles with particle size of 300 nm**

The porous silica particles of Example 1-1-(4) were reacted with (3-aminopropyl)triethoxysilane (APTES) to be positively charged.

Specifically, 100 mg of porous silica particles were dispersed in a 10 mL toluene in a 100 mL round bottom flask with a bath sonicator. Then, 1 mL of APTES was added and stirred at 400 rpm and 130 °C for 12 hours.

After the reaction, the product was slowly cooled to room temperature and centrifuged at 8000 rpm for 10 minutes to remove the supernatant, further centrifuged at 8000 rpm and 25 °C for 10 minutes, and then washed five times with ethanol and distilled water alternately.

Thereafter, the product was dried in an oven at 70 °C to harvest powdery porous silica particles having an amino group on the surface thereof and inside of the pores.

**2) Particles with particle size of 200 nm**

① The porous silica particles of Example 1-1-(1) were positively charged by reacting the particles with (3-aminopropyl)triethoxysilane (APTES), and were modified in the same manner as the method of Example 1-2-(1)-1), except that 0.4 ml of APTES was added and the reaction time was 3 hours.

② The porous silica particles of Example 1-1-(9) were positively charged by reacting the particles with (3-aminopropyl)triethoxysilane (APTES), and were modified in the same manner as the method of Example 1-2-(1)-1).

③ The porous silica particles of Example 1-1-(10) were positively charged by reacting the particles with (3-aminopropyl)triethoxysilane (APTES), and were modified in the same manner as the method of Example 1-2-(1)-1).

**(2) Introduction of hydrophobic groups**

**1) Propyl group**

The porous silica particles of Example 1-1-(1) were reacted with trimethoxy(propyl)silane to introduce propyl groups into the surface of the particles and inside of the pores, and were subjected to modification by the same method as Example 1-2-(1), except that 0.35 ml of trimethoxy(propyl)silane was added instead of APTES, followed by 12 hours of reaction

**2) Octyl group**

The porous silica particles of Example 1-1-(1) were reacted with trimethoxy-n-octylsilane to introduce propyl groups on the surface of the particles and inside of the pores, and were subjected to modification by the same method as Example 1-2-(1), except that 0.5 ml of trimethoxy-n-octylsilane was added instead of APTES, followed by 12 hours of reaction.

**(3) Negatively charging**

**1) Carboxyl group**

The porous silica particles of Example 1-1-(1) were negatively charged by reacting the particles with succinic anhydride. Further, the charged particles were subjected to modification in the same manner as the method of Example 1-2-(1)-1), except that DMSO (dimethyl sulfoxide) was used instead of toluene, 80 mg of succinic anhydride was added instead of APTES to allow reaction at room temperature for 24 hours under stirring, and DMSO was used instead of distilled water.

**2) Thiol group**

The particles were subjected to modification in the same manner as the method of Example 1-2-(1)-1), except that 1.1 mL of MPTES was used instead of APTES.

**3) Sulfonic acid group**

100 mg of the porous silica nanoparticles of Example 1-2-(3)-2) were dispersed in 1 mL of 1 M aqueous sulfuric acid solution and 20 mL of 30% hydrogen peroxide solution, and stirred at room temperature to induce oxidation, thereby oxidizing a thiol group into a sulfonic acid group. Thereafter, the product was washed and dried in the same manner as the method of Example 1-2-(1)-1).

**4) Methyl phosphonate group**

The particles were subjected to modification in the same manner as the method of Example 1-2-(1)-1), except that 3 mL of THMP was used instead of APTES, 10 mL of distilled water was used instead of toluene and 1.5 ml of hydrochloric acid solution was added.

**3. Identification of particle formation and pore expansion**

Small pore particles and porous silica particles prepared in Experimental Examples 1-1-(1) to (3) were observed under a microscope to determine whether the small pore particles were uniformly formed or the pores were sufficiently expanded to uniformly form the porous silica particles (FIGS. 1 to 4).

FIG. 1 is photographs of the porous silica particles in Example 1-1-(1), and FIG. 2 is photographs of the porous silica particles in Example 1-1-(2), and from these drawings, it can be seen that spherical porous silica particles having sufficiently expanded pores were formed evenly.

FIG. 3 is photographs of the small pore particles in Example 1-1-(1), and FIG. 4 is comparative photographs of the small pore particles in Examples 1-1-(1) and 1-1-(3), and from these drawings, it can be seen that spherical small pore particles were formed evenly.

**4. Calculation of BET surface area and pore volume**

Surface areas and pore volumes of the small pore particles in Example 1-1-(1) and the porous silica particles of Examples 1-1-(1), (7), (8) and (10) were calculated. The surface areas were calculated by Brunauer-Emmett-Teller (BET) method, and the pore size distributions were calculated by Barrett-Joyner-Halenda (BJH) method.

Micrographs of the particles are shown in FIG. 5, and the calculation results are shown in Table 1 below.

[TABLE 1]

| Section | Pore diameter (nm) | BET surface area ($m^2/g$) | Pore volume (mL/g) |
|---|---|---|---|
| Small pore | 2.1 | 1337 | 0.69 |

(continued)

| Section | Pore diameter (nm) | BET surface area ($m^2/g$) | Pore volume (mL/g) |
|---|---|---|---|
| particle in Example 1-1-(1) | | | |
| Example 1-1-(7) | 4.3 | 630 | 0.72 |
| Example 1-1-(8) | 6.9 | 521 | 0.79 |
| Example 1-1-(1) | 10.4 | 486 | 0.82 |
| Example 1-1-(10) | 23 | 395 | 0.97 |

**5. Identification of biodegradability**

In order to identify biodegradability of the porous silica particles in Example 1-1-(1), biodegradability at 37 °C in SBF (pH 7.4) was observed under a microscope at 0 hours, 120 hours and 360 hours, and results thereof are shown in FIG. 6.

Referring to FIG. 6, it can be seen that the porous silica particles are biodegraded and almost degraded after 360 hours.

6. **Measurement of absorbance ratio**

Absorbance ratio over time was measured according to Equation 1 below.

[Equation 1]

$$A_t/A_0$$

(wherein $A_0$ is absorbance of the porous silica particles measured by putting 5 ml of suspension containing 1 mg/ml of the porous silica particles into a cylindrical permeable membrane having pores with a pore diameter of 50 kDa, 15 ml of the same solvent as the suspension comes into contact with an outside of the permeable membrane, and the inside/outside of the permeable membrane are horizontally stirred at 60 rpm and 37 °C, and

$A_t$ indicates absorbance of the porous silica particles measured after lapse of "t" hours since $A_o$ was measured).

Specifically, 5 mg of porous silica particle powder was dissolved in 5 ml of SBF (pH 7.4). Thereafter, 5 ml of porous silica particle solution was placed in a permeable membrane having pores with a pore diameter of 50 kDa shown in FIG. 7. 15 ml of SBF was added to the outer membrane, and the SBF on the outer membrane was replaced every 12 hours. Degradation of the porous silica particles was performed at 37 °C under horizontal stirring at 60 rpm.

Then, the absorbance was measured by UV-vis spectroscopy and analyzed at $\lambda$ = 640 nm.

**(1) Measurement of absorbance ratio**

Absorbance ratio of the porous silica particles in Example 1-1-(1) was measured according to the above method, and results thereof are shown in FIG. 8.

Referring to FIG. 8, it can be seen that t, at which the absorbance ratio becomes 1/2, is about 58 hours to demonstrate very slow degradation.

**(2) Particle size**

Absorbances of the porous silica particles in Examples 1-1-(1), (5) and (6) were measured according to Equation 1 above, and results thereof are shown in FIG. 9 (SBF used as the suspension and the solvent).

Referring to FIG. 9, it can be seen that t is decreased as the particle size is increased.

**(3) Average pore diameter**

Absorbances of the porous silica particles in Examples 1-1-(1) and (9) and the microporous silica particles in Example 1-1-(1) as a control were measured according to Equation 1 above, and results thereof are shown in FIG. 10 (SBF used as the suspension and the solvent).

Referring to FIG 10, it can be seen that the porous silica particles of the inventive example have a significantly larger t than the control.

**(4) pH**

Absorbance of the porous silica particles in Example 1-1-(4) for each pH was measured. The absorbance was measured in SBF and in Tris at pH 2, 5, and 7.4, and results thereof are shown in FIG. 11.

Referring to FIG 11, it could be seen that, although there is a difference in t in relation to pH, t at which all absorbance ratio becomes 1/2 was 24 or more.

**(5) Charging**

Absorbance of the porous silica particles in Example 1-2-(1)-1) was measured, and results thereof are shown in FIG. 12 (Tris (pH 7.4) used as the suspension and the solvent).

Referring to FIG 12, it could be seen that t at which the absorbance ratio of the positively charged particles becomes 1/2 was 24 or more.

**7. Support of polypeptides having epithelial or endothelial cell growth promoting ability, or substances for increasing activity or expression thereof**

(1) As the lactoferrin protein, a protein consisting of the sequence of SEQ ID NO: 1 was used. As the particles, the particles of Example 1-2-(3)-4) having a methyl phosphonate group introduced on the surface thereof were used. 40 μg of the porous silica particle powder and 4 μg of the protein were mixed in 200 μl of 1 × PBS, and then incubated at room temperature for 1 hour. The same particles as described above were used for carrying the lactoferrin protein described below.

(2) With regard to plasmid DNA, the promoter portion was amplified from the pCMV vector to prepare a KanaR2-ColE1-CMV promoter portion. The LTF encoding portion was amplified by PCR from the human cDNA library, thereby preparing an LTF ORF of about 3 kb in length (pLEM-hLTF-101, SEQ ID NO: 2).

As the particles, the particles of Example 1-2-(1)-1) in which amino groups were introduced on the surface of the particles and inside of the pores were used.

After mixing 10 μg of the porous silica particles and 0.25 μg of the LTF ORF, the mixture was loaded under 1 × PBS condition at room temperature for 30 minutes. The same particles as described above were used to carry the lactoferrin plasmid described below.

**Example 2. Identification of wound healing effects**

**(1) Assessment of VEGF mRNA expression level**

**1) Use of lactoferrin protein**

After seeding C2C12 cells in a 12-well plate by 5.0 × $10^4$ cells, the cells were incubated for 24 hours. Then, pre-starvation treatment was performed in a serum-free medium for 12 hours. Cells were treated with 200 μg/mL LTF protein after replacement with 0.1% FBS-containing medium. According to the time (0, 1, 2, 3, 6, 12, 24, 48 hours), the cells were subjected to trizol treatment in order to extract mRNA, and VEGF mRNA expression was confirmed by PCR.

**2) Use of particles carrying lactoferrin plasmid**

After seeding C2C12 cells in a 12-well plate by 5.0 × $10^4$ cells, the cells were incubated for 24 hours. Then, pre-starvation treatment was performed in a serum-free medium for 12 hours. LTF pDNA@DDV complex prepared by carrying 1 μg of LTF plasmid DNA on 20 μg of DDV was used to treat the cells for 4 hours under serum-free conditions.

Then, the cells were washed twice with 1 × PBS, the medium was replaced with 0.1% FBS-containing medium, the cells were subjected to trizol treatment in the order of time (0, 4, 8, 12, 24 and 48 hours) in order to extract mRNA, and LTF and VEGF mRNA expression levels were confirmed by PCR.

As a result, when C2C12 cell-line was treated with Recombinant LTF (rLTF, lactoferrin protein), the expression level of VEGF mRNA was induced at a high level in 6 hours, but thereafter, was gradually decreased.

However, when transfecting LTF plasmid DNA (pLTF) supported on DDV into C2C12 cell-line, the expression of VEGF mRNA increased after 4 hours and was continued to be induced at a high level even after 48 hours (FIG. 13).

### (2) Assessment of cell proliferation inducing effects

After seeding HEK-293T cells in a 12-well plate by 5.0 × $10^4$ cells, the cells were incubated for 24 hours. 1.0 μg of LTF pDNA was added to 20.0 μg of DDV to prepare an LTF pDNA@DDV complex, followed by treating the cells with this complex under serum-free conditions for 4 hours (mock was treated with only 1.0 μg of LTF pDNA). After washing the cells once with 1 × PBS, the medium was replaced with a 1% FBS-containing medium, and the cells were incubated for 48 hours. After 48 hours, the medium in which HEK-294T cells were cultured was collected.

After seeding HT-1080 cells in a 96-well plate by 2.0 × $10^3$ cells, the cells were incubated for 24 hours. Then, pre-starvation treatment was performed for 12 hours in a serum-free medium. The cells were treated with the serum-free medium, 10% FBS medium, 1% FBS medium + the medium collected from Mock HEK-294T cells, 1% FBS medium + the medium collected from HEK-293T cells treated with 50% LTE pDNA DDV, and the medium collected from HEK-293T cells treated with 25% LTF pDNA DDV, respectively.

In order to determine and compare cell proliferations after 24, 48, 72 and 96 hours, the cells were treated with 10% CCK-8 solution and incubated for 1 hour. Thereafter, the cells were compared through absorbance at a wavelength of 450 nm.

As a result, the proliferation rate of HT-1080 cell-line was very low in the culture with 1% FBS, but HT-1080 cell-line with conditioned medium showed induction of cell proliferation in a form similar to that of the culture solution with 10% FBS (FIG. 14).

FIG. 14 illustrates bar graphs at 24, 48, 72 and 96 hours in order from the left.

### (3) Assessment of cell proliferation inducing effects and angiogenesis induction

#### (1) HT-1080 cells

Cell proliferation of the cells was determined by treating the HT-1080 cells with lactoferrin protein supported on DDV.

After seeding HT-1080 cells in a 96-well plate by 3.0 × $10^3$ cells, the cells were incubated for 24 hours. After washing the cells once with 1 × PBS, the cells were treated with a FBS-free medium, a 10% FBS medium, 1% FBS + DDV (0, 12.5, 25, 50, 100, 200, 400 μg/mL) carrying lactoferrin protein, and FBS-free medium + DDV (0, 12.5, 25, 50, 100, 200, 400 μg/mL) carrying lactoferrin protein, respectively, and then incubated for 48 hours. After 48 hours, the cells were washed once with 1 × PBS, treated with 10% CCK-8 solution, and then incubated for 1 hour. Thereafter, absorbance was confirmed at 450 nm in order to determine an extent of proliferation of the cells.

#### (2) PAM-212 cells

Recombinant LTF protein supported on DDV was used to treat PAM-212 cells to determine the extent of cell proliferation.

After seeding PAM-212 cells in a 96-well plate by 2.0 × $10^3$ cells, the cells were incubated for 24 hours. The cells were washed once with 1 × PBS, and then treated with FBS-free media, 10% FBS, 1% FBS + lactoferrin protein (100 μg/mL), 1% FBS + DDV carrying lactoferrin protein (100 μg/mL), and 1% FBS, respectively. The cells were washed once with 1 × PBS at 48 and 96 hours, respectively, followed by treatment with 10% CCK-8 solution. Thereafter, the cells were incubated for 1 hour. Then, absorbance was confirmed at 450 nm wavelength in order to determine an extent of proliferation of the cells.

#### (3) HUVEC cells

The VEGF protein and lactoferrin protein supported on DDV were used to treat HUVEC cells in order to identify effects of inducing the cells into a vascular form.

After seeding HUVEC cells in a 96-well plate by 1.5 × $10^4$ cells, the cells were treated with a vehicle, DDV (40 ng/ml) carrying VEFG protein (the same particles as lactoferrin protein-carrying particles), and DDV (20 μg/mL) carrying lactoferrin protein, respectively, followed by incubation for 4 hours. After 4 hours, a degree of induction of the cells into the reticulum such as a vascular shape was confirmed under a microscope.

As a result, it was identified that the cell proliferation effect was excellent when the lactoferrin protein was supported on DDV and delivered (FIG. 15).

### (4) Identification of injury healing effects

#### 1) Preparation of injury healing animal model

After shaving the back of each experimental animal (rodents, C57BL/6), the animal was injured by a biopsy punch (5 mm in diameter).

Then, the prepared composition (50 μl) was injected into the skin around the injury lesion, and the wound was closed with a circular silicone cover (outer diameter: 30 mm, inner diameter: 5 mm) and sutured with a surgical thread for observation and management. To prevent infection of the wound, a Tegaderm film was attached onto the wound and the wound was wrapped with a pressure bandage.

#### 2) Identification of effects

① To injury-induced experimental animals, 1 × PBS, DDV (500 μg) carrying lactoferrin protein, LTF pDNA (25 μg), DDV (500 μg), and DDV (25 μg) carrying LTF pDNA, respectively, were injected into the skin around the injury lesion. The wound was observed every 2 days, and the wound repairing extent was monitored.

In the case of DDV carrying lactoferrin, the injury healing effects were increased. Specifically, DDV carrying pLTF showed the most excellent injury healing effects (FIG. 16) .

② To injury-induced experimental animals, 1 × PBS, DDV (500 μg) carrying lactoferrin protein, LTF pDNA (25 μg), DDV (500 μg), DDV (25 μg) carrying LTF pDNA, DDV (25 μg) carrying EGF pDNA, and DDV (25 μg) carrying HGF pDNA, respectively, were injected into the skin around the injury lesion. Each of the wounds was observed every 2 days, and the wound repairing extent was monitored. The wound repairing extents were compared using an image analysis program (Image J) according to Formula of (a size of the injury on the day of imaging/a size of the initial injury) × 100 (%).

In the case of DDV carrying lactoferrin, the injury healing effects were increased. Specifically, DDV carrying pLTF showed the most excellent injury healing effects (FIG. 17) .

③ To injury-induced experimental animals, 1 × PBS and DDV (1 mg) carrying lactoferrin protein was injected into the skin around the injury lesion. The wound was observed every 2 days, and the wound repairing extent was monitored. The injury repairing extents were compared using an image analysis program (Image J) according to Formula of (a size of the injury on the day of imaging/a size of the initial injury) × 100 (%).

In the case of DDV carrying lactoferrin, the injury healing effects were increased (FIG. 18).

④ Histopathological examination

DDV carrying lactoferrin protein or lactoferrin pDNA was intradermally injected around lesion sites of the injury healing animal model and, after 14 days, an increase in injury healing effect was monitored through histopathological examination.

After euthanizing the experimental animals, the skin was collected at the injury site and the tissues were fixed with 4% PFA. The tissues were made into frozen blocks, sliced to 7 mm thickness, stained, and placed on a glass slide for observation under a microscope.

As a result, the injury healing effects were increased in the case of DDV carrying lactoferrin protein or pDNA and, in particular, DDV carrying pDNA showed excellent effects (FIG. 19).

## Claims

**1.** A pharmaceutical composition for treatment of wounds, comprising:

porous silica particles on which a polypeptide having epithelial or endothelial cell growth promoting ability or a substance for increasing activity or expression thereof is supported,
wherein the porous silica particles are **characterized in that** t, at which an absorbance ratio in the following Equation 1 becomes 1/2, is 24 or more:

[Equation 1]

$$A_t/A_0$$

(wherein $A_0$ is absorbance of the porous silica particles measured by putting 5 ml of suspension containing 1 mg/ml of porous silica particles into a cylindrical permeable membrane having pores with a pore diameter of 50 kDa,
15 ml of the same solvent as the suspension comes into contact with an outside of the permeable membrane, and the inside/outside of the permeable membrane are horizontally stirred at 60 rpm and at 37 °C,
pH of the suspension is 7.4, and
$A_t$ indicates absorbance of the porous silica particle measured after lapse of "t" hours since $A_o$ was measured) .

**2.** The composition according to claim 1, wherein the porous silica particles are prepared by: reacting silica particles having pores with a pore diameter of less than 5 nm with a swelling agent at 120 to 180 °C for 24 to 96 hours to expand the pores with a pore diameter of less than 5 nm; and calcining the silica particles having expanded pores at a temperature of 400 °C or higher for at least 3 hours.

**3.** The composition according to claim 1, wherein an average diameter of the porous silica particles ranges from 150 to 1000 nm, a BET surface area ranges from 200 to 700 $m^2/g$, and a volume per gram ranges from 0.7 to 2.2 ml.

**4.** The composition according to claim 1, wherein the polypeptide is lactoferrin, an epidermal growth factor, a hepatocyte growth factor or a vascular endothelial growth factor.

**5.** The composition according to claim 4, wherein the lactoferrin protein is composed of a sequence of SEQ ID NO: 1.

**6.** The composition according to claim 4, wherein the epidermal growth factor is composed of a sequence of SEQ ID NO: 2, the hepatocyte growth factor is composed of a sequence of SEQ ID NO: 3, and the vascular endothelial growth factor is composed of a sequence of SEQ ID NO: 4.

**7.** The composition according to claim 1, wherein the substance is a plasmid on which a gene encoding the sequence of SEQ ID NO: 1 is supported.

**8.** The composition according to claim 1, wherein the porous silica particles carry a polypeptide having epithelial or endothelial cell growth promoting ability, and are negatively charged at neutral pH on an outer surface of the particles or an inside of the pores.

**9.** The composition according to claim 1, wherein the porous silica particles carry a plasmid, on which a gene encoding the polypeptide having epithelial or endothelial cell growth promoting ability is supported, and are positively charged at neutral pH on an outer surface of the particles or an inside of the pores.

[FIG. 1]

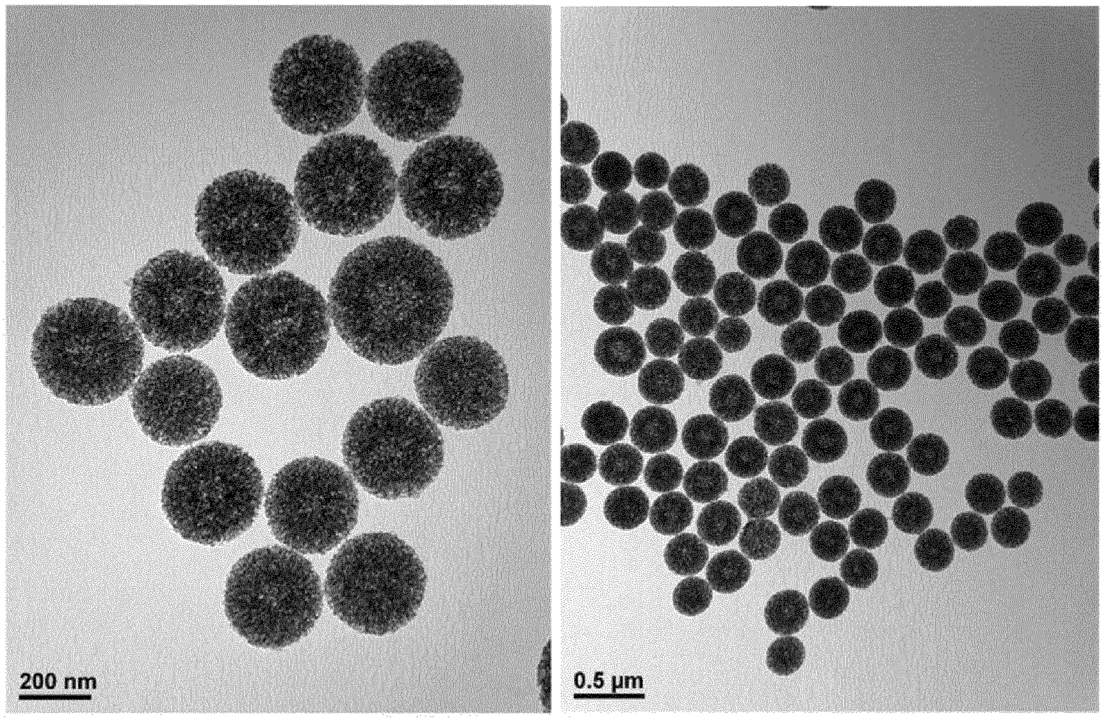


[FIG. 2]

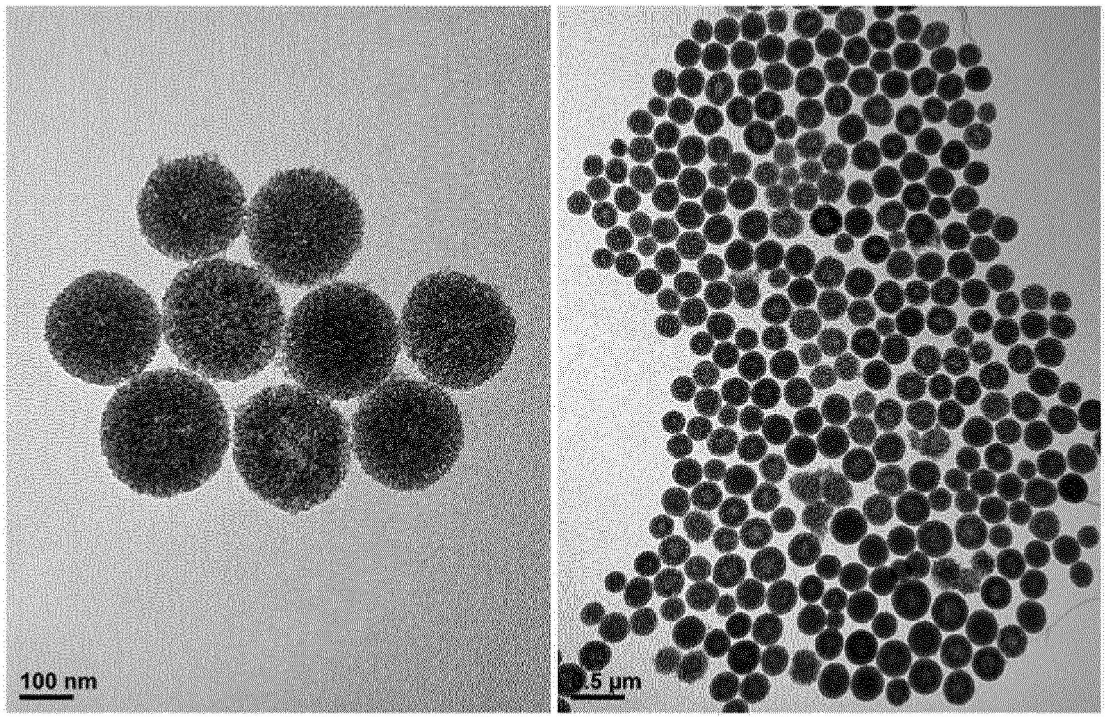

[FIG. 3]

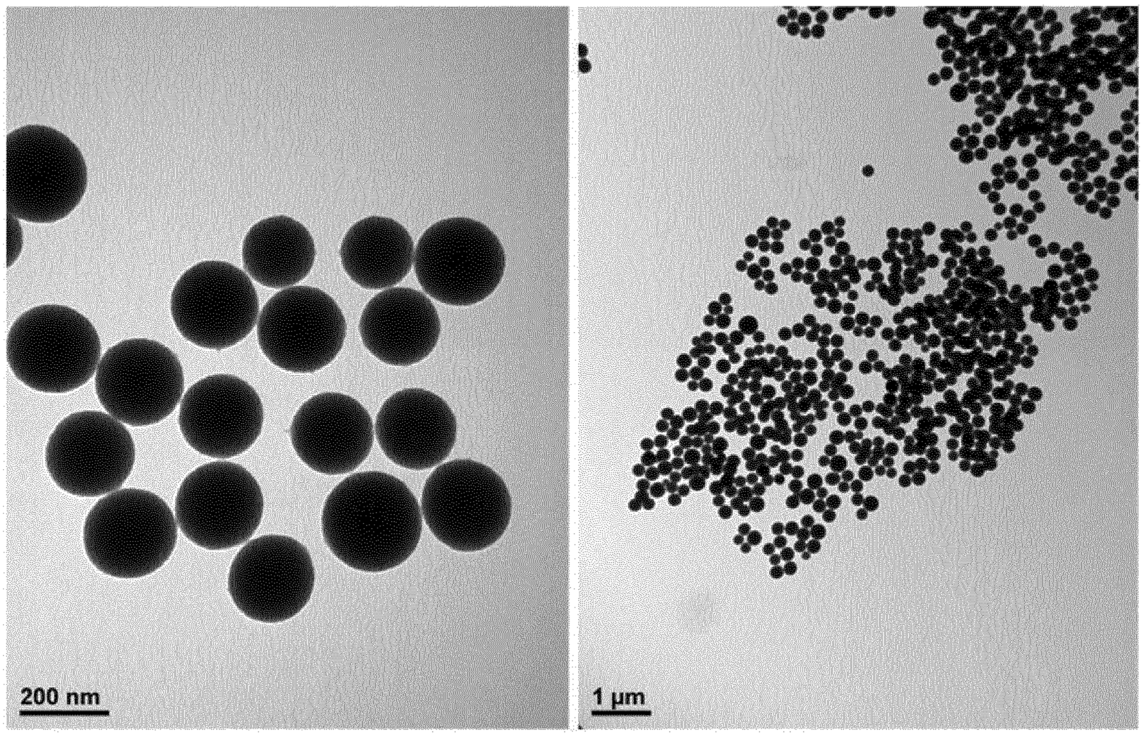


[FIG. 4]

2 L scale　　10 L scale

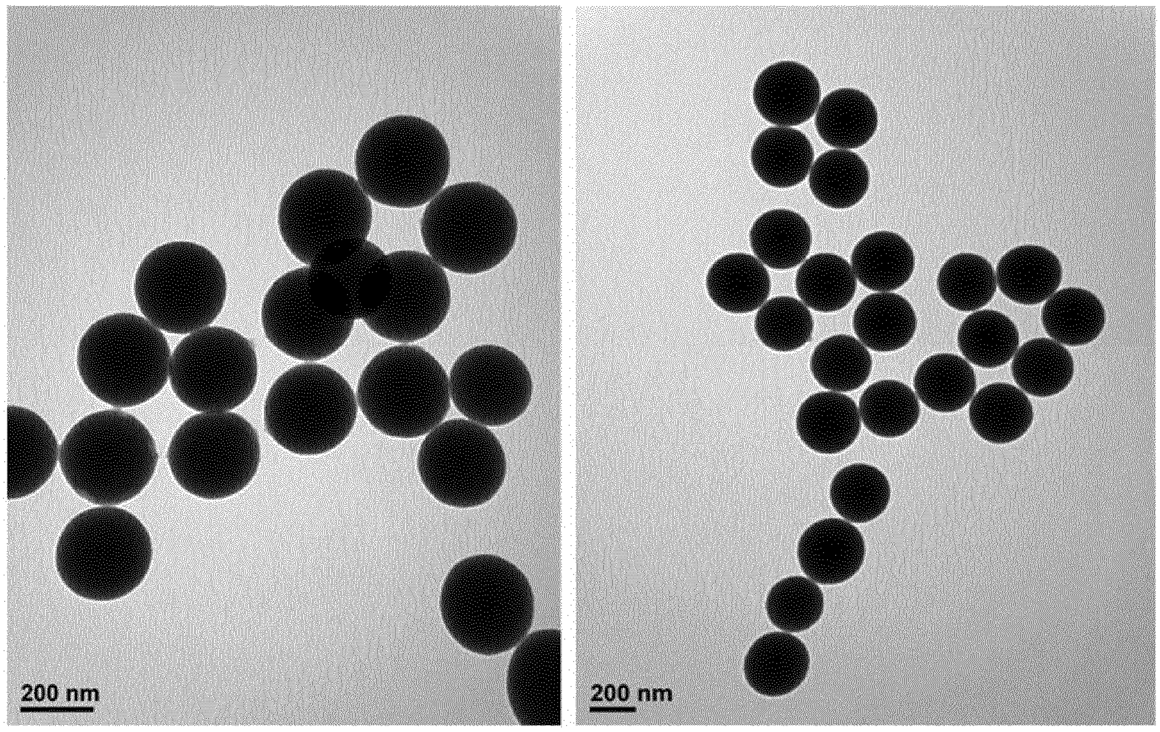

[FIG. 5]

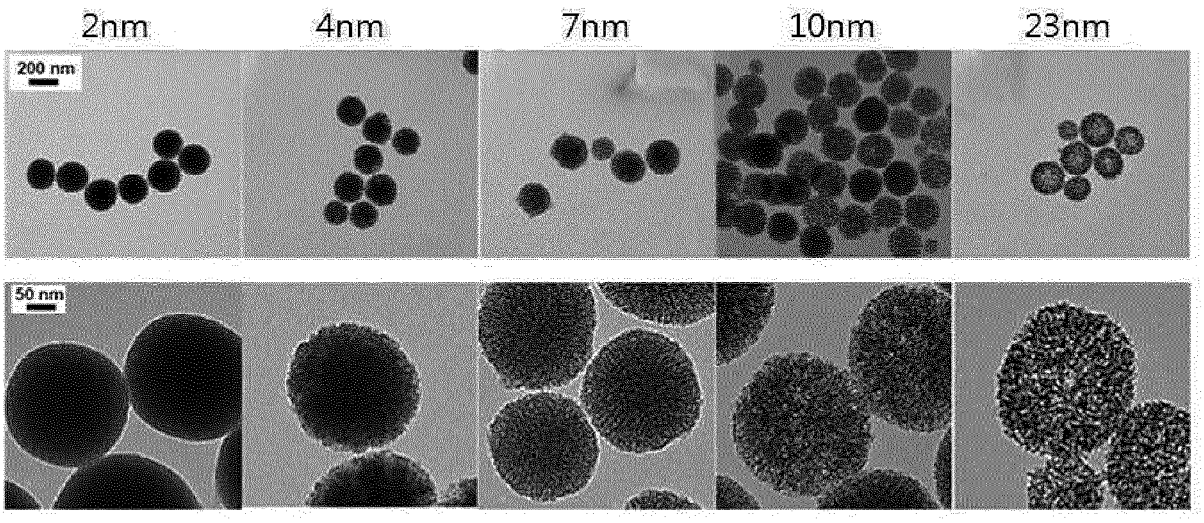


[FIG. 6]

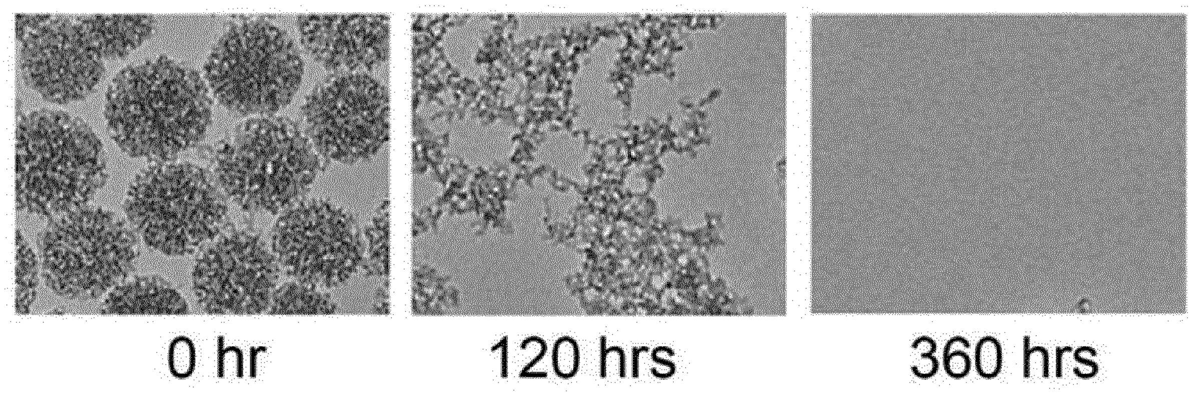

[FIG. 7]

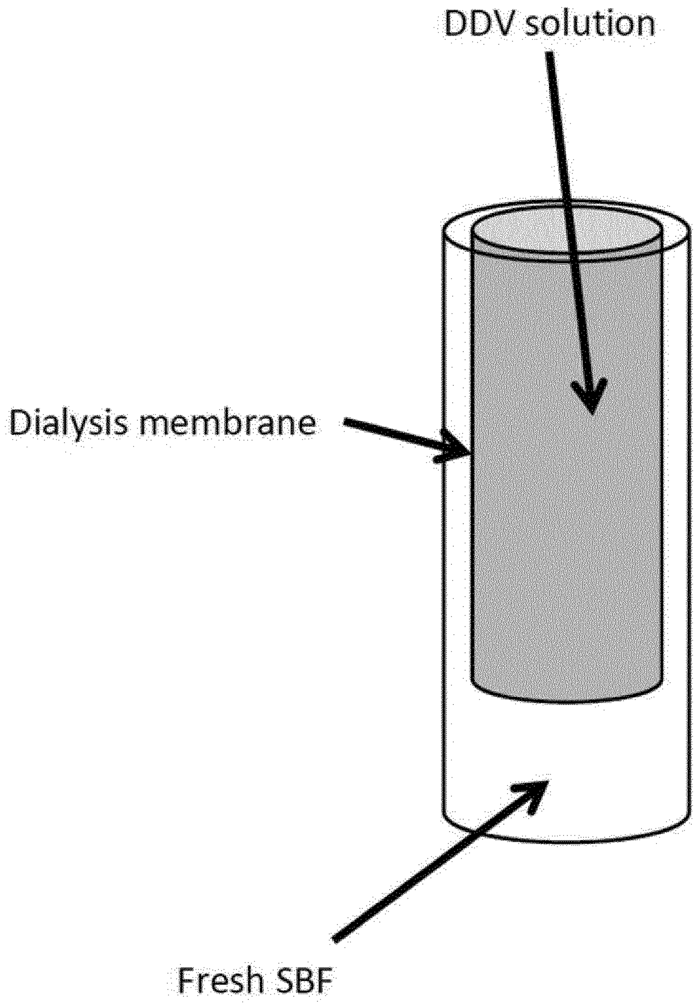
DDV solution
Dialysis membrane
Fresh SBF

[FIG. 8]

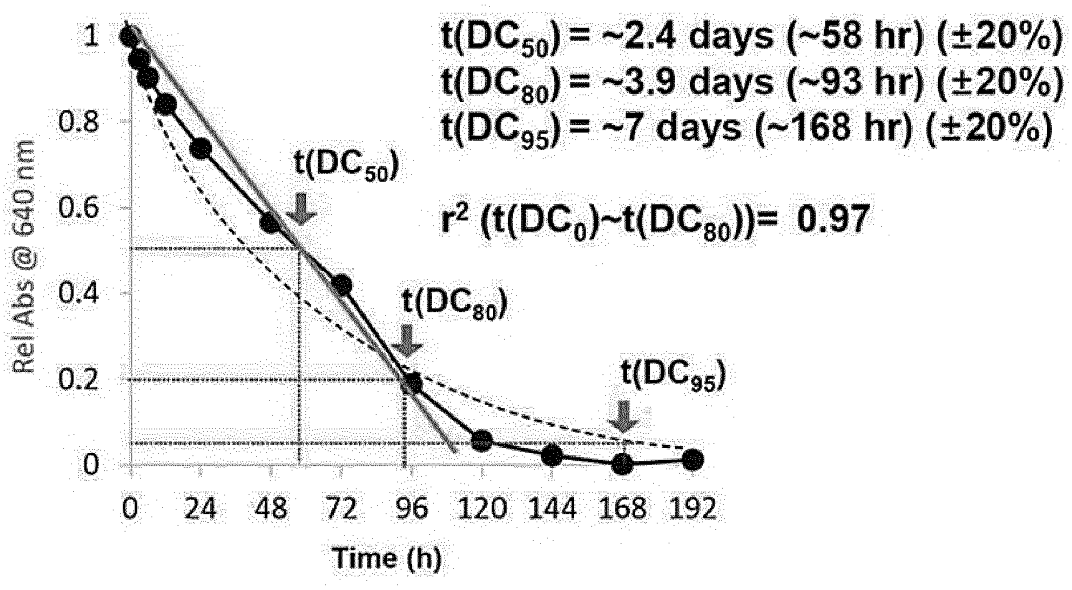
t(DC50) = ~2.4 days (~58 hr) (±20%)
t(DC80) = ~3.9 days (~93 hr) (±20%)
t(DC95) = ~7 days (~168 hr) (±20%)
r2 (t(DC0)~t(DC80))= 0.97
t(DC50)
t(DC80)
t(DC95)
Rel Abs @ 640 nm
1
0.8
0.6
0.4
0.2
0
0 24 48 72 96 120 144 168 192
Time (h)

[FIG. 9]

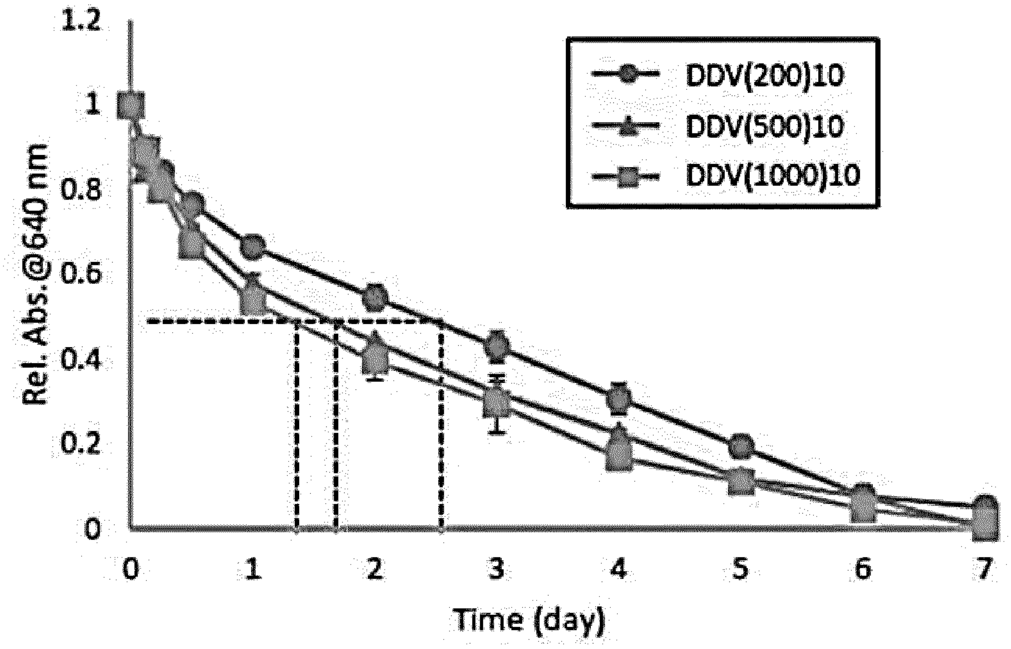


| Sample | $t_{50\%}$ |
|---|---|
| $DDV(200)_{10}$ | 57.4 h |
| $DDV(500)_{10}$ | 37.5 h |
| $DDV(1000)_{10}$ | 30.0 h |

[FIG. 10]

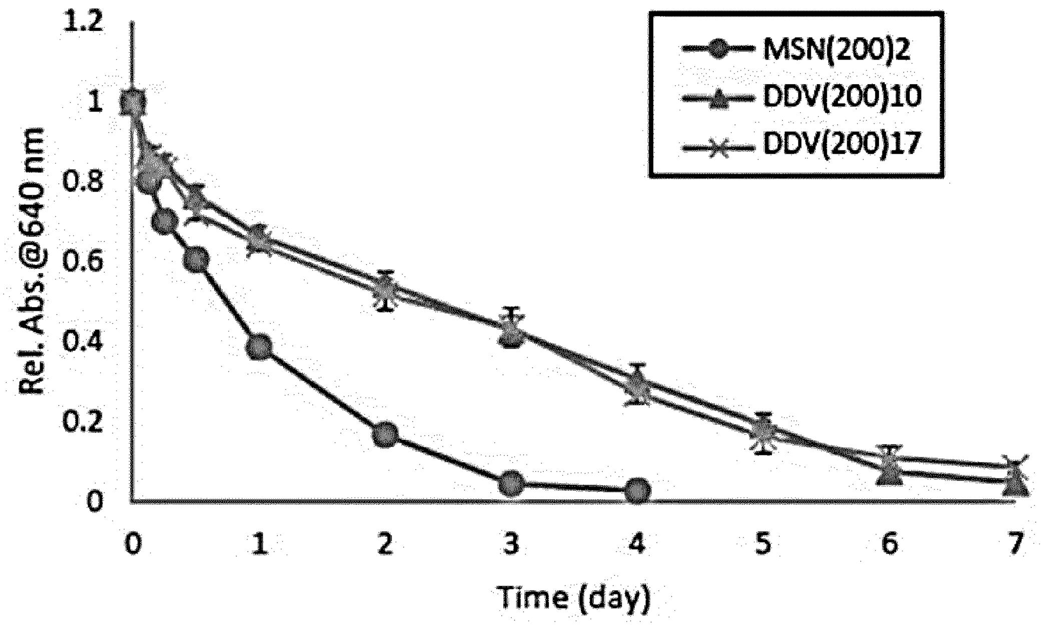


| Samples | $t_{50\%}$ |
|---|---|
| $MSN(200)_2$ | 17.9 h |
| $DDV(200)_{10}$ | 57.4 h |
| $DDV(200)_{17}$ | 53.6 h |

[FIG. 11]

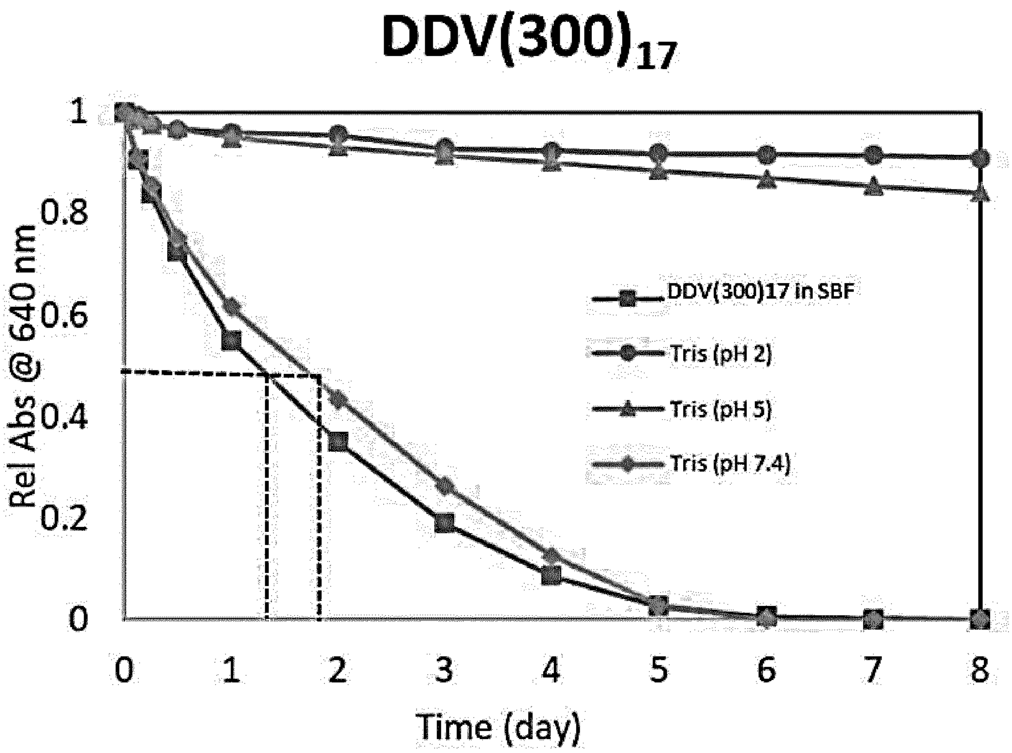
DDV(300)17
Rel Abs @ 640 nm
Time (day)
DDV(300)17 in SBF
Tris (pH 2)
Tris (pH 5)
Tris (pH 7.4)
0
0.2
0.4
0.6
0.8
1
0
1
2
3
4
5
6
7
8

[FIG. 12]

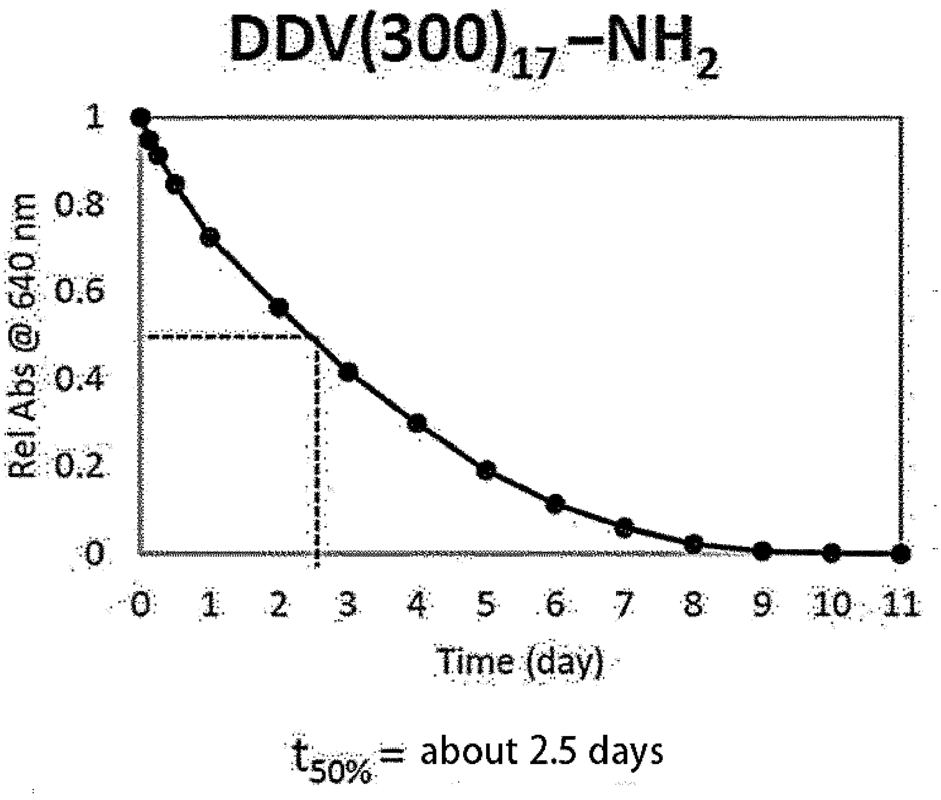
DDV(300)17-NH2
Rel Abs @ 640 nm
Time (day)
0
0.2
0.4
0.6
0.8
1
0
1
2
3
4
5
6
7
8
9
10
11
t50% = about 2.5 days

[FIG. 13]

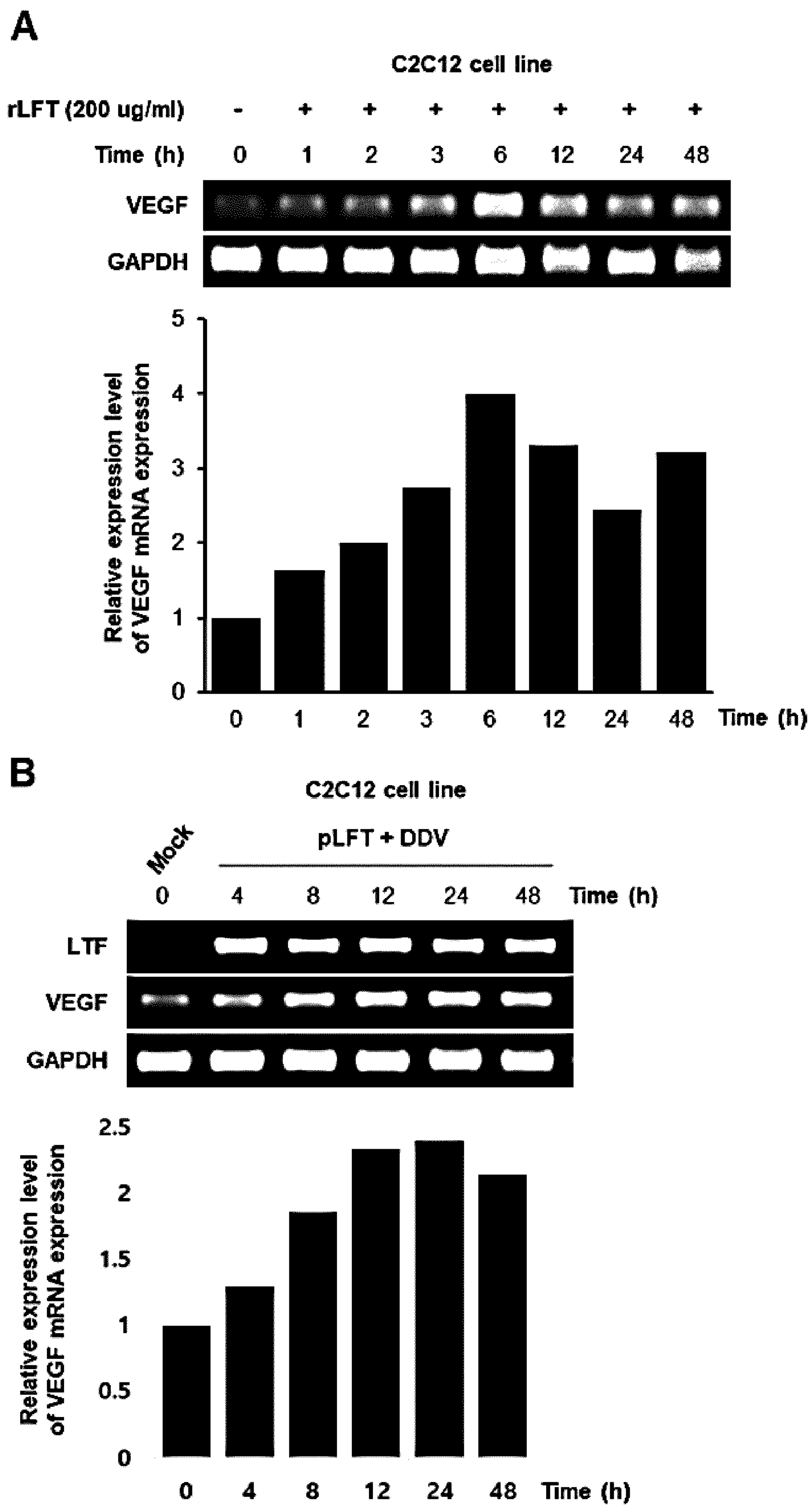
A
C2C12 cell line
rLFT (200 ug/ml) - + + + + + + +
Time (h) 0 1 2 3 6 12 24 48
VEGF
GAPDH
Relative expression level of VEGF mRNA expression
0 1 2 3 4 5
0 1 2 3 6 12 24 48 Time (h)
B
C2C12 cell line
Mock
pLFT + DDV
0 4 8 12 24 48 Time (h)
LTF
VEGF
GAPDH
Relative expression level of VEGF mRNA expression
0 0.5 1 1.5 2 2.5
0 4 8 12 24 48 Time (h)

[FIG. 14]

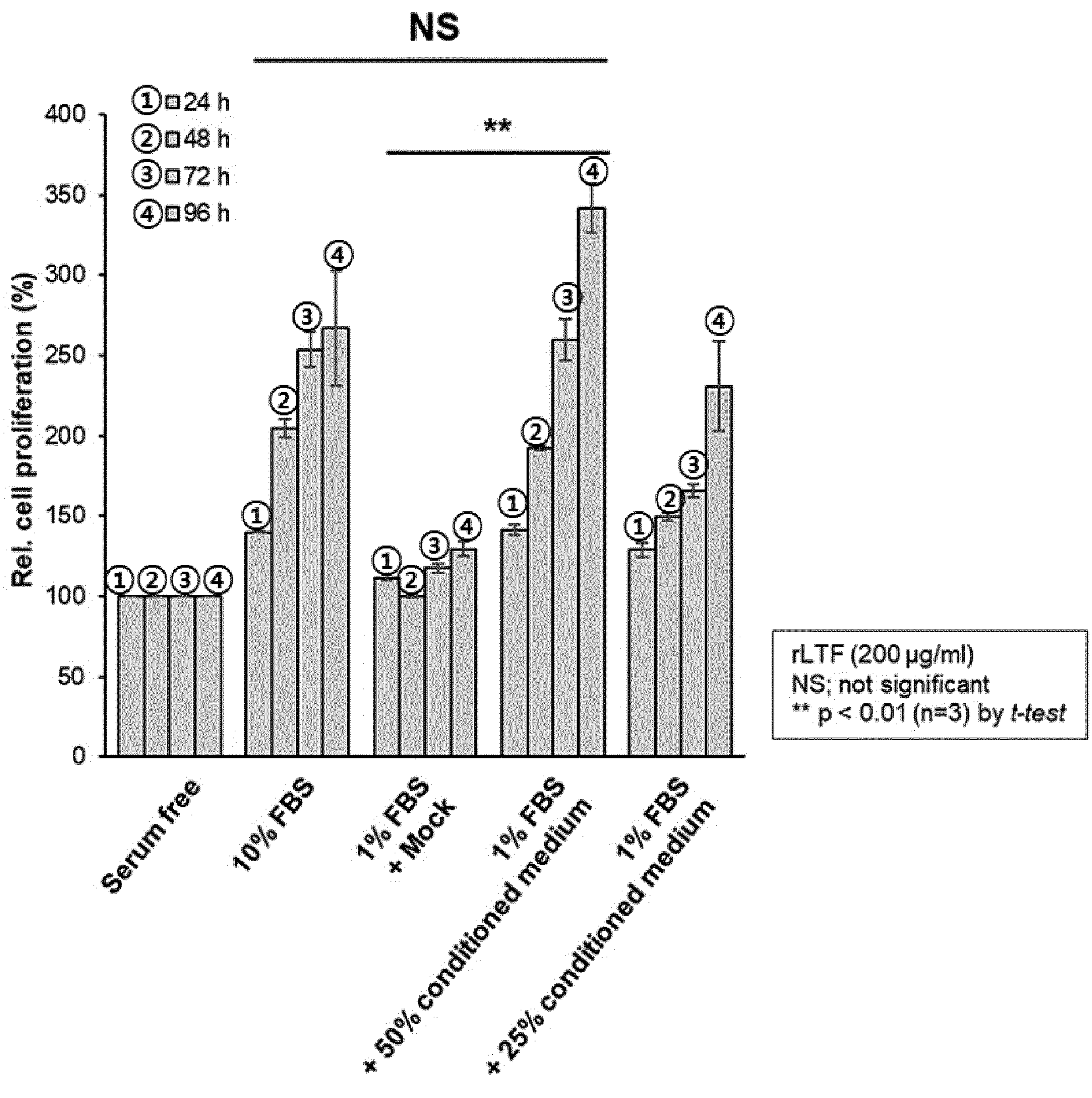
NS
**
①24 h
②48 h
③72 h
④96 h
Rel. cell proliferation (%)
400
350
300
250
200
150
100
50
0
Serum free
10% FBS
1% FBS + Mock
1% FBS + 50% conditioned medium
1% FBS + 25% conditioned medium
rLTF (200 μg/ml)
NS; not significant
** p < 0.01 (n=3) by t-test

[FIG. 15]

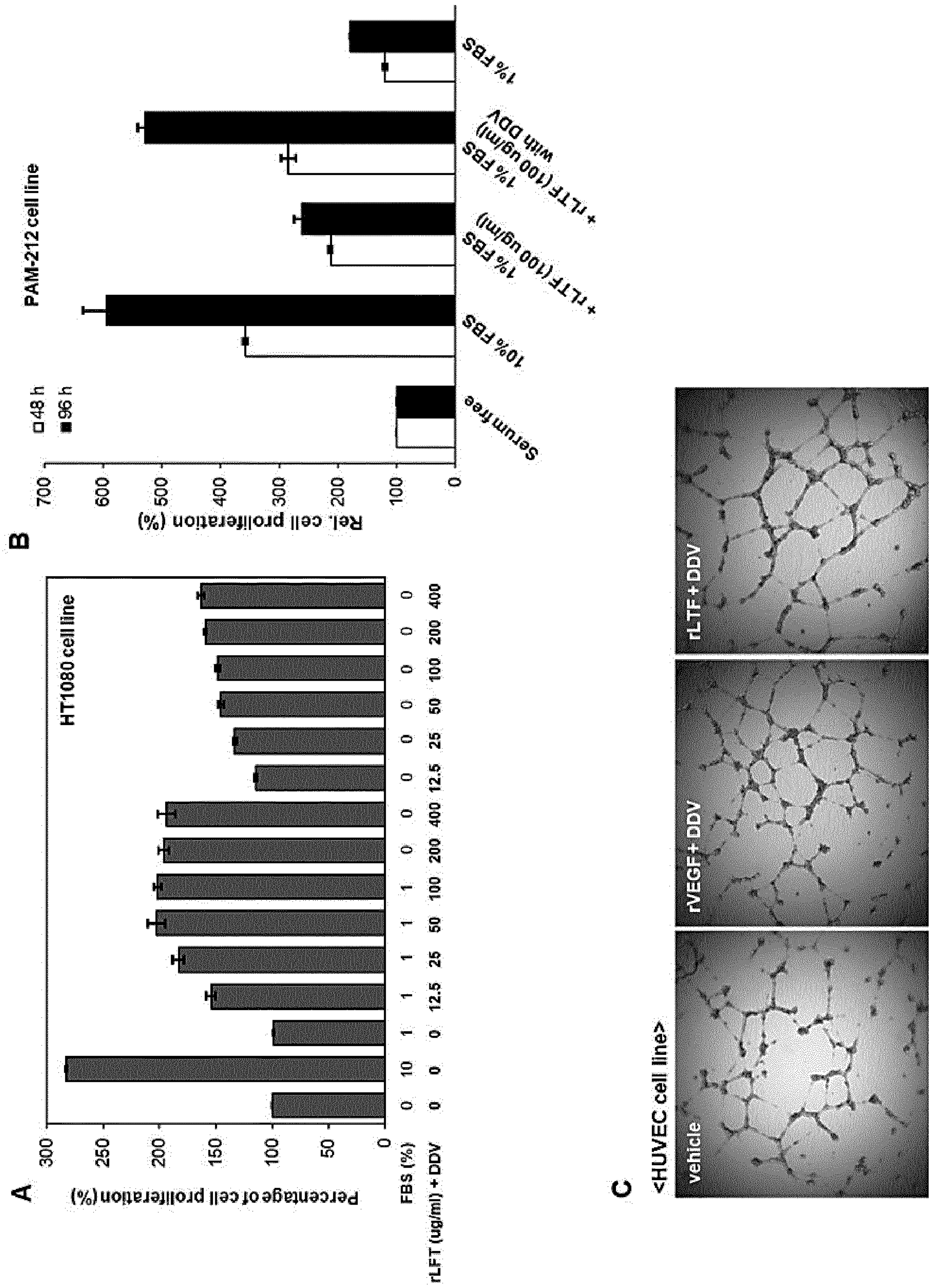
A
HT1080 cell line
Percentage of cell proliferation (%)
FBS (%)
rLFT (ug/ml) + DDV
B
PAM-212 cell line
Rel. cell proliferation (%)
48 h
96 h
Serum free
10% FBS
1% FBS + rLTF (100 ug/ml)
1% FBS + rLTF (100 ug/ml) with DDV
1% FBS
C
<HUVEC cell line>
vehicle
rVEGF+ DDV
rLTF + DDV

[FIG. 16]

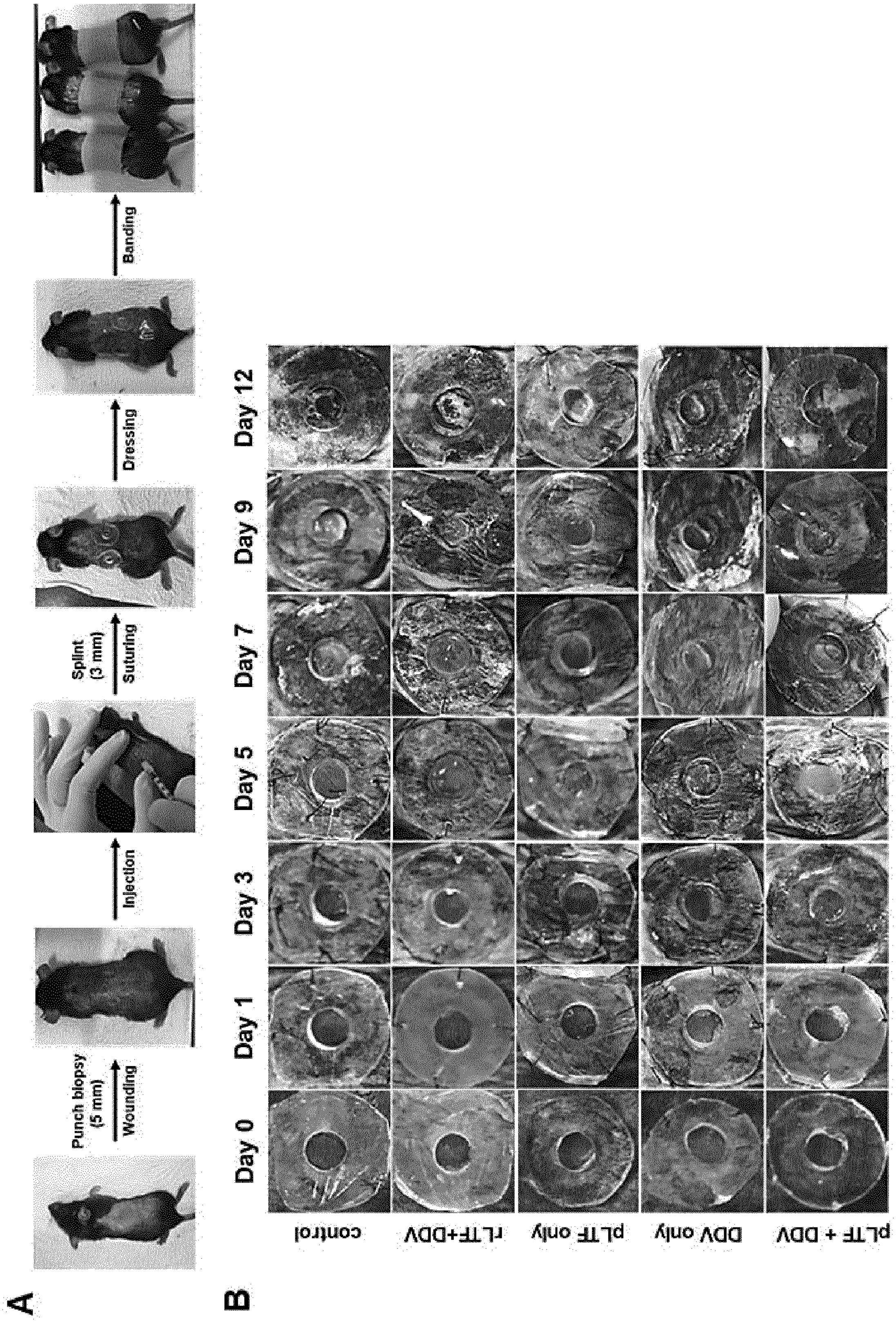
A
Punch biopsy (5 mm)
Wounding
Injection
Splint (3 mm)
Suturing
Dressing
Banding
B
Day 0
Day 1
Day 3
Day 5
Day 7
Day 9
Day 12
control
rLTF+DDV
pLTF only
DDV only
pLTF + DDV

[FIG. 17]

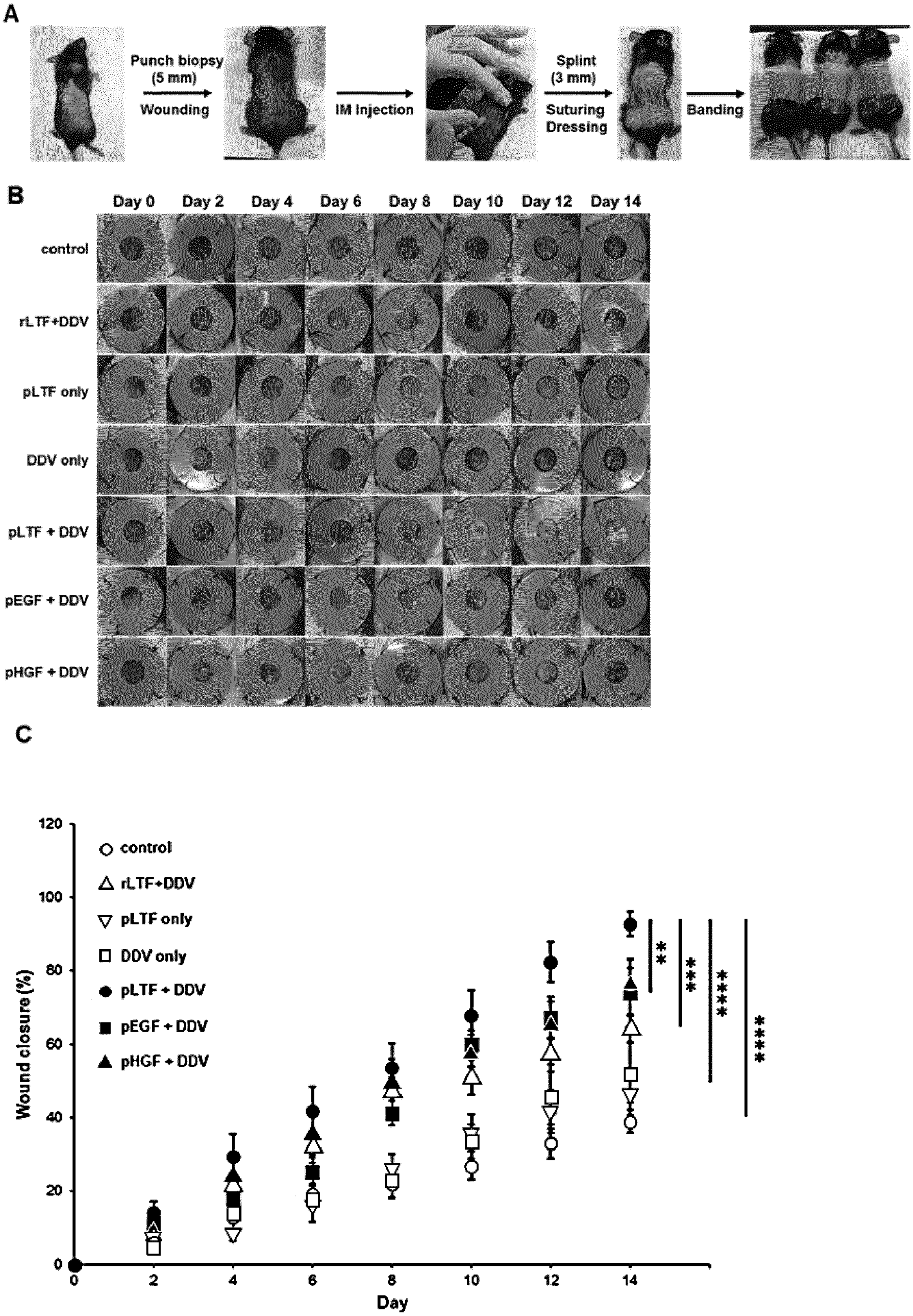
A
Punch biopsy
(5 mm)
Wounding
IM Injection
Splint
(3 mm)
Suturing
Dressing
Banding
B
Day 0
Day 2
Day 4
Day 6
Day 8
Day 10
Day 12
Day 14
control
rLTF+DDV
pLTF only
DDV only
pLTF + DDV
pEGF + DDV
pHGF + DDV
C
Wound closure (%)
Day
control
rLTF+DDV
pLTF only
DDV only
pLTF + DDV
pEGF + DDV
pHGF + DDV
**
***
****
****

[FIG. 18]

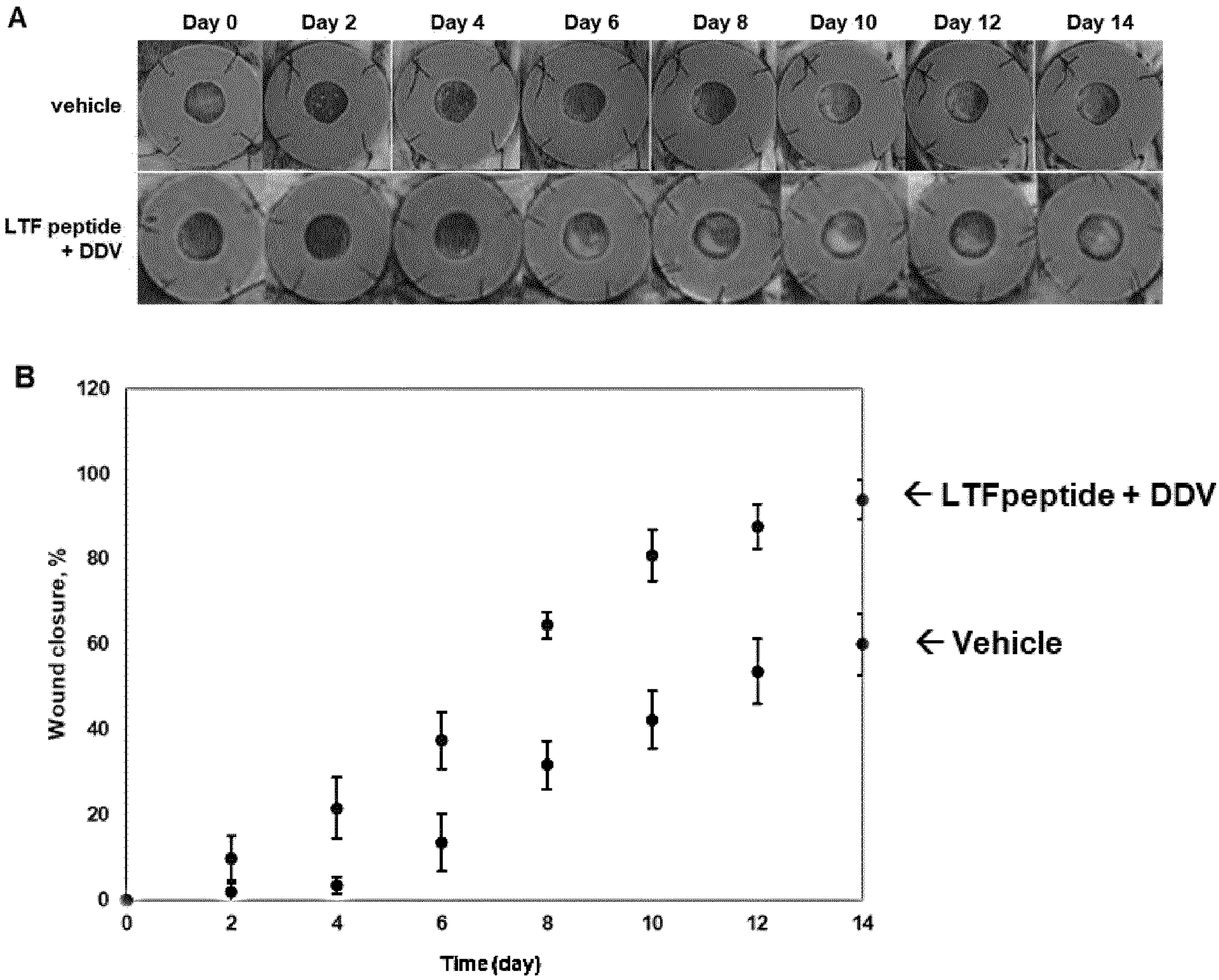
A
Day 0
Day 2
Day 4
Day 6
Day 8
Day 10
Day 12
Day 14
vehicle
LTF peptide + DDV
B
Wound closure, %
Time (day)
← LTFpeptide + DDV
← Vehicle

[FIG. 19]

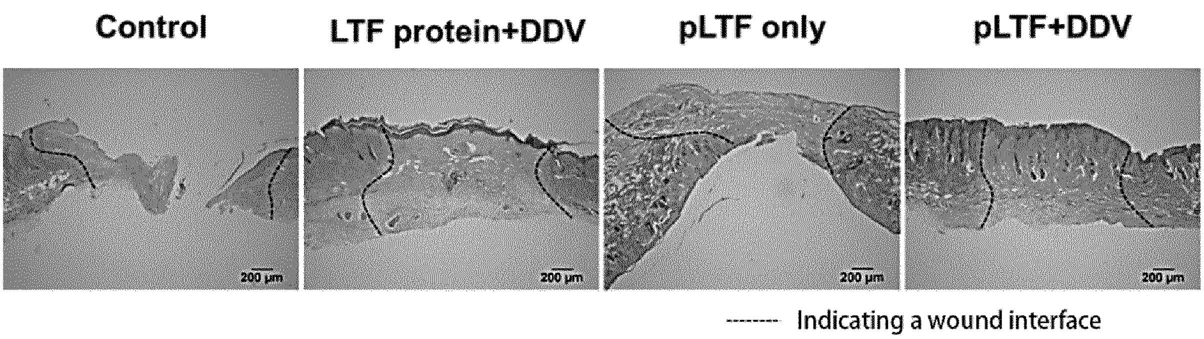
Control
LTF protein+DDV
pLTF only
pLTF+DDV
200 μm
-------- Indicating a wound interface

**INTERNATIONAL SEARCH REPORT**

International application No.
PCT/KR2019/009531

A. CLASSIFICATION OF SUBJECT MATTER

*A61K 9/14(2006.01)i, A61K 9/51(2006.01)i, A61K 38/40(2006.01)i, A61K 38/18(2006.01)i, A61P 17/02(2006.01)i*

According to International Patent Classification (IPC) or to both national classification and IPC

B. FIELDS SEARCHED

Minimum documentation searched (classification system followed by classification symbols)
A61K 9/14; A61K 31/7105; A61K 38/16; A61K 39/395; A61K 47/36; A61K 47/48; A61K 7/00; A61K 9/00; A61K 9/16; A61K 9/51; A61K 38/40; A61K 38/18; A61P 17/02

Documentation searched other than minimum documentation to the extent that such documents are included in the fields searched
Korean utility models and applications for utility models: IPC as above
Japanese utility models and applications for utility models: IPC as above

Electronic data base consulted during the international search (name of data base and, where practicable, search terms used)
eKOMPASS (KIPO internal) & Keywords: porosity, silica, growth factor, scar, absorbance

C. DOCUMENTS CONSIDERED TO BE RELEVANT

| Category* | Citation of document, with indication, where appropriate, of the relevant passages | Relevant to claim No. |
|---|---|---|
| X | KR 10-2016-0011565 A (LEMONEX INC. et al.) 01 February 2016<br>See abstract; paragraphs [0028], [0035], [0037]-[0038], [0088], [0093]-[0096]; table 3; claims 1, 5, 7. | 1-9 |
| A | JP 07-196529 A (MORINAGA MILK IND. CO., LTD.) 01 August 1995<br>See abstract; claim 1. | 1-9 |
| A | KR 10-2014-0010285 A (SEOUL NATIONAL UNIVERSITY R&DB FOUNDATION) 24 January 2014<br>See abstract; claims 1-14. | 1-9 |
| A | KR 10-2015-0014560 A (SOGANG UNIVERSITY RESEARCH & BUSINESS DEVELOPMENT FOUNDATION) 09 February 2015<br>See abstract; claims 1-11. | 1-9 |
| PX | KR 10-2018-0091768 A (LEMONEX INC.) 16 August 2018<br>See abstract; paragraph [0069], claims 1-32. | 1-9 |

☐ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 29 OCTOBER 2019 (29.10.2019) | **29 OCTOBER 2019 (29.10.2019)** |

| Name and mailing address of the ISA/KR | Authorized officer |
|---|---|
| Korean Intellectual Property Office<br>Government Complex Daejeon Building 4, 189, Cheongsa-ro, Seo-gu, Daejeon, 35208, Republic of Korea<br>Facsimile No. +82-42-481-8578 | Telephone No. |

Form PCT/ISA/210 (second sheet) (January 2015)

INTERNATIONAL SEARCH REPORT
Information on patent family members

International application No.
PCT/KR2019/009531

| Patent document cited in search report | Publication date | Patent family member | Publication date |
|---|---|---|---|
| KR 10-2016-0011565 A | 01/02/2016 | CN 107072951 A | 18/08/2017 |
| | | EP 3173074 A1 | 31/05/2017 |
| | | EP 3173074 A4 | 07/03/2018 |
| | | JP 2017-529384 A | 05/10/2017 |
| | | JP 6426288 B2 | 21/11/2018 |
| | | KR 10-1799557 B1 | 20/11/2017 |
| | | US 2017-0172923 A1 | 22/06/2017 |
| | | WO 2016-013751 A1 | 28/01/2016 |
| JP 07-196529 A | 01/08/1995 | None | |
| KR 10-2014-0010285 A | 24/01/2014 | KR 10-1388958 B1 | 24/04/2014 |
| KR 10-2015-0014560 A | 09/02/2015 | KR 10-1528197 B1 | 15/06/2015 |
| KR 10-2018-0091768 A | 16/08/2018 | AU 2018-216591 A1 | 29/08/2018 |
| | | CA 3052561 A1 | 09/08/2018 |
| | | SG 11201907260P A | 27/09/2019 |
| | | WO 2018-143787 A1 | 09/08/2018 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Pharmaceutical Science. U.S.P., Remington, Mack Publishing Company

<110> LEMONEX INC.

<120> MEDICINAL COMPOSITION FOR TREATING WOUND

<130> 19p07004

<150> US 62/712,321
<151> 2018-07-31

<150> KR 10-2019-0092942
<151> 2019-07-31

<160> 8

<170> KoPatentIn 3.0

<210> 1
<211> 711
<212> PRT
<213> Homo sapiens

<400> 1

```
Met Lys Leu Val Phe Leu Val Leu Leu Phe Leu Gly Ala Leu Gly Leu
  1               5                  10                  15

Cys Leu Ala Gly Arg Arg Arg Arg Ser Val Gln Trp Cys Thr Val Ser
             20                  25                  30

Gln Pro Glu Ala Thr Lys Cys Phe Gln Trp Gln Arg Asn Met Arg Arg
         35                  40                  45

Val Arg Gly Pro Pro Val Ser Cys Ile Lys Arg Asp Ser Pro Ile Gln
     50                  55                  60

Cys Ile Gln Ala Ile Ala Glu Asn Arg Ala Asp Ala Val Thr Leu Asp
 65                  70                  75                  80

Gly Gly Phe Ile Tyr Glu Ala Gly Leu Ala Pro Tyr Lys Leu Arg Pro
                 85                  90                  95

Val Ala Ala Glu Val Tyr Gly Thr Glu Arg Gln Pro Arg Thr His Tyr
            100                 105                 110

Tyr Ala Val Ala Val Val Lys Lys Gly Gly Ser Phe Gln Leu Asn Glu
        115                 120                 125

Leu Gln Gly Leu Lys Ser Cys His Thr Gly Leu Arg Arg Thr Ala Gly
    130                 135                 140

Trp Asn Val Pro Ile Gly Thr Leu Arg Pro Phe Leu Asn Trp Thr Gly
145                 150                 155                 160

Pro Pro Glu Pro Ile Glu Ala Ala Val Ala Arg Phe Phe Ser Ala Ser
                165                 170                 175

Cys Val Pro Gly Ala Asp Lys Gly Gln Phe Pro Asn Leu Cys Arg Leu
            180                 185                 190

Cys Ala Gly Thr Gly Glu Asn Lys Cys Ala Phe Ser Ser Gln Glu Pro
        195                 200                 205

Tyr Phe Ser Tyr Ser Gly Ala Phe Lys Cys Leu Arg Asp Gly Ala Gly
    210                 215                 220
```

```
Asp Val Ala Phe Ile Arg Glu Ser Thr Val Phe Glu Asp Leu Ser Asp
225                 230                 235                 240

Glu Ala Glu Arg Asp Glu Tyr Glu Leu Leu Cys Pro Asp Asn Thr Arg
                245                 250                 255

Lys Pro Val Asp Lys Phe Lys Asp Cys His Leu Ala Arg Val Pro Ser
            260                 265                 270

His Ala Val Val Ala Arg Ser Val Asn Gly Lys Glu Asp Ala Ile Trp
        275                 280                 285

Asn Leu Leu Arg Gln Ala Gln Glu Lys Phe Gly Lys Asp Lys Ser Pro
    290                 295                 300

Lys Phe Gln Leu Phe Gly Ser Pro Ser Gly Gln Lys Asp Leu Leu Phe
305                 310                 315                 320

Lys Asp Ser Ala Ile Gly Phe Ser Arg Val Pro Pro Arg Ile Asp Ser
                325                 330                 335

Gly Leu Tyr Leu Gly Ser Gly Tyr Phe Thr Ala Ile Gln Asn Leu Arg
            340                 345                 350

Lys Ser Glu Glu Glu Val Ala Ala Arg Arg Ala Arg Val Val Trp Cys
        355                 360                 365

Ala Val Gly Glu Gln Glu Leu Arg Lys Cys Asn Gln Trp Ser Gly Leu
    370                 375                 380

Ser Glu Gly Ser Val Thr Cys Ser Ser Ala Ser Thr Thr Glu Asp Cys
385                 390                 395                 400

Ile Ala Leu Val Leu Lys Gly Glu Ala Asp Ala Met Ser Leu Asp Gly
                405                 410                 415

Gly Tyr Val Tyr Thr Ala Gly Lys Cys Gly Leu Val Pro Val Leu Ala
            420                 425                 430

Glu Asn Tyr Lys Ser Gln Gln Ser Ser Asp Pro Asp Pro Asn Cys Val
        435                 440                 445

Asp Arg Pro Val Glu Gly Tyr Leu Ala Val Ala Val Val Arg Arg Ser
    450                 455                 460

Asp Thr Ser Leu Thr Trp Asn Ser Val Lys Gly Lys Lys Ser Cys His
465                 470                 475                 480

Thr Ala Val Asp Arg Thr Ala Gly Trp Asn Ile Pro Met Gly Leu Leu
                485                 490                 495

Phe Asn Gln Thr Gly Ser Cys Lys Phe Asp Glu Tyr Phe Ser Gln Ser
            500                 505                 510

Cys Ala Pro Gly Ser Asp Pro Arg Ser Asn Leu Cys Ala Leu Cys Ile
        515                 520                 525

Gly Asp Glu Gln Gly Glu Asn Lys Cys Val Pro Asn Ser Asn Glu Arg
    530                 535                 540

Tyr Tyr Gly Tyr Thr Gly Ala Phe Arg Cys Leu Ala Glu Asn Ala Gly
545                 550                 555                 560
```

Asp Val Ala Phe Val Lys Asp Val Thr Val Leu Gln Asn Thr Asp Gly
565 570 575

Asn Asn Asn Glu Ala Trp Ala Lys Asp Leu Lys Leu Ala Asp Phe Ala
580 585 590

Leu Leu Cys Leu Asp Gly Lys Arg Lys Pro Val Thr Glu Ala Arg Ser
595 600 605

Cys His Leu Ala Met Ala Pro Asn His Ala Val Val Ser Arg Met Asp
610 615 620

Lys Val Glu Arg Leu Lys Gln Val Leu Leu His Gln Gln Ala Lys Phe
625 630 635 640

Gly Arg Asn Gly Ser Asp Cys Pro Asp Lys Phe Cys Leu Phe Gln Ser
645 650 655

Glu Thr Lys Asn Leu Leu Phe Asn Asp Asn Thr Glu Cys Leu Ala Arg
660 665 670

Leu His Gly Lys Thr Thr Tyr Glu Lys Tyr Leu Gly Pro Gln Tyr Val
675 680 685

Ala Gly Ile Thr Asn Leu Lys Lys Cys Ser Thr Ser Pro Leu Leu Glu
690 695 700

Ala Cys Glu Phe Leu Arg Lys
705 710

<210> 2
<211> 1207
<212> PRT
<213> Homo sapiens

<400> 2
Met Leu Leu Thr Leu Ile Ile Leu Leu Pro Val Val Ser Lys Phe Ser
1 5 10 15

Phe Val Ser Leu Ser Ala Pro Gln His Trp Ser Cys Pro Glu Gly Thr
20 25 30

Leu Ala Gly Asn Gly Asn Ser Thr Cys Val Gly Pro Ala Pro Phe Leu
35 40 45

Ile Phe Ser His Gly Asn Ser Ile Phe Arg Ile Asp Thr Glu Gly Thr
50 55 60

Asn Tyr Glu Gln Leu Val Val Asp Ala Gly Val Ser Val Ile Met Asp
65 70 75 80

Phe His Tyr Asn Glu Lys Arg Ile Tyr Trp Val Asp Leu Glu Arg Gln
85 90 95

Leu Leu Gln Arg Val Phe Leu Asn Gly Ser Arg Gln Glu Arg Val Cys
100 105 110

Asn Ile Glu Lys Asn Val Ser Gly Met Ala Ile Asn Trp Ile Asn Glu
115 120 125

Glu Val Ile Trp Ser Asn Gln Gln Glu Gly Ile Ile Thr Val Thr Asp

130 135 140

Met Lys Gly Asn Asn Ser His Ile Leu Leu Ser Ala Leu Lys Tyr Pro
145 150 155 160

Ala Asn Val Ala Val Asp Pro Val Glu Arg Phe Ile Phe Trp Ser Ser
165 170 175

Glu Val Ala Gly Ser Leu Tyr Arg Ala Asp Leu Asp Gly Val Gly Val
180 185 190

Lys Ala Leu Leu Glu Thr Ser Glu Lys Ile Thr Ala Val Ser Leu Asp
195 200 205

Val Leu Asp Lys Arg Leu Phe Trp Ile Gln Tyr Asn Arg Glu Gly Ser
210 215 220

Asn Ser Leu Ile Cys Ser Cys Asp Tyr Asp Gly Gly Ser Val His Ile
225 230 235 240

Ser Lys His Pro Thr Gln His Asn Leu Phe Ala Met Ser Leu Phe Gly
245 250 255

Asp Arg Ile Phe Tyr Ser Thr Trp Lys Met Lys Thr Ile Trp Ile Ala
260 265 270

Asn Lys His Thr Gly Lys Asp Met Val Arg Ile Asn Leu His Ser Ser
275 280 285

Phe Val Pro Leu Gly Glu Leu Lys Val Val His Pro Leu Ala Gln Pro
290 295 300

Lys Ala Glu Asp Asp Thr Trp Glu Pro Glu Gln Lys Leu Cys Lys Leu
305 310 315 320

Arg Lys Gly Asn Cys Ser Ser Thr Val Cys Gly Gln Asp Leu Gln Ser
325 330 335

His Leu Cys Met Cys Ala Glu Gly Tyr Ala Leu Ser Arg Asp Arg Lys
340 345 350

Tyr Cys Glu Asp Val Asn Glu Cys Ala Phe Trp Asn His Gly Cys Thr
355 360 365

Leu Gly Cys Lys Asn Thr Pro Gly Ser Tyr Tyr Cys Thr Cys Pro Val
370 375 380

Gly Phe Val Leu Leu Pro Asp Gly Lys Arg Cys His Gln Leu Val Ser
385 390 395 400

Cys Pro Arg Asn Val Ser Glu Cys Ser His Asp Cys Val Leu Thr Ser
405 410 415

Glu Gly Pro Leu Cys Phe Cys Pro Glu Gly Ser Val Leu Glu Arg Asp
420 425 430

Gly Lys Thr Cys Ser Gly Cys Ser Ser Pro Asp Asn Gly Gly Cys Ser
435 440 445

Gln Leu Cys Val Pro Leu Ser Pro Val Ser Trp Glu Cys Asp Cys Phe
450 455 460

Pro Gly Tyr Asp Leu Gln Leu Asp Glu Lys Ser Cys Ala Ala Ser Gly

465 470 475 480

Pro Gln Pro Phe Leu Leu Phe Ala Asn Ser Gln Asp Ile Arg His Met
485 490 495

His Phe Asp Gly Thr Asp Tyr Gly Thr Leu Leu Ser Gln Gln Met Gly
500 505 510

Met Val Tyr Ala Leu Asp His Asp Pro Val Glu Asn Lys Ile Tyr Phe
515 520 525

Ala His Thr Ala Leu Lys Trp Ile Glu Arg Ala Asn Met Asp Gly Ser
530 535 540

Gln Arg Glu Arg Leu Ile Glu Glu Gly Val Asp Val Pro Glu Gly Leu
545 550 555 560

Ala Val Asp Trp Ile Gly Arg Arg Phe Tyr Trp Thr Asp Arg Gly Lys
565 570 575

Ser Leu Ile Gly Arg Ser Asp Leu Asn Gly Lys Arg Ser Lys Ile Ile
580 585 590

Thr Lys Glu Asn Ile Ser Gln Pro Arg Gly Ile Ala Val His Pro Met
595 600 605

Ala Lys Arg Leu Phe Trp Thr Asp Thr Gly Ile Asn Pro Arg Ile Glu
610 615 620

Ser Ser Ser Leu Gln Gly Leu Gly Arg Leu Val Ile Ala Ser Ser Asp
625 630 635 640

Leu Ile Trp Pro Ser Gly Ile Thr Ile Asp Phe Leu Thr Asp Lys Leu
645 650 655

Tyr Trp Cys Asp Ala Lys Gln Ser Val Ile Glu Met Ala Asn Leu Asp
660 665 670

Gly Ser Lys Arg Arg Arg Leu Thr Gln Asn Asp Val Gly His Pro Phe
675 680 685

Ala Val Ala Val Phe Glu Asp Tyr Val Trp Phe Ser Asp Trp Ala Met
690 695 700

Pro Ser Val Ile Arg Val Asn Lys Arg Thr Gly Lys Asp Arg Val Arg
705 710 715 720

Leu Gln Gly Ser Met Leu Lys Pro Ser Ser Leu Val Val Val His Pro
725 730 735

Leu Ala Lys Pro Gly Ala Asp Pro Cys Leu Tyr Gln Asn Gly Gly Cys
740 745 750

Glu His Ile Cys Lys Lys Arg Leu Gly Thr Ala Trp Cys Ser Cys Arg
755 760 765

Glu Gly Phe Met Lys Ala Ser Asp Gly Lys Thr Cys Leu Ala Leu Asp
770 775 780

Gly His Gln Leu Leu Ala Gly Gly Glu Val Asp Leu Lys Asn Gln Val
785 790 795 800

Thr Pro Leu Asp Ile Leu Ser Lys Thr Arg Val Ser Glu Asp Asn Ile

805 810 815

Thr Glu Ser Gln His Met Leu Val Ala Glu Ile Met Val Ser Asp Gln
820 825 830

Asp Asp Cys Ala Pro Val Gly Cys Ser Met Tyr Ala Arg Cys Ile Ser
835 840 845

Glu Gly Glu Asp Ala Thr Cys Gln Cys Leu Lys Gly Phe Ala Gly Asp
850 855 860

Gly Lys Leu Cys Ser Asp Ile Asp Glu Cys Glu Met Gly Val Pro Val
865 870 875 880

Cys Pro Pro Ala Ser Ser Lys Cys Ile Asn Thr Glu Gly Gly Tyr Val
885 890 895

Cys Arg Cys Ser Glu Gly Tyr Gln Gly Asp Gly Ile His Cys Leu Asp
900 905 910

Ile Asp Glu Cys Gln Leu Gly Val His Ser Cys Gly Glu Asn Ala Ser
915 920 925

Cys Thr Asn Thr Glu Gly Gly Tyr Thr Cys Met Cys Ala Gly Arg Leu
930 935 940

Ser Glu Pro Gly Leu Ile Cys Pro Asp Ser Thr Pro Pro Pro His Leu
945 950 955 960

Arg Glu Asp Asp His His Tyr Ser Val Arg Asn Ser Asp Ser Glu Cys
965 970 975

Pro Leu Ser His Asp Gly Tyr Cys Leu His Asp Gly Val Cys Met Tyr
980 985 990

Ile Glu Ala Leu Asp Lys Tyr Ala Cys Asn Cys Val Val Gly Tyr Ile
995 1000 1005

Gly Glu Arg Cys Gln Tyr Arg Asp Leu Lys Trp Trp Glu Leu Arg His
1010 1015 1020

Ala Gly His Gly Gln Gln Gln Lys Val Ile Val Val Ala Val Cys Val
1025 1030 1035 1040

Val Val Leu Val Met Leu Leu Leu Leu Ser Leu Trp Gly Ala His Tyr
1045 1050 1055

Tyr Arg Thr Gln Lys Leu Leu Ser Lys Asn Pro Lys Asn Pro Tyr Glu
1060 1065 1070

Glu Ser Ser Arg Asp Val Arg Ser Arg Arg Pro Ala Asp Thr Glu Asp
1075 1080 1085

Gly Met Ser Ser Cys Pro Gln Pro Trp Phe Val Val Ile Lys Glu His
1090 1095 1100

Gln Asp Leu Lys Asn Gly Gly Gln Pro Val Ala Gly Glu Asp Gly Gln
1105 1110 1115 1120

Ala Ala Asp Gly Ser Met Gln Pro Thr Ser Trp Arg Gln Glu Pro Gln
1125 1130 1135

Leu Cys Gly Met Gly Thr Glu Gln Gly Cys Trp Ile Pro Val Ser Ser

1140 1145 1150

Asp Lys Gly Ser Cys Pro Gln Val Met Glu Arg Ser Phe His Met Pro
1155 1160 1165

Ser Tyr Gly Thr Gln Thr Leu Glu Gly Gly Val Glu Lys Pro His Ser
1170 1175 1180

Leu Leu Ser Ala Asn Pro Leu Trp Gln Gln Arg Ala Leu Asp Pro Pro
1185 1190 1195 1200

His Gln Met Glu Leu Thr Gln
1205

<210> 3
<211> 723
<212> PRT
<213> Homo sapiens

<400> 3
Met Trp Val Thr Lys Leu Leu Pro Ala Leu Leu Leu Gln His Val Leu
1 5 10 15

Leu His Leu Leu Leu Leu Pro Ile Ala Ile Pro Tyr Ala Glu Gly Gln
20 25 30

Arg Lys Arg Arg Asn Thr Ile His Glu Phe Lys Lys Ser Ala Lys Thr
35 40 45

Thr Leu Ile Lys Ile Asp Pro Ala Leu Lys Ile Lys Thr Lys Lys Val
50 55 60

Asn Thr Ala Asp Gln Cys Ala Asn Arg Cys Thr Arg Asn Lys Gly Leu
65 70 75 80

Pro Phe Thr Cys Lys Ala Phe Val Phe Asp Lys Ala Arg Lys Gln Cys
85 90 95

Leu Trp Phe Pro Phe Asn Ser Met Ser Ser Gly Val Lys Lys Glu Phe
100 105 110

Gly His Glu Phe Asp Leu Tyr Glu Asn Lys Asp Tyr Ile Arg Asn Cys
115 120 125

Ile Ile Gly Lys Gly Arg Ser Tyr Lys Gly Thr Val Ser Ile Thr Lys
130 135 140

Ser Gly Ile Lys Cys Gln Pro Trp Ser Ser Met Ile Pro His Glu His
145 150 155 160

Ser Tyr Arg Gly Lys Asp Leu Gln Glu Asn Tyr Cys Arg Asn Pro Arg
165 170 175

Gly Glu Glu Gly Gly Pro Trp Cys Phe Thr Ser Asn Pro Glu Val Arg
180 185 190

Tyr Glu Val Cys Asp Ile Pro Gln Cys Ser Glu Val Glu Cys Met Thr
195 200 205

Cys Asn Gly Glu Ser Tyr Arg Gly Leu Met Asp His Thr Glu Ser Gly
210 215 220

```
Lys Ile Cys Gln Arg Trp Asp His Gln Thr Pro His Arg His Lys Phe
225                 230                 235                 240

Leu Pro Glu Arg Tyr Pro Asp Lys Gly Phe Asp Asp Asn Tyr Cys Arg
                245                 250                 255

Asn Pro Asp Gly Gln Pro Arg Pro Trp Cys Tyr Thr Leu Asp Pro His
            260                 265                 270

Thr Arg Trp Glu Tyr Cys Ala Ile Lys Thr Cys Ala Asp Asn Thr Met
        275                 280                 285

Asn Asp Thr Asp Val Pro Leu Glu Thr Thr Glu Cys Ile Gln Gly Gln
    290                 295                 300

Gly Glu Gly Tyr Arg Gly Thr Val Asn Thr Ile Trp Asn Gly Ile Pro
305                 310                 315                 320

Cys Gln Arg Trp Asp Ser Gln Tyr Pro His Glu His Asp Met Thr Pro
                325                 330                 335

Glu Asn Phe Lys Cys Lys Asp Leu Arg Glu Asn Tyr Cys Arg Asn Pro
            340                 345                 350

Asp Gly Ser Glu Ser Pro Trp Cys Phe Thr Thr Asp Pro Asn Ile Arg
        355                 360                 365

Val Gly Tyr Cys Ser Gln Ile Pro Asn Cys Asp Met Ser His Gly Gln
    370                 375                 380

Asp Cys Tyr Arg Gly Asn Gly Lys Asn Tyr Met Gly Asn Leu Ser Gln
385                 390                 395                 400

Thr Arg Ser Gly Leu Thr Cys Ser Met Trp Asp Lys Asn Met Glu Asp
                405                 410                 415

Leu His Arg His Ile Phe Trp Glu Pro Asp Ala Ser Lys Leu Asn Glu
            420                 425                 430

Asn Tyr Cys Arg Asn Pro Asp Asp Asp Ala His Gly Pro Trp Cys Tyr
        435                 440                 445

Thr Gly Asn Pro Leu Ile Pro Trp Asp Tyr Cys Pro Ile Ser Arg Cys
    450                 455                 460

Glu Gly Asp Thr Thr Pro Thr Ile Val Asn Leu Asp His Pro Val Ile
465                 470                 475                 480

Ser Cys Ala Lys Thr Lys Gln Leu Arg Val Val Asn Gly Ile Pro Thr
                485                 490                 495

Arg Thr Asn Ile Gly Trp Met Val Ser Leu Arg Tyr Arg Asn Lys His
            500                 505                 510

Ile Cys Gly Gly Ser Leu Ile Lys Glu Ser Trp Val Leu Thr Ala Arg
        515                 520                 525

Gln Cys Phe Pro Ser Arg Asp Leu Lys Asp Tyr Glu Ala Trp Leu Gly
    530                 535                 540

Ile His Asp Val His Gly Arg Gly Asp Glu Lys Cys Lys Gln Val Leu
545                 550                 555                 560
```

```
Asn Val Ser Gln Leu Val Tyr Gly Pro Glu Gly Ser Asp Leu Val Leu
                565                 570                 575

Met Lys Leu Ala Arg Pro Ala Val Leu Asp Asp Phe Val Ser Thr Ile
            580                 585                 590

Asp Leu Pro Asn Tyr Gly Cys Thr Ile Pro Glu Lys Thr Ser Cys Ser
        595                 600                 605

Val Tyr Gly Trp Gly Tyr Thr Gly Leu Ile Asn Tyr Asp Gly Leu Leu
    610                 615                 620

Arg Val Ala His Leu Tyr Ile Met Gly Asn Glu Lys Cys Ser Gln His
625                 630                 635                 640

His Arg Gly Lys Val Thr Leu Asn Glu Ser Glu Ile Cys Ala Gly Ala
                645                 650                 655

Glu Lys Ile Gly Ser Gly Pro Cys Glu Gly Asp Tyr Gly Gly Pro Leu
            660                 665                 670

Val Cys Glu Gln His Lys Met Arg Met Val Leu Gly Val Ile Val Pro
        675                 680                 685

Gly Arg Gly Cys Ala Ile Pro Asn Arg Pro Gly Ile Phe Val Arg Val
    690                 695                 700

Ala Tyr Tyr Ala Lys Trp Ile His Lys Ile Ile Leu Thr Tyr Lys Val
705                 710                 715                 720

Pro Gln Ser



<210>  4
<211>  191
<212>  PRT
<213>  Homo sapiens

<400>  4
Met Asn Phe Leu Leu Ser Trp Val His Trp Ser Leu Ala Leu Leu Leu
1               5                   10                  15

Tyr Leu His His Ala Lys Trp Ser Gln Ala Ala Pro Met Ala Glu Gly
            20                  25                  30

Gly Gly Gln Asn His His Glu Val Val Lys Phe Met Asp Val Tyr Gln
        35                  40                  45

Arg Ser Tyr Cys His Pro Ile Glu Thr Leu Val Asp Ile Phe Gln Glu
    50                  55                  60

Tyr Pro Asp Glu Ile Glu Tyr Ile Phe Lys Pro Ser Cys Val Pro Leu
65                  70                  75                  80

Met Arg Cys Gly Gly Cys Cys Asn Asp Glu Gly Leu Glu Cys Val Pro
                85                  90                  95

Thr Glu Glu Ser Asn Ile Thr Met Gln Ile Met Arg Ile Lys Pro His
            100                 105                 110

Gln Gly Gln His Ile Gly Glu Met Ser Phe Leu Gln His Asn Lys Cys
        115                 120                 125
```

Glu Cys Arg Pro Lys Lys Asp Arg Ala Arg Gln Glu Asn Pro Cys Gly
130 135 140

Pro Cys Ser Glu Arg Arg Lys His Leu Phe Val Gln Asp Pro Gln Thr
145 150 155 160

Cys Lys Cys Ser Cys Lys Asn Thr Asp Ser Arg Cys Lys Ala Arg Gln
165 170 175

Leu Glu Leu Asn Glu Arg Thr Cys Arg Cys Asp Lys Pro Arg Arg
180 185 190

<210> 5
<211> 6212
<212> DNA
<213> Artificial Sequence

<220>
<223> pLTF

<400> 5
cgagaagcgg aagagcgccc aatacgcaaa ccgcctctcc ccgcgcgttg gccgattcat 60

taatgcagct ggcacgacag gtttcccgac tggaaagcgg gcagtgagcg caacgcaatt 120

aatgtgagtt agctcactca ttaggcaccc caggctttac actttatgct tccggctcgt 180

atgttgtgtg gaattgtgag cggataacaa tttcacacag gaaacagcta tgaccatgat 240

tacgccaagc tctagctaga ggtcgaccaa ttctcatgtt tgacagctta tcatcgcaga 300

tccgggcaac gttgttgcca ttgctgcagg cgcagaactg gtaggtatgg aagatctata 360

cattgaatca atattggcaa ttagccatat tagtcattgg ttatatagca taaatcaata 420

ttggctattg gccattgcat acgttgtatc tatatcataa tatgtacatt tatattggct 480

catgtccaat atgaccgcca tgttgacatt gattattgac tagttattaa tagtaatcaa 540

ttacggggtc attagttcat agcccatata tggagttccg cgttacataa cttacggtaa 600

atggcccgcc tggctgaccg cccaacgacc cccgcccatt gacgtcaata atgacgtatg 660

ttcccatagt aacgccaata gggactttcc attgacgtca atgggtggag tatttacggt 720

aaactgccca cttggcagta catcaagtgt atcatatgcc aagtccgccc cctattgacg 780

tcaatgacgg taaatggccc gcctggcatt atgcccagta catgacctta cgggactttc 840

ctacttggca gtacatctac gtattagtca tcgctattac catggtgatg cggttttggc 900

agtacaccaa tgggcgtgga tagcggtttg actcacgggg atttccaagt ctccacccca 960

ttgacgtcaa tgggagtttg ttttggcacc aaaatcaacg ggactttcca aaatgtcgta 1020

ataaccccgc cccgttgacg caaatgggcg gtaggcgtgt acggtgggag gtctatataa 1080

gcagagctcg tttagtgaac cgtcagatcc tcactctctt ccgcatcgct gtctgcgagg 1140

gccagctgtt gggctcgcgg ttgaggacaa actcttcgcg gtctttccag tactcttgga 1200
tcggaaaccc gtcggcctcc gaacggtact ccgccaccga gggacctgag cgagtccgca 1260
tcgaccggat cggaaaacct ctcgagaaag gcgtctaacc agtcacagtc gcaaggtagg 1320
ctgagcaccg tggcgggcgg cagcgggtgg cggtcggggt tgtttctggc ggaggtgctg 1380
ctgatgatgt aattaaagta ggcggtcttg agacggcgga tggtcgaggt gaggtgtggc 1440
aggcttgaga tccagctgtt ggggtgagta ctccctctca aaagcgggca ttacttctgc 1500
gctaagattg tcagtttcca aaaacgagga ggatttgata ttcacctggc ccgatctggc 1560
catacacttg agtgacaatg acatccactt tgcctttctc tccacaggtg tccactccca 1620
ggtccaagtt taaactgcgg ccgccaccat ggccctggtg tttctggtgc tgctgttcct 1680
gggcgccctg ggactgtgtc tggccggcag gcggagatct gtgcagtggt gcgccgtgtc 1740
tcagcccgag gccaccaagt gcttccagtg gcagcggaac atgcggaaag tgcgcggacc 1800
ccccgtgtcc tgcatcaagc gcgacagccc catccagtgc atccaggcca ttgccgagaa 1860
cagagccgac gccgtgaccc tggacggcgg ctttatctac gaagccggac tggcccctta 1920
taagctgagg cccgtggccg ccgaagtgta cggcaccgag cggcagccca gaacccacta 1980
ctacgccgtg gccgtggtca agaagggcgg cagcttccag ctgaacgagc tgcagggcct 2040
gaagtcctgc cacaccggcc tgagaagaac cgccggctgg aacgtgccta tcggcaccct 2100
gcggcccttt ctgaactgga ccggccctcc tgagcctatt gaggccgctg tggctagatt 2160
cttcagcgcc agctgcgtgc ctggcgccga taagggccag ttccccaacc tgtgcagact 2220
gtgtgctgga accggcgaga acaagtgcgc cttcagcagc caggaaccct acttcagcta 2280
cagcggcgcc ttcaagtgcc tgagagatgg cgctggcgac gtggccttca tccgggagag 2340
caccgtgttc gaggacctga gcgacgaggc cgagagggac gagtacgagc tgctgtgccc 2400
cgacaacacc cggaagcccg tggacaagtt caaggactgc cacctggcca gagtgccttc 2460
tcacgccgtg gtggccagaa gcgtgaacgg caaagaggac gccatctgga atctgctgcg 2520
gcaggcccag gaaaagttcg gcaaggacaa gagccccaag ttccagctgt tcggctctcc 2580
tagcggccag aaggatctgc tgttcaagga cagcgccatc ggcttcagca gagtgccccc 2640
cagaatcgac agcggcctgt acctgggcag cggctacttc accgccatcc agaacctgag 2700
aaagtccgag gaagaggtgg ccgccagacg ggctagagtc gtgtggtgtg ccgtgggcga 2760
gcaggaactg cggaagtgca accagtggag cggcctgagc gagggcagcg tgacatgtag 2820
cagcgccagc accaccgagg actgtatcgc cctggtgctg aagggcgagg ccgatgccat 2880
gtctctggat ggaggctacg tgtacaccgc cggcaagtgt ggactggtgc ccgtgctggc 2940
cgagaactac aagagccagc agagcagcga ccccgacccc aactgcgtgg acagacccgt 3000
ggagggatac ctggccgtgg ctgtcgtgag aagaagcgac accagcctga cctggaacag 3060

cgtgaagggc aagaagtctt gtcacaccgc cgtggaccgg accgccgggt ggaatatccc 3120

catgggcctg ctgtttaacc agaccggcag ctgcaagttc gacgagtact tcagccagag 3180

ctgcgcccct ggctctgatc ctagaagcaa cctgtgcgcc ctgtgcatcg gagatgagca 3240

gggcgagaat aagtgtgtgc ccaacagcaa cgagcggtac tacggctaca caggcgcctt 3300

tcgctgtctg gctgaaaatg ccggggatgt ggccttcgtg aaggacgtga ccgtgctgca 3360

gaacaccgac ggcaacaaca acgaggcctg ggccaaggac ctgaagctgg ccgacttcgc 3420

cctgctgtgc ctggacggca agagaaagcc cgtgaccgag gccagatcct gtcacctggc 3480

tatggctccc aaccacgctg tggtgtcccg gatggacaag gtcgaacgcc tgaaacaggt 3540

gctgctgcac cagcaggcca agttcggcag aaacggcagc gactgccccg ataagttctg 3600

cctgttccag agcgagacaa agaacctcct gttcaacgac aacaccgagt gcctggccag 3660

actgcacggc aagaccacct acgagaagta cctgggccct cagtacgtgg ccggcatcac 3720

caacctgaag aagtgcagca ccagcccct gctggaagcc tgcgagttcc tgcggaaggg 3780

cgcgccgtcg acctccatca cggcctataa gagtgagggg gagtcagcgg agttctcctt 3840

cccactcaac cttggagagg aaagcctgca gggagagttg agatggaagg cagagaaggc 3900

tccttcttcc cagtcctgga tcaccttctc cctaaagaac caaaaggtgt ctgtgcagaa 3960

gtctactagc aaccccaagt tccagctgtc cgaaacgctc ccactcaccc ttcagatacc 4020

ccaggtctcc cttcagtttg ctggttctgg caacctgacc ctgactctgg acagagggat 4080

actgtatcag gaagtgaacc tggtggtgat gaaagtgact cagcccgaca gcaacacttt 4140

gacctgtgag gtgatgggac ccacctcacc caagatgaga ctgatcttga agcaggagaa 4200

tcaggaggcc agggtctcca ggcaggagaa agtgattcaa gtgcaggccc ctgaagcagg 4260

ggtgtggcaa tgtctactga gtgaaggtga agaggtcaag atggactcca agatccaggt 4320

tttatccaaa gggttgaatt ccggatcact gcatcatatt ctggatgcac agaaaatggt 4380

gtggaatcat cgttaagtct agaaagcttg atatctagta atgagtttaa acgggggagg 4440

ctaactgaaa cacggaagga gacaataccg gaaggaaccc gcgctatgac ggcaataaaa 4500

agacagaata aaacgcacgg gtgttgggtc gtttgttcat aaacgcgggg ttcggtccca 4560

gggctggcac tctgtcgata ccccaccgag accccattgg ggccaatacg cccgcgtttc 4620

ttccttttcc ccaccccacc ccccaagttc gggtgaaggc ccagggctcg cagccaacgt 4680

cggggcggca ggccctgcca tagggatcca tgagccatat tcaacgggaa acgtcgaggc 4740

cgcgattaaa ttccaacatg gatgctgatt tatatgggta taaatgggct cgcgataatg 4800

tcgggcaatc aggtgcgaca atctatcgct tgtatgggaa gcccgatgcg ccagagttgt 4860

ttctgaaaca tggcaaaggt agcgttgcca atgatgttac agatgagatg gtcagactaa 4920

actggctgac ggaatttatg cctcttccga ccatcaagca ttttatccgt actcctggtg 4980

atgcatggtt actcaccact gcgatccccg gaaaaacagc attccaggta ttagaagaat 5040

atcctgattc aggtgaaaat attgttgatg cgctggcagt gttcctgcgc cggttgcatt 5100

cgattcctgt ttgtaattgt ccttttaaca gcgatcgcgt atttcgtctc gctcaggcgc 5160

aatcacgaat gaataacggt ttggttgatg cgagtgattt tgatgacgag cgtaatggct 5220

ggcctgttga acaagtctgg aaagaaatgc ataaactttt gccattctca ccggattcag 5280

tcgtcactca tggtgatttc tcacttgata accttatttt tgacgagggg aaattaatag 5340

gttgtattga tgttggacga gtcggaatcg cagaccgata ccaggatctt gccatcctat 5400

ggaactgcct cggtgagttt tctccttcat tacagaaacg gctttttcaa aaatatggta 5460

ttgataatcc tgatatgaat aaattgcagt ttcatttgat gctcgatgag tttttctaat 5520

cagaattggt taattggttg taacattatt cagattgggc cccgttccac tgagcgtcag 5580

accccgtaga aaagatcaaa ggatcttctt gagatccttt ttttctgcgc gtaatctgct 5640

gcttgcaaac aaaaaaacca ccgctaccag cggtggtttg tttgccggat caagagctac 5700

caactctttt tccgaaggta actggcttca gcagagcgca gataccaaat actgttcttc 5760

tagtgtagcc gtagttaggc caccacttca agaactctgt agcaccgcct acatacctcg 5820

ctctgctaat cctgttacca gtggctgctg ccagtggcga taagtcgtgt cttaccgggt 5880

tggactcaag acgatagtta ccggataagg cgcagcggtc gggctgaacg gggggttcgt 5940

gcacacagcc cagcttggag cgaacgacct acaccgaact gagataccta cagcgtgagc 6000

tatgagaaag cgccacgctt cccgaaggga gaaaggcgga caggtatccg gtaagcggca 6060

gggtcggaac aggagagcgc acgagggagc ttccaggggg aaacgcctgg tatctttata 6120

gtcctgtcgg gtttcgccac ctctgacttg agcgtcgatt tttgtgatgc tcgtcagggg 6180

ggcggagcct atggaaaaac gccagcaacg cg 6212

<210> 6
<211> 7088
<212> DNA
<213> Artificial Sequence

<220>
<223> pEGF

<400> 6
cgagaagcgg aagagcgccc aatacgcaaa ccgcctctcc ccgcgcgttg gccgattcat 60

taatgcagct ggcacgacag gtttcccgac tggaaagcgg gcagtgagcg caacgcaatt 120

aatgtgagtt agctcactca ttaggcaccc caggctttac actttatgct tccggctcgt 180

atgttgtgtg gaattgtgag cggataacaa tttcacacag gaaacagcta tgaccatgat 240

```
tacgccaagc tctagctaga ggtcgaccaa ttctcatgtt tgacagctta tcatcgcaga 300
tccgggcaac gttgttgcca ttgctgcagg cgcagaactg gtaggtatgg aagatctata 360
cattgaatca atattggcaa ttagccatat tagtcattgg ttatatagca taaatcaata 420
ttggctattg gccattgcat acgttgtatc tatatcataa tatgtacatt tatattggct 480
catgtccaat atgaccgcca tgttgacatt gattattgac tagttattaa tagtaatcaa 540
ttacggggtc attagttcat agcccatata tggagttccg cgttacataa cttacggtaa 600
atggcccgcc tggctgaccg cccaacgacc cccgcccatt gacgtcaata atgacgtatg 660
ttcccatagt aacgccaata gggactttcc attgacgtca atgggtggag tatttacggt 720
aaactgccca cttggcagta catcaagtgt atcatatgcc aagtccgccc cctattgacg 780
tcaatgacgg taaatggccc gcctggcatt atgcccagta catgacctta cgggactttc 840
ctacttggca gtacatctac gtattagtca tcgctattac catggtgatg cggttttggc 900
agtacaccaa tgggcgtgga tagcggtttg actcacgggg atttccaagt ctccacccca 960
ttgacgtcaa tgggagtttg ttttggcacc aaaatcaacg ggactttcca aaatgtcgta 1020
ataaccccgc cccgttgacg caaatgggcg gtaggcgtgt acggtgggag gtctatataa 1080
gcagagctcg tttagtgaac cgtcagatcc tcactctctt ccgcatcgct gtctgcgagg 1140
gccagctgtt gggctcgcgg ttgaggacaa actcttcgcg gtctttccag tactcttgga 1200
tcggaaaccc gtcggcctcc gaacggtact ccgccaccga gggacctgag cgagtccgca 1260
tcgaccggat cggaaaacct ctcgagaaag gcgtctaacc agtcacagtc gcaaggtagg 1320
ctgagcaccg tggcgggcgg cagcgggtgg cggtcggggt tgtttctggc ggaggtgctg 1380
ctgatgatgt aattaaagta ggcggtcttg agacggcgga tggtcgaggt gaggtgtggc 1440
aggcttgaga tccagctgtt ggggtgagta ctccctctca aaagcgggca ttacttctgc 1500
gctaagattg tcagtttcca aaaacgagga ggatttgata ttcacctggc ccgatctggc 1560
catacacttg agtgacaatg acatccactt tgcctttctc tccacaggtg tccactccca 1620
ggtccaagtt taaactgcgg ccgccaccat gctgctcact cttatcattc tgttgccagt 1680
agtttcaaaa tttagttttg ttagtctctc agcaccgcag cactggagct gtcctgaagg 1740
tactctcgca ggaaatggga attctacttg tgtgggtcct gcacccttct taattttctc 1800
ccatggaaat agtatcttta ggattgacac agaaggaacc aattatgagc aattggtggt 1860
ggatgctggt gtctcagtga tcatggattt tcattataat gagaaaagaa tctattgggt 1920
ggatttagaa agacaacttt tgcaaagagt tttctgaat gggtcaaggc aagagagagt 1980
atgtaatata gagaaaaatg tttctggaat ggcaataaat tggataaatg aagaagttat 2040
ttggtcaaat caacaggaag gaatcattac agtaacagat atgaaaggaa ataattccca 2100
cattctttta agtgctttaa aatatcctgc aaatgtagca gttgatccag tagaaaggtt 2160
```

tatattttgg tcttcagagg tggctggaag cctttataga gcagatctcg atggtgtggg 2220

agtgaaggct ctgttggaga catcagagaa aataacagct gtgtcattgg atgtgcttga 2280

taagcggctg ttttggattc agtacaacag agaaggaagc aattctctta tttgctcctg 2340

tgattatgat ggaggttctg tccacattag taaacatcca acacagcata atttgtttgc 2400

aatgtccctt tttggtgacc gtatcttcta ttcaacatgg aaaatgaaga caatttggat 2460

agccaacaaa cacactggaa aggacatggt tagaattaac ctccattcat catttgtacc 2520

acttggtgaa ctgaaagtag tgcatccact tgcacaaccc aaggcagaag atgacacttg 2580

ggagcctgag cagaaacttt gcaaattgag gaaaggaaac tgcagcagca ctgtgtgtgg 2640

gcaagacctc cagtcacact tgtgcatgtg tgcagaggga tacgccctaa gtcgagaccg 2700

gaagtactgt gaagatgtta atgaatgtgc tttttggaat catggctgta ctcttgggtg 2760

taaaaacacc cctggatcct attactgcac gtgccctgta ggatttgttc tgcttcctga 2820

tgggaaacga tgtcatcaac ttgtttcctg tccacgcaat gtgtctgaat gcagccatga 2880

ctgtgttctg acatcagaag gtcccttatg tttctgtcct gaaggctcag tgcttgagag 2940

agatgggaaa acatgtagcg gttgttcctc acccgataat ggtggatgta gccagctctg 3000

cgttcctctt agcccagtat cctgggaatg tgattgcttt cctgggtatg acctacaact 3060

ggatgaaaaa agctgtgcag cttcaggacc acaaccattt ttgctgtttg ccaattctca 3120

agatattcga cacatgcatt ttgatggaac agactatgga actctgctca gccagcagat 3180

gggaatggtt tatgccctag atcatgaccc tgtggaaaat aagatatact ttgcccatac 3240

agccctgaag tggatagaga gagctaatat ggatggttcc cagcgagaaa ggcttattga 3300

ggaaggagta gatgtgccag aaggtcttgc tgtggactgg attggccgta gattctattg 3360

gacagacaga gggaaatctc tgattggaag gagtgattta aatgggaaac gttccaaaat 3420

aatcactaag gagaacatct ctcaaccacg aggaattgct gttcatccaa tggccaagag 3480

attattctgg actgatacag ggattaatcc acgaattgaa agttcttccc tccaaggcct 3540

tggccgtctg gttatagcca gctctgatct aatctggccc agtggaataa cgattgactt 3600

cttaactgac aagttgtact ggtgcgatgc caagcagtct gtgattgaaa tggccaatct 3660

ggatggttca aaacgccgaa gacttaccca gaatgatgta ggtcacccat ttgctgtagc 3720

agtgtttgag gattatgtgt ggttctcaga ttgggctatg ccatcagtaa taagagtaaa 3780

caagaggact ggcaaagata gagtacgtct ccaaggcagc atgctgaagc cctcatcact 3840

ggttgtggtt catccattgg caaaaccagg agcagatccc tgcttatatc aaaacggagg 3900

ctgtgaacat atttgcaaaa agaggcttgg aactgcttgg tgttcgtgtc gtgaaggttt 3960

tatgaaagcc tcagatggga aaacgtgtct ggctctggat ggtcatcagc tgttggcagg 4020

tggtgaagtt gatctaaaga accaagtaac accattggac atcttgtcca agactagagt 4080

gtcagaagat aacattacag aatctcaaca catgctagtg gctgaaatca tggtgtcaga 4140

tcaagatgac tgtgctcctg tgggatgcag catgtatgct cggtgtattt cagagggaga 4200

ggatgccaca tgtcagtgtt tgaaaggatt tgctggggat ggaaaactat gttctgatat 4260

agatgaatgt gagatgggtg tcccagtgtg ccccccctgcc tcctccaagt gcatcaacac 4320

cgaaggtggt tatgtctgcc ggtgctcaga aggctaccaa ggagatggga ttcactgtct 4380

tgatattgat gagtgccaac tgggggtgca cagctgtgga gagaatgcca gctgcacaaa 4440

tacagaggga ggctatacct gcatgtgtgc tggacgcctg tctgaaccag gactgatttg 4500

ccctgactct actccacccc ctcacctcag ggaagatgac caccactatt ccgtaagaaa 4560

tagtgactct gaatgtcccc tgtcccacga tgggtactgc ctccatgatg gtgtgtgcat 4620

gtatattgaa gcattggaca agtatgcatg caactgtgtt gttggctaca tcggggagcg 4680

atgtcagtac cgagacctga agtggtggga actgcgccac gctggccacg ggcagcagca 4740

gaaggtcatc gtggtggctg tctgcgtggt ggtgcttgtc atgctgctcc tcctgagcct 4800

gtgggggggcc cactactaca ggactcagaa gctgctatcg aaaaacccaa agaatcctta 4860

tgaggagtcg agcagagatg tgaggagtcg caggcctgct gacactgagg atgggatgtc 4920

ctcttgccct caaccttggt ttgtggttat aaaagaacac caagacctca agaatggggg 4980

tcaaccagtg gctggtgagg atggccaggc agcagatggg tcaatgcaac caacttcatg 5040

gaggcaggag ccccagttat gtggaatggg cacagagcaa ggctgctgga ttccagtatc 5100

cagtgataag ggctcctgtc cccaggtaat ggagcgaagc tttcatatgc cctcctatgg 5160

gacacagacc cttgaagggg gtgtcgagaa gccccattct ctcctatcag ctaacccatt 5220

atggcaacaa agggccctgg acccaccaca ccaaatggag ctgactcagt gagtctagaa 5280

agcttgatat ctagtaatga gtttaaacgg gggaggctaa ctgaaacacg gaaggagaca 5340

ataccggaag gaacccgcgc tatgacggca ataaaaagac agaataaaac gcacgggtgt 5400

tgggtcgttt gttcataaac gcggggttcg gtcccagggc tggcactctg tcgatacccc 5460

accgagaccc cattggggcc aatacgcccg cgtttcttcc ttttccccac cccacccccc 5520

aagttcgggt gaaggcccag ggctcgcagc caacgtcggg gcggcaggcc ctgccatagg 5580

gatccatgag ccatattcaa cgggaaacgt cgaggccgcg attaaattcc aacatggatg 5640

ctgatttata tgggtataaa tgggctcgcg ataatgtcgg gcaatcaggt gcgacaatct 5700

atcgcttgta tgggaagccc gatgcgccag agttgtttct gaaacatggc aaaggtagcg 5760

ttgccaatga tgttacagat gagatggtca gactaaactg gctgacggaa tttatgcctc 5820

ttccgaccat caagcatttt atccgtactc ctggtgatgc atggttactc accactgcga 5880

tccccggaaa aacagcattc caggtattag aagaatatcc tgattcaggt gaaaatattg 5940

ttgatgcgct ggcagtgttc ctgcgccggt tgcattcgat tcctgtttgt aattgtcctt 6000

ttaacagcga tcgcgtattt cgtctcgctc aggcgcaatc acgaatgaat aacggtttgg 6060

ttgatgcgag tgattttgat gacgagcgta atggctggcc tgttgaacaa gtctggaaag 6120

aaatgcataa acttttgcca ttctcaccgg attcagtcgt cactcatggt gatttctcac 6180

ttgataacct tatttttgac gaggggaaat taataggttg tattgatgtt ggacgagtcg 6240

gaatcgcaga ccgataccag gatcttgcca tcctatggaa ctgcctcggt gagttttctc 6300

cttcattaca gaaacggctt tttcaaaaat atggtattga taatcctgat atgaataaat 6360

tgcagtttca tttgatgctc gatgagtttt tctaatcaga attggttaat tggttgtaac 6420

attattcaga ttgggccccg ttccactgag cgtcagaccc cgtagaaaag atcaaaggat 6480

cttcttgaga tcctttttt ctgcgcgtaa tctgctgctt gcaaacaaaa aaaccaccgc 6540

taccagcggt ggtttgtttg ccggatcaag agctaccaac tctttttccg aaggtaactg 6600

gcttcagcag agcgcagata ccaaatactg ttcttctagt gtagccgtag ttaggccacc 6660

acttcaagaa ctctgtagca ccgcctacat acctcgctct gctaatcctg ttaccagtgg 6720

ctgctgccag tggcgataag tcgtgtctta ccgggttgga ctcaagacga tagttaccgg 6780

ataaggcgca gcggtcgggc tgaacggggg gttcgtgcac acagcccagc ttggagcgaa 6840

cgacctacac cgaactgaga tacctacagc gtgagctatg agaaagcgcc acgcttcccg 6900

aagggagaaa ggcggacagg tatccggtaa gcggcagggt cggaacagga gagcgcacga 6960

gggagcttcc agggggaaac gcctggtatc tttatagtcc tgtcgggttt cgccacctct 7020

gacttgagcg tcgatttttg tgatgctcgt caggggggcg gagcctatgg aaaaacgcca 7080

gcaacgcg 7088

<210> 7
<211> 5651
<212> DNA
<213> Artificial Sequence

<220>
<223> pHGF

<400> 7
cgagaagcgg aagagcgccc aatacgcaaa ccgcctctcc ccgcgcgttg gccgattcat 60

taatgcagct ggcacgacag gtttcccgac tggaaagcgg gcagtgagcg caacgcaatt 120

aatgtgagtt agctcactca ttaggcaccc caggctttac actttatgct tccggctcgt 180

atgttgtgtg gaattgtgag cggataacaa tttcacacag gaaacagcta tgaccatgat 240

tacgccaagc tctagctaga ggtcgaccaa ttctcatgtt tgacagctta tcatcgcaga 300

tccgggcaac gttgttgcca ttgctgcagg cgcagaactg gtaggtatgg aagatctata 360

```
cattgaatca atattggcaa ttagccatat tagtcattgg ttatatagca taaatcaata      420
ttggctattg gccattgcat acgttgtatc tatatcataa tatgtacatt tatattggct      480
catgtccaat atgaccgcca tgttgacatt gattattgac tagttattaa tagtaatcaa      540
ttacggggtc attagttcat agcccatata tggagttccg cgttacataa cttacggtaa      600
atggcccgcc tggctgaccg cccaacgacc cccgcccatt gacgtcaata atgacgtatg      660
ttcccatagt aacgccaata gggactttcc attgacgtca atgggtggag tatttacggt      720
aaactgccca cttggcagta catcaagtgt atcatatgcc aagtccgccc cctattgacg      780
tcaatgacgg taaatggccc gcctggcatt atgcccagta catgacctta cgggactttc      840
ctacttggca gtacatctac gtattagtca tcgctattac catggtgatg cggttttggc      900
agtacaccaa tgggcgtgga tagcggtttg actcacgggg atttccaagt ctccacccca      960
ttgacgtcaa tgggagtttg ttttggcacc aaaatcaacg ggactttcca aaatgtcgta     1020
ataaccccgc cccgttgacg caaatgggcg gtaggcgtgt acggtgggag gtctatataa     1080
gcagagctcg tttagtgaac cgtcagatcc tcactctctt ccgcatcgct gtctgcgagg     1140
gccagctgtt gggctcgcgg ttgaggacaa actcttcgcg gtctttccag tactcttgga     1200
tcggaaaccc gtcggcctcc gaacggtact ccgccaccga gggacctgag cgagtccgca     1260
tcgaccggat cggaaaacct ctcgagaaag gcgtctaacc agtcacagtc gcaaggtagg     1320
ctgagcaccg tggcgggcgg cagcgggtgg cggtcggggt tgtttctggc ggaggtgctg     1380
ctgatgatgt aattaaagta ggcggtcttg agacggcgga tggtcgaggt gaggtgtggc     1440
aggcttgaga tccagctgtt ggggtgagta ctccctctca aaagcgggca ttacttctgc     1500
gctaagattg tcagtttcca aaaacgagga ggatttgata ttcacctggc ccgatctggc     1560
catacacttg agtgacaatg acatccactt tgcctttctc tccacaggtg tccactccca     1620
ggtccaagtt taaactgcgg ccgccaccat gtgggtgacc aaactcctgc cagccctgct     1680
gctgcagcat gtcctcctgc atctcctcct gctccccatc gccatcccct atgcagaggg     1740
acaaaggaaa agaagaaata caattcatga attcaaaaaa tcagcaaaga ctaccctaat     1800
caaaatagat ccagcactga agataaaaac caaaaaagtg aatactgcag accaatgtgc     1860
taatagatgt actaggaata aaggacttcc attcacttgc aaggcttttg tttttgataa     1920
agcaagaaaa caatgcctct ggttcccctt caatagcatg tcaagtggag tgaaaaaaga     1980
atttggccat gaatttgacc tctatgaaaa caaagactac attagaaact gcatcattgg     2040
taaaggacgc agctacaagg gaacagtatc tatcactaag agtggcatca aatgtcagcc     2100
ctggagttcc atgataccac acgaacacag ctttttgcct tcgagctatc ggggtaaaga     2160
cctacaggaa aactactgtc gaaatcctcg aggggaagaa gggggaccct ggtgtttcac     2220
```

aagcaatcca gaggtacgct acgaagtctg tgacattcct cagtgttcag aagttgaatg 2280
catgacctgc aatggggaga gttatcgagg tctcatggat catacagaat caggcaagat 2340
ttgtcagcgc tgggatcatc agacaccaca ccggcacaaa ttcttgcctg aaagatatcc 2400
cgacaagggc tttgatgata attattgccg caatcccgat ggccagccga ggccatggtg 2460
ctatactctt gaccctcaca cccgctggga gtactgtgca attaaaacat gcgctgacaa 2520
tactatgaat gacactgatg ttcctttgga aacaactgaa tgcatccaag gtcaaggaga 2580
aggctacagg ggcactgtca ataccatttg gaatggaatt ccatgtcagc gttgggattc 2640
tcagtatcct cacgagcatg acatgactcc tgaaaatttc aagtgcaagg acctacgaga 2700
aaattactgc cgaaatccag atgggtctga atcaccctgg tgttttacca ctgatccaaa 2760
catccgagtt ggctactgct cccaaattcc aaactgtgat atgtcacatg gacaagattg 2820
ttatcgtggg aatggcaaaa attatatggg caacttatcc caaacaagat ctggactaac 2880
atgttcaatg tgggacaaga acatggaaga cttacatcgt catatcttct gggaaccaga 2940
tgcaagtaag ctgaatgaga attactgccg aaatccagat gatgatgctc atggaccctg 3000
gtgctacacg ggaaatccac tcattccttg ggattattgc cctatttctc gttgtgaagg 3060
tgataccaca cctacaatag tcaatttaga ccatcccgta atatcttgtg ccaaaacgaa 3120
acaattgcga gttgtaaatg ggattccaac acgaacaaac ataggatgga tggttagttt 3180
gagatacaga aataaacata tctgcggagg atcattgata aaggagagtt gggttcttac 3240
tgcacgacag tgtttccctt ctcgagactt gaaagattat gaagcttggc ttggaattca 3300
tgatgtccac ggaagaggag atgagaaatg caaacaggtt ctcaatgttt cccagctggt 3360
atatggccct gaaggatcag atctggtttt aatgaagctt gccaggcctg ctgtcctgga 3420
tgattttgtt agtacgattg atttacctaa ttatggatgc acaattcctg aaaagaccag 3480
ttgcagtgtt tatggctggg gctacactgg attgatcaac tatgatggcc tattacgagt 3540
ggcacatctc tatataatgg gaaatgagaa atgcagccag catcatcgag ggaaggtgac 3600
tctgaatgag tctgaaatat gtgctggggc tgaaaagatt ggatcaggac catgtgaggg 3660
ggattatggt ggcccacttg tttgtgagca acataaaatg agaatggttc ttggtgtcat 3720
tgttcctggt cgtggatgtg ccattccaaa tcgtcctggt atttttgtcc gagtagcata 3780
ttatgcaaaa tggatacaca aaattatttt aacatataag gtaccacagt cataggtcta 3840
gaaagcttga tatctagtaa tgagtttaaa cgggggaggc taactgaaac acggaaggag 3900
acaataccgg aaggaacccg cgctatgacg gcaataaaaa gacagaataa aacgcacggg 3960
tgttgggtcg tttgttcata aacgcggggt tcggtcccag ggctggcact ctgtcgatac 4020
cccaccgaga ccccattggg gccaatacgc ccgcgtttct tccttttccc caccccaccc 4080
cccaagttcg ggtgaaggcc caggctcgc agccaacgtc ggggcggcag gccctgccat 4140

agggatccat gagccatatt caacgggaaa cgtcgaggcc gcgattaaat tccaacatgg 4200

atgctgattt atatgggtat aaatgggctc gcgataatgt cgggcaatca ggtgcgacaa 4260

tctatcgctt gtatgggaag cccgatgcgc cagagttgtt tctgaaacat ggcaaaggta 4320

gcgttgccaa tgatgttaca gatgagatgg tcagactaaa ctggctgacg gaatttatgc 4380

ctcttccgac catcaagcat tttatccgta ctcctggtga tgcatggtta ctcaccactg 4440

cgatccccgg aaaaacagca ttccaggtat tagaagaata tcctgattca ggtgaaaata 4500

ttgttgatgc gctggcagtg ttcctgcgcc ggttgcattc gattcctgtt tgtaattgtc 4560

cttttaacag cgatcgcgta tttcgtctcg ctcaggcgca atcacgaatg aataacggtt 4620

tggttgatgc gagtgatttt gatgacgagc gtaatggctg gcctgttgaa caagtctgga 4680

aagaaatgca taaacttttg ccattctcac cggattcagt cgtcactcat ggtgatttct 4740

cacttgataa ccttattttt gacgagggga aattaatagg ttgtattgat gttggacgag 4800

tcggaatcgc agaccgatac caggatcttg ccatcctatg gaactgcctc ggtgagtttt 4860

ctccttcatt acagaaacgg ctttttcaaa aatatggtat tgataatcct gatatgaata 4920

aattgcagtt tcatttgatg ctcgatgagt ttttctaatc agaattggtt aattggttgt 4980

aacattattc agattgggcc ccgttccact gagcgtcaga ccccgtagaa aagatcaaag 5040

gatcttcttg agatcctttt tttctgcgcg taatctgctg cttgcaaaca aaaaaaccac 5100

cgctaccagc ggtggtttgt ttgccggatc aagagctacc aactcttttt ccgaaggtaa 5160

ctggcttcag cagagcgcag ataccaaata ctgttcttct agtgtagccg tagttaggcc 5220

accacttcaa gaactctgta gcaccgccta catacctcgc tctgctaatc ctgttaccag 5280

tggctgctgc cagtggcgat aagtcgtgtc ttaccgggtt ggactcaaga cgatagttac 5340

cggataaggc gcagcggtcg ggctgaacgg ggggttcgtg cacacagccc agcttggagc 5400

gaacgaccta caccgaactg agatacctac agcgtgagct atgagaaagc gccacgcttc 5460

ccgaagggag aaaggcggac aggtatccgg taagcggcag ggtcggaaca ggagagcgca 5520

cgagggagct tccaggggga aacgcctggt atctttatag tcctgtcggg tttcgccacc 5580

tctgacttga gcgtcgattt ttgtgatgct cgtcaggggg gcggagccta tggaaaaacg 5640

ccagcaacgc g 5651

<210> 8
<211> 4040
<212> DNA
<213> Artificial Sequence

<220>
<223> pVEGF

<400> 8
cgagaagcgg aagagcgccc aatacgcaaa ccgcctctcc ccgcgcgttg gccgattcat 60

taatgcagct ggcacgacag gtttcccgac tggaaagcgg gcagtgagcg caacgcaatt 120

aatgtgagtt agctcactca ttaggcaccc caggctttac actttatgct tccggctcgt 180

atgttgtgtg gaattgtgag cggataacaa tttcacacag gaaacagcta tgaccatgat 240

tacgccaagc tctagctaga ggtcgaccaa ttctcatgtt tgacagctta tcatcgcaga 300

tccgggcaac gttgttgcca ttgctgcagg cgcagaactg gtaggtatgg aagatctata 360

cattgaatca atattggcaa ttagccatat tagtcattgg ttatatagca taaatcaata 420

ttggctattg gccattgcat acgttgtatc tatatcataa tatgtacatt tatattggct 480

catgtccaat atgaccgcca tgttgacatt gattattgac tagttattaa tagtaatcaa 540

ttacggggtc attagttcat agcccatata tggagttccg cgttacataa cttacggtaa 600

atggcccgcc tggctgaccg cccaacgacc cccgcccatt gacgtcaata atgacgtatg 660

ttcccatagt aacgccaata gggactttcc attgacgtca atgggtggag tatttacggt 720

aaactgccca cttggcagta catcaagtgt atcatatgcc aagtccgccc cctattgacg 780

tcaatgacgg taaatggccc gcctggcatt atgcccagta catgacctta cgggactttc 840

ctacttggca gtacatctac gtattagtca tcgctattac catggtgatg cggttttggc 900

agtacaccaa tgggcgtgga tagcggtttg actcacgggg atttccaagt ctccacccca 960

ttgacgtcaa tgggagtttg ttttggcacc aaaatcaacg ggactttcca aaatgtcgta 1020

ataaccccgc cccgttgacg caaatgggcg gtaggcgtgt acggtgggag gtctatataa 1080

gcagagctcg tttagtgaac cgtcagatcc tcactctctt ccgcatcgct gtctgcgagg 1140

gccagctgtt gggctcgcgg ttgaggacaa actcttcgcg gtctttccag tactcttgga 1200

tcggaaaccc gtcggcctcc gaacggtact ccgccaccga gggacctgag cgagtccgca 1260

tcgaccggat cggaaaacct ctcgagaaag gcgtctaacc agtcacagtc gcaaggtagg 1320

ctgagcaccg tggcgggcgg cagcgggtgg cggtcggggt tgtttctggc ggaggtgctg 1380

ctgatgatgt aattaaagta ggcggtcttg agacggcgga tggtcgaggt gaggtgtggc 1440

aggcttgaga tccagctgtt ggggtgagta ctccctctca aaagcgggca ttacttctgc 1500

gctaagattg tcagtttcca aaaacgagga ggatttgata ttcacctggc ccgatctggc 1560

catacacttg agtgacaatg acatccactt tgcctttctc tccacaggtg tccactccca 1620

ggtccaagtt taaactgcgg ccgccaccat gaactttctg ctgtcttggg tgcattggag 1680

ccttgccttg ctgctctacc tccaccatgc caagtggtcc caggctgcac ccatggcaga 1740

aggagggggg cagaatcatc acgaagtggt gaagttcatg gatgtctatc agcgcagcta 1800

ctgccatcca atcgagaccc tggtggacat cttccaggag taccctgatg agatcgagta 1860

catcttcaag ccatcctgtg tgcccctgat gcgatgcggg ggctgctgca atgacgaggg 1920
cctggagtgt gtgcccactg aggagtccaa catcaccatg cagattatgc ggatcaaacc 1980
tcaccaaggc cagcacatag gagagatgag cttcctacag cacaacaaat gtgaatgcag 2040
accaaagaaa gatagagcaa gacaagaaaa tccctgtggg ccttgctcag agcggagaaa 2100
gcatttgttt gtacaagatc cgcagacgtg taaatgttcc tgcaaaaaca cagactcgcg 2160
ttgcaaggcg aggcagcttg agttaaacga acgtacttgc agatgtgaca agccgaggcg 2220
gtgagtctag aaagcttgat atctagtaat gagtttaaac gggggaggct aactgaaaca 2280
cggaaggaga caataccgga aggaacccgc gctatgacgg caataaaaag acagaataaa 2340
acgcacgggt gttgggtcgt ttgttcataa acgcggggtt cggtcccagg gctggcactc 2400
tgtcgatacc ccaccgagac cccattgggg ccaatacgcc cgcgtttctt ccttttcccc 2460
accccacccc ccaagttcgg gtgaaggccc agggctcgca gccaacgtcg ggcggcaggg 2520
ccctgccata gggatccatg agccatattc aacgggaaac gtcgaggccg cgattaaatt 2580
ccaacatgga tgctgattta tatgggtata aatgggctcg cgataatgtc gggcaatcag 2640
gtgcgacaat ctatcgcttg tatgggaagc ccgatgcgcc agagttgttt ctgaaacatg 2700
gcaaaggtag cgttgccaat gatgttacag atgagatggt cagactaaac tggctgacgg 2760
aatttatgcc tcttccgacc atcaagcatt ttatccgtac tcctggtgat gcatggttac 2820
tcaccactgc gatccccgga aaaacagcat tccaggtatt agaagaatat cctgattcag 2880
gtgaaaatat tgttgatgcg ctggcagtgt tcctgcgccg gttgcattcg attcctgttt 2940
gtaattgtcc ttttaacagc gatcgcgtat ttcgtctcgc tcaggcgcaa tcacgaatga 3000
ataacggttt ggttgatgcg agtgattttg atgacgagcg taatggctgg cctgttgaac 3060
aagtctggaa agaaatgcat aaacttttgc cattctcacc ggattcagtc gtcactcatg 3120
gtgatttctc acttgataac cttatttttg acgaggggaa attaataggt tgtattgatg 3180
ttggacgagt cggaatcgca gaccgatacc aggatcttgc catcctatgg aactgcctcg 3240
gtgagttttc tccttcatta cagaaacggc tttttcaaaa atatggtatt gataatcctg 3300
atatgaataa attgcagttt catttgatgc tcgatgagtt tttctaatca gaattggtta 3360
attggttgta acattattca gattgggccc cgttccactg agcgtcagac cccgtagaaa 3420
agatcaaagg atcttcttga gatccttttt ttctgcgcgt aatctgctgc ttgcaaacaa 3480
aaaaaccacc gctaccagcg gtggtttgtt tgccggatca agagctacca actctttttc 3540
cgaaggtaac tggcttcagc agagcgcaga taccaaatac tgttcttcta gtgtagccgt 3600
agttaggcca ccacttcaag aactctgtag caccgcctac atacctcgct ctgctaatcc 3660
tgttaccagt ggctgctgcc agtggcgata agtcgtgtct taccgggttg gactcaagac 3720
gatagttacc ggataaggcg cagcggtcgg gctgaacggg gggttcgtgc acacagccca 3780

```
gcttggagcg aacgacctac accgaactga gatacctaca gcgtgagcta tgagaaagcg      3840
ccacgcttcc cgaagggaga aaggcggaca ggtatccggt aagcggcagg gtcggaacag      3900
gagagcgcac gagggagctt ccagggggaa acgcctggta tctttatagt cctgtcgggt      3960
ttcgccacct ctgacttgag cgtcgatttt tgtgatgctc gtcagggggg cggagcctat      4020
ggaaaaacgc cagcaacgcg                                                  4040
```